# EUROPEAN PATENT APPLICATION

(11) **EP 3 904 385 A1**
(43) Date of publication of application: **03.11.2021**
(21) Application number: 19902682.4
(22) Date of filing: 27.12.2019
(51) Int. Cl.: C07K 16/28, C12N 15/13, A61K 39/395, A61P 37/02, A61P 37/08, A61P 11/06, A61P 17/00, A61P 35/00

(54) **ANTIBODY AGAINST HUMAN IL-4RA AND USE THEREOF**

(30) Priority: 27.12.2018 CN 201811618948
(71) Applicant: Akeso Biopharma, Inc., Zhongshan, Guangdong 528437 (CN)
(72) Inventor: LI, Baiyong, Zhongshan, Guangdong 528437 (CN); XIA, Yu, Zhongshan, Guangdong 528437 (CN); WANG, Zhongmin, Zhongshan, Guangdong 528437 (CN); ZHANG, Peng, Zhongshan, Guangdong 528437 (CN)
(74) Representative: Nederlandsch Octrooibureau
(86) International application number: PCT/CN2019/129193
(87) International publication number: WO 2020/135710

(57) **Abstract**

The present invention relates to an anti-human interleukin-4-receptor A antibody, a pharmaceutical composition or a kit comprising the antibody, and use thereof.

SEQUENCE LISTING

<110>  Akeso Biopharma, Inc

<120>  ANTIBODY AGAINST HUMAN IL-4RA AND USE THEREOF

<130>  P6104823PCT-EP

<150>  CN 201811618948.8
<151>  2018-12-27

<160>  143

<170>  PatentIn version 3.3

<210>  1
<211>  348
<212>  DNA
<213>  Artificial Sequence
<220>
<223>  Artificial Sequence 1

<400>  1

gaggtgcagc tgcagcagag cggaccagag ctggtgaagc ctggcgcctc tgtgaagatc      60
agctgtaaga cctccggcta caccttcaca gagtatacaa tccactgggt gaagcagaac     120
cacggcaaga gcctggagtg gatcggcggc atcaatccta acaatggcgg caccgtgtac     180
aaccagaact tcaagggcaa ggccaccctg acagtggaca agagctcctc taccgcctat     240
atggagctga ggtctctgac aagcgaggac tccgccgtgt actattgcgc cagagtgcgg     300
agaggcatgg attactgggg ccagggcacc tccgtgacag tgagctcc                  348

<210>  2
<211>  116
<212>  PRT
<213>  Artificial Sequence
<220>
<223>  Artificial Sequence 2

<400>  2

Glu Val Gln Leu Gln Gln Ser Gly Pro Glu Leu Val Lys Pro Gly Ala
1               5                   10                  15
Ser Val Lys Ile Ser Cys Lys Thr Ser Gly Tyr Thr Phe Thr Glu Tyr
            20                  25                  30
Thr Ile His Trp Val Lys Gln Asn His Gly Lys Ser Leu Glu Trp Ile
        35                  40                  45
Gly Gly Ile Asn Pro Asn Asn Gly Gly Thr Val Tyr Asn Gln Asn Phe
    50                  55                  60
Lys Gly Lys Ala Thr Leu Thr Val Asp Lys Ser Ser Ser Thr Ala Tyr
65                  70                  75                  80
Met Glu Leu Arg Ser Leu Thr Ser Glu Asp Ser Ala Val Tyr Tyr Cys
                85                  90                  95
Ala Arg Val Arg Arg Gly Met Asp Tyr Trp Gly Gln Gly Thr Ser Val
            100                 105                 110
Thr Val Ser Ser
        115

<210>  3
<211>  321
<212>  DNA
<213>  Artificial Sequence
<220>
<223>  Artificial Sequence 3

<400>  3

gacatcgtga tgacccagtc ccacaagttt atgtccacat ctgtgggcga cagggtgtcc      60
atcacctgta aggcctctca ggatgtgacc acagccgtgg cctggtacca gcagaagcca     120
ggccagtctc ccaagctgct gatctatagc gcctcctacc ggtatacagg cgtgcccgac     180
agattcaccg gctctggcag cggcacagat ttcaccttta caatcagctc cgtgcaggca     240
gaggacctgg ccgtgtacta ttgccagcag cactactctg ccccttggac cttcggcgga     300
ggaacaaacc tggagatcaa g                                               321

<210>  4
<211>  107
<212>  PRT
<213>  Artificial Sequence
<220>
<223>  Artificial Sequence 4

<400>  4

Asp Ile Val Met Thr Gln Ser His Lys Phe Met Ser Thr Ser Val Gly
1               5                   10                  15
Asp Arg Val Ser Ile Thr Cys Lys Ala Ser Gln Asp Val Thr Thr Ala
            20                  25                  30
Val Ala Trp Tyr Gln Gln Lys Pro Gly Gln Ser Pro Lys Leu Leu Ile
        35                  40                  45
Tyr Ser Ala Ser Tyr Arg Tyr Thr Gly Val Pro Asp Arg Phe Thr Gly
    50                  55                  60
Ser Gly Ser Gly Thr Asp Phe Thr Phe Thr Ile Ser Ser Val Gln Ala
65                  70                  75                  80
Glu Asp Leu Ala Val Tyr Tyr Cys Gln Gln His Tyr Ser Ala Pro Trp
                85                  90                  95
Thr Phe Gly Gly Gly Thr Asn Leu Glu Ile Lys
            100                 105

<210>  5
<211>  360
<212>  DNA
<213>  Artificial Sequence
<220>
<223>  Artificial Sequence 5

<400>  5

gatgtgaagc tggtggagtc cggcggcgac ctggtgaacc tgggcggaag cctgaagctg      60
agctgtgccg ccagcggctt cacattttcc tccagctaca tgagctgggt gaggcagaca     120
cccgagagga gactggagct ggtggccgcc atcaacagca atggcggcaa gacatactac     180
cctgataccg tgaagggcag attcaccatc agcagggaca acgccaagaa caccctgtac     240
ctgcagatgt ccggcctgaa gacagaggac acagccctgt actactgcac caggcagagg     300
ggcaactacg tgggcgccat ggactactgg ggccagggca caagcgtgac cgtgagctcc     360

<210>  6
<211>  120
<212>  PRT
<213>  Artificial Sequence
<220>
<223>  Artificial Sequence 6

<400>  6

Asp Val Lys Leu Val Glu Ser Gly Gly Asp Leu Val Asn Leu Gly Gly
1               5                   10                  15
Ser Leu Lys Leu Ser Cys Ala Ala Ser Gly Phe Thr Phe Ser Ser Ser
            20                  25                  30

Tyr Met Ser Trp Val Arg Gln Thr Pro Glu Arg Arg Leu Glu Leu Val
        35                  40                  45
Ala Ala Ile Asn Ser Asn Gly Gly Lys Thr Tyr Tyr Pro Asp Thr Val
    50                  55                  60
Lys Gly Arg Phe Thr Ile Ser Arg Asp Asn Ala Lys Asn Thr Leu Tyr
65                  70                  75                  80
Leu Gln Met Ser Gly Leu Lys Thr Glu Asp Thr Ala Leu Tyr Tyr Cys
                85                  90                  95
Thr Arg Gln Arg Gly Asn Tyr Val Gly Ala Met Asp Tyr Trp Gly Gln
            100                 105                 110
Gly Thr Ser Val Thr Val Ser Ser
        115                 120

<210>  7
<211>  321
<212>  DNA
<213>  Artificial Sequence
<220>
<223>  Artificial Sequence 7

<400>  7

gacatcgtga tgacacagtc ccacaagttt atgtccacat ccgtgggcga tagggtgtcc      60
atcacatgca aggccagcca ggacgtgagc acagccgtgg tgtggtacca gcagaagcct     120
ggccagagcc ccaccctgct gatctactcc gccagctaca ggtacacagg cgtgcctgac     180
agattcacag gctccggcag cggcaccgat ttcaccttta ccatctcctc cgtgcaggcc     240
gaggatctgg ccgtgtacta ctgccaccag tactacggca gcccccccac attcggcggc     300
ggaacaaagc tggagatcaa g                                               321

<210>  8
<211>  107
<212>  PRT
<213>  Artificial Sequence
<220>
<223>  Artificial Sequence 8

<400>  8

Asp Ile Val Met Thr Gln Ser His Lys Phe Met Ser Thr Ser Val Gly
1               5                   10                  15
Asp Arg Val Ser Ile Thr Cys Lys Ala Ser Gln Asp Val Ser Thr Ala
            20                  25                  30
Val Val Trp Tyr Gln Gln Lys Pro Gly Gln Ser Pro Thr Leu Leu Ile
        35                  40                  45
Tyr Ser Ala Ser Tyr Arg Tyr Thr Gly Val Pro Asp Arg Phe Thr Gly
    50                  55                  60
Ser Gly Ser Gly Thr Asp Phe Thr Phe Thr Ile Ser Ser Val Gln Ala
65                  70                  75                  80
Glu Asp Leu Ala Val Tyr Tyr Cys His Gln Tyr Tyr Gly Ser Pro Pro
                85                  90                  95
Thr Phe Gly Gly Gly Thr Lys Leu Glu Ile Lys
            100                 105

<210>  9
<211>  8
<212>  PRT
<213>  Artificial Sequence
<220>
<223>  Artificial Sequence 9

<400>  9

Gly Tyr Thr Phe Thr Glu Tyr Thr

1                 5

<210>  10
<211>  8
<212>  PRT
<213>  Artificial Sequence
<220>
<223>  Artificial Sequence 10

<400>  10

Ile Asn Pro Asn Asn Gly Gly Thr
1                 5

<210>  11
<211>  9
<212>  PRT
<213>  Artificial Sequence
<220>
<223>  Artificial Sequence 11

<400>  11

Ala Arg Val Arg Arg Gly Met Asp Tyr
1                 5

<210>  12
<211>  6
<212>  PRT
<213>  Artificial Sequence
<220>
<223>  Artificial Sequence 12

<400>  12

Gln Asp Val Thr Thr Ala
1                 5

<210>  13
<211>  3
<212>  PRT
<213>  Artificial Sequence
<220>
<223>  Artificial Sequence 13

<400>  13

Ser Ala Ser
1

<210>  14
<211>  9
<212>  PRT
<213>  Artificial Sequence
<220>
<223>  Artificial Sequence 14

<400>  14

Gln Gln His Tyr Ser Ala Pro Trp Thr
1                 5

<210>  15

<211>  8
<212>  PRT
<213>  Artificial Sequence
<220>
<223>  Artificial Sequence 15

<400>  15

Gly Phe Thr Phe Ser Ser Ser Tyr
1               5

<210>  16
<211>  8
<212>  PRT
<213>  Artificial Sequence
<220>
<223>  Artificial Sequence 16

<400>  16

Ile Asn Ser Asn Gly Gly Lys Thr
1               5

<210>  17
<211>  13
<212>  PRT
<213>  Artificial Sequence
<220>
<223>  Artificial Sequence 17

<400>  17

Thr Arg Gln Arg Gly Asn Tyr Val Gly Ala Met Asp Tyr
1               5                   10

<210>  18
<211>  6
<212>  PRT
<213>  Artificial Sequence
<220>
<223>  Artificial Sequence 18

<400>  18

Gln Asp Val Ser Thr Ala
1               5

<210>  19
<211>  3
<212>  PRT
<213>  Artificial Sequence
<220>
<223>  Artificial Sequence 19

<400>  19

Ser Ala Ser
1

<210>  20
<211>  9
<212>  PRT
<213>  Artificial Sequence

<220>
<223>  Artificial Sequence 20

<400>  20

His Gln Tyr Tyr Gly Ser Pro Pro Thr
1               5

<210>  21
<211>  348
<212>  DNA
<213>  Artificial Sequence
<220>
<223>  Artificial Sequence 21

<400>  21

caggtgcagc tgcagcagtc cggagcagag gtggtgaagc caggagccag cgtgaagatc      60
tcctgtaaga cctctggcta caccttcaca gagtatacaa tccactgggt gaagcaggca     120
cacggacaga gcctggagtg gatcggcggc atcaacccta acaatggcgg caccgtgtac     180
aatcagaagt ttcagggcaa ggccaccctg acagtggaca agtctaccag cacagcctat     240
atggagctga ggtccctgac ctctgaggac acagccgtgt actattgcgc ccgggtgcgg     300
agaggcatgg attactgggg ccagggcacc tccgtgacag tgagctcc                  348

<210>  22
<211>  116
<212>  PRT
<213>  Artificial Sequence
<220>
<223>  Artificial Sequence 22

<400>  22

Gln Val Gln Leu Gln Gln Ser Gly Ala Glu Val Val Lys Pro Gly Ala
1               5                   10                  15
Ser Val Lys Ile Ser Cys Lys Thr Ser Gly Tyr Thr Phe Thr Glu Tyr
            20                  25                  30
Thr Ile His Trp Val Lys Gln Ala His Gly Gln Ser Leu Glu Trp Ile
        35                  40                  45
Gly Gly Ile Asn Pro Asn Asn Gly Gly Thr Val Tyr Asn Gln Lys Phe
    50                  55                  60
Gln Gly Lys Ala Thr Leu Thr Val Asp Lys Ser Thr Ser Thr Ala Tyr
65                  70                  75                  80
Met Glu Leu Arg Ser Leu Thr Ser Glu Asp Thr Ala Val Tyr Tyr Cys
                85                  90                  95
Ala Arg Val Arg Arg Gly Met Asp Tyr Trp Gly Gln Gly Thr Ser Val
            100                 105                 110
Thr Val Ser Ser
        115

<210>  23
<211>  321
<212>  DNA
<213>  Artificial Sequence
<220>
<223>  Artificial Sequence 23

<400>  23

gacatccaga tgacccagtc tcctaagtct ctgagcacat ccgtgggcga ccgggtgacc      60
atcacatgta gagccagcca ggatgtgacc acagcagtgg catggtacca gcagaagcct     120
ggcaagtccc ctaagctgct gatctattct gccagctaca ggtataccgg agtgccatct     180
cggttctccg gctctggcag cggcacagac ttcaccttta caatcagctc cgtgcagcca     240

gaggatctgg ccacctacta ttgccagcag cactacagcg ccccatggac ctttggcgga     300
ggaacaaacc tggagatcaa g                                                321

<210>  24
<211>  107
<212>  PRT
<213>  Artificial Sequence
<220>
<223>  Artificial Sequence 24

<400>  24

Asp Ile Gln Met Thr Gln Ser Pro Lys Ser Leu Ser Thr Ser Val Gly
1               5                   10                  15
Asp Arg Val Thr Ile Thr Cys Arg Ala Ser Gln Asp Val Thr Thr Ala
            20                  25                  30
Val Ala Trp Tyr Gln Gln Lys Pro Gly Lys Ser Pro Lys Leu Leu Ile
        35                  40                  45
Tyr Ser Ala Ser Tyr Arg Tyr Thr Gly Val Pro Ser Arg Phe Ser Gly
    50                  55                  60
Ser Gly Ser Gly Thr Asp Phe Thr Phe Thr Ile Ser Ser Val Gln Pro
65                  70                  75                  80
Glu Asp Leu Ala Thr Tyr Tyr Cys Gln Gln His Tyr Ser Ala Pro Trp
                85                  90                  95
Thr Phe Gly Gly Gly Thr Asn Leu Glu Ile Lys
            100                 105

<210>  25
<211>  348
<212>  DNA
<213>  Artificial Sequence
<220>
<223>  Artificial Sequence 25

<400>  25

caggtgcagc tggtgcagtc cggagcagag gtggtgaagc caggagccag cgtgaaggtg      60
tcctgtaaga cctctggcta caccttcaca gagtatacaa tccactgggt gcggcaggca     120
ccaggacagt ctctggagtg gatcggcggc atcaacccta acaatggcgg caccaactac     180
aatcagaagt ttcagggcaa ggtgaccctg acagtggaca agtccacctc tacagcctat     240
atggagctga gctccctgag gtctgaggac acagccgtgt actattgcgc ccgcgtgcgg     300
agaggcatgg attactgggg ccagggcacc agcgtgacag tgtctagc                  348

<210>  26
<211>  116
<212>  PRT
<213>  Artificial Sequence
<220>
<223>  Artificial Sequence 26

<400>  26

Gln Val Gln Leu Val Gln Ser Gly Ala Glu Val Val Lys Pro Gly Ala
1               5                   10                  15
Ser Val Lys Val Ser Cys Lys Thr Ser Gly Tyr Thr Phe Thr Glu Tyr
            20                  25                  30
Thr Ile His Trp Val Arg Gln Ala Pro Gly Gln Ser Leu Glu Trp Ile
        35                  40                  45
Gly Gly Ile Asn Pro Asn Asn Gly Gly Thr Asn Tyr Asn Gln Lys Phe
    50                  55                  60
Gln Gly Lys Val Thr Leu Thr Val Asp Lys Ser Thr Ser Thr Ala Tyr
65                  70                  75                  80
Met Glu Leu Ser Ser Leu Arg Ser Glu Asp Thr Ala Val Tyr Tyr Cys

                    85                      90                      95
Ala Arg Val Arg Arg Gly Met Asp Tyr Trp Gly Gln Gly Thr Ser Val
            100                     105                     110
Thr Val Ser Ser
        115

<210>  27
<211>  321
<212>  DNA
<213>  Artificial Sequence
<220>
<223>  Artificial Sequence 27

<400>  27

gacatccaga tgacccagag ccctagctcc ctgagcgcct ccgtgggcga cagggtgacc      60
atcacatgta gagcctccca ggatgtgacc acagcagtgg catggtacca gcagaagcca     120
ggcaaggccc ctaagctgct gatctactcc gcctctagca ggtataccgg agtgccatct     180
cggttctctg gcagcggctc cggcacagac tttaccctga caatctcctc tgtgcagcca     240
gaggatctgg ccacatacta ttgccagcag cactattctg ccccctggac ctttggcggc     300
ggcacaaacc tggagatcaa g                                               321

<210>  28
<211>  107
<212>  PRT
<213>  Artificial Sequence
<220>
<223>  Artificial Sequence 28

<400>  28

Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Val Thr Thr Ala
            20                  25                  30
Val Ala Trp Tyr Gln Gln Lys Pro Gly Lys Ala Pro Lys Leu Leu Ile
        35                  40                  45
Tyr Ser Ala Ser Ser Arg Tyr Thr Gly Val Pro Ser Arg Phe Ser Gly
    50                  55                  60
Ser Gly Ser Gly Thr Asp Phe Thr Leu Thr Ile Ser Ser Val Gln Pro
65                  70                  75                  80
Glu Asp Leu Ala Thr Tyr Tyr Cys Gln Gln His Tyr Ser Ala Pro Trp
                85                  90                  95
Thr Phe Gly Gly Gly Thr Asn Leu Glu Ile Lys
            100                 105

<210>  29
<211>  348
<212>  DNA
<213>  Artificial Sequence
<220>
<223>  Artificial Sequence 29

<400>  29

caggtgcagc tggtgcagtc cggagcagag gtggtgaagc caggagccag cgtgaaggtg      60
tcctgtaagg cctctggcta caccttcaca gagtatacca tccactgggt gcggcaggca     120
ccaggacagg gactggagtg gatcggcggc atcaacccta acaatggcgg cacaaattac     180
gcccagaagt ttcagggcag ggtgaccatc acagtggaca agtccacctc tacagcctat     240
atggagctga gctccctgag gtctgaggac accgccgtgt actattgcgc ccgcgtgcgg     300
agaggcatgg attactgggg ccagggcacc agcgtgacag tgtctagc                  348

<210>  30

<211>  116
<212>  PRT
<213>  Artificial Sequence
<220>
<223>  Artificial Sequence 30

<400>  30

Gln Val Gln Leu Val Gln Ser Gly Ala Glu Val Val Lys Pro Gly Ala
1               5                   10                  15
Ser Val Lys Val Ser Cys Lys Ala Ser Gly Tyr Thr Phe Thr Glu Tyr
            20                  25                  30
Thr Ile His Trp Val Arg Gln Ala Pro Gly Gln Gly Leu Glu Trp Ile
        35                  40                  45
Gly Gly Ile Asn Pro Asn Asn Gly Gly Thr Asn Tyr Ala Gln Lys Phe
    50                  55                  60
Gln Gly Arg Val Thr Ile Thr Val Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Val Arg Arg Gly Met Asp Tyr Trp Gly Gln Gly Thr Ser Val
            100                 105                 110
Thr Val Ser Ser
        115

<210>  31
<211>  321
<212>  DNA
<213>  Artificial Sequence
<220>
<223>  Artificial Sequence 31

<400>  31

gacatccaga tgacccagag ccctagctcc ctgagcgcct ccgtgggcga cagggtgacc      60
atcacatgta gagcctccca ggatgtgacc acagccctgg catggtacca gcagaagcca     120
ggcaaggccc ctaagctgct gatctactcc gcctctagcc tgtataccgg agtgccatct     180
cggttctctg gcagcggctc cggcacagac tttaccctga caatctcctc tctgcagcca     240
gaggatttcg ccacatacta ttgccagcag cactattctg ccccctggac ctttggcggc     300
ggcacaaacc tggagatcaa g                                               321

<210>  32
<211>  107
<212>  PRT
<213>  Artificial Sequence
<220>
<223>  Artificial Sequence 32

<400>  32

Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Val Thr Thr Ala
            20                  25                  30
Leu Ala Trp Tyr Gln Gln Lys Pro Gly Lys Ala Pro Lys Leu Leu Ile
        35                  40                  45
Tyr Ser Ala Ser Ser Leu Tyr Thr Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln His Tyr Ser Ala Pro Trp
                85                  90                  95
Thr Phe Gly Gly Gly Thr Asn Leu Glu Ile Lys

            100                 105

<210> 33
<211> 348
<212> DNA
<213> Artificial Sequence
<220>
<223> Artificial Sequence 33

<400> 33

caggtgcagc tggtgcagtc cggagcagag gtggtgaagc caggagccag cgtgaaggtg 60
tcctgtaaga cctctggcta caccttcaca gagtatacaa tccactgggt gcggcaggca 120
ccaggacagt ctctggagtg gatcggcggc atcaacccta acaatggcgg caccgtctac 180
aatcagaagt ttcagggcaa ggtgaccctg acagtggaca agtccacctc tacagcctat 240
atggagctga gctccctgag gtctgaggac acagccgtgt actattgcgc ccgcgtgcgg 300
agaggcatgg attactgggg ccagggcacc agcgtgacag tgtctagc 348

<210> 34
<211> 116
<212> PRT
<213> Artificial Sequence
<220>
<223> Artificial Sequence 34

<400> 34

Gln Val Gln Leu Val Gln Ser Gly Ala Glu Val Val Lys Pro Gly Ala
1               5                   10                  15
Ser Val Lys Val Ser Cys Lys Thr Ser Gly Tyr Thr Phe Thr Glu Tyr
            20                  25                  30
Thr Ile His Trp Val Arg Gln Ala Pro Gly Gln Ser Leu Glu Trp Ile
        35                  40                  45
Gly Gly Ile Asn Pro Asn Asn Gly Gly Thr Val Tyr Asn Gln Lys Phe
    50                  55                  60
Gln Gly Lys Val Thr Leu Thr Val Asp Lys Ser Thr Ser Thr Ala Tyr
65                  70                  75                  80
Met Glu Leu Ser Ser Leu Arg Ser Glu Asp Thr Ala Val Tyr Tyr Cys
                85                  90                  95
Ala Arg Val Arg Arg Gly Met Asp Tyr Trp Gly Gln Gly Thr Ser Val
            100                 105                 110
Thr Val Ser Ser
        115

<210> 35
<211> 321
<212> DNA
<213> Artificial Sequence
<220>
<223> Artificial Sequence 35

<400> 35

gacatccaga tgacccagag ccctagctcc ctgagcgcct ccgtgggcga cagggtgacc 60
atcacatgta gagcctccca ggatgtgacc acagcagtgg catggtacca gcagaagcca 120
ggcaaggccc ctaagctgct gatctactcc gcctcttaca ggtataccgg agtgccatct 180
cggttctctg gcagcggctc cggcacagac tttaccctga caatctcctc tgtgcagcca 240
gaggatctgg ccacatacta ttgccagcag cactattctg ccccctggac ctttggcggc 300
ggcacaaacc tggagatcaa g 321

<210> 36
<211> 107
<212> PRT

<213>  Artificial Sequence
<220>
<223>  Artificial Sequence 36

<400>  36

Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Asp Val Thr Thr Ala
            20                  25                  30
Val Ala Trp Tyr Gln Gln Lys Pro Gly Lys Ala Pro Lys Leu Leu Ile
        35                  40                  45
Tyr Ser Ala Ser Tyr Arg Tyr Thr Gly Val Pro Ser Arg Phe Ser Gly
    50                  55                  60
Ser Gly Ser Gly Thr Asp Phe Thr Leu Thr Ile Ser Ser Val Gln Pro
65                  70                  75                  80
Glu Asp Leu Ala Thr Tyr Tyr Cys Gln Gln His Tyr Ser Ala Pro Trp
                85                  90                  95
Thr Phe Gly Gly Gly Thr Asn Leu Glu Ile Lys
            100                 105

<210>  37
<211>  360
<212>  DNA
<213>  Artificial Sequence
<220>
<223>  Artificial Sequence 37

<400>  37

gacgtgcagc tggtggagag cggcggcggc ctggtgcagc tgggcggctc cctgaggctg      60
tcttgtgcag ccagcggctt cacctttagc tcctcttaca tgtcttgggt gcggcagaca     120
cccgagaaga gactggagct ggtggccgcc atcaactcta atggcggcaa gacctactat     180
gccgacagcg tgaagggcag gtttaccatc tcccgcgata actctaagaa tacactgtat     240
ctgcagatga gctccctgag agccgaggac accgccgtgt actattgcac acggcagaga     300
ggcaactacg tgggcgccat ggattattgg ggccagggca ccagcgtgac agtgtctagc     360

<210>  38
<211>  120
<212>  PRT
<213>  Artificial Sequence
<220>
<223>  Artificial Sequence 38

<400>  38

Asp Val Gln Leu Val Glu Ser Gly Gly Gly Leu Val Gln Leu Gly Gly
1               5                   10                  15
Ser Leu Arg Leu Ser Cys Ala Ala Ser Gly Phe Thr Phe Ser Ser Ser
            20                  25                  30
Tyr Met Ser Trp Val Arg Gln Thr Pro Glu Lys Arg Leu Glu Leu Val
        35                  40                  45
Ala Ala Ile Asn Ser Asn Gly Gly Lys Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Ser Ser Leu Arg Ala Glu Asp Thr Ala Val Tyr Tyr Cys
                85                  90                  95
Thr Arg Gln Arg Gly Asn Tyr Val Gly Ala Met Asp Tyr Trp Gly Gln
            100                 105                 110
Gly Thr Ser Val Thr Val Ser Ser
        115                 120

<210>  39
<211>  321
<212>  DNA
<213>  Artificial Sequence
<220>
<223>  Artificial Sequence 39

<400>  39

gacatccaga tgacccagtc tcccagctcc ctgtccacat ctgtgggcga ccgggtgacc      60
atcacctgta aggcctctca ggatgtgagc accgccgtgg tgtggtacca gcagaagcca     120
ggcaagagcc ccaagctgct gatctatagc gcctcctacc ggtataccgg cgtgccttcc     180
agattctctg gcagcggctc cggcacagac ttcaccttta caatctctag cgtgcagcca     240
gaggatatcg ccacatacta ttgccaccag tactatggca gccctcccac cttcggcggc     300
ggcacaaagc tggagatcaa g                                               321

<210>  40
<211>  107
<212>  PRT
<213>  Artificial Sequence
<220>
<223>  Artificial Sequence 40

<400>  40

Asp Ile Gln Met Thr Gln Ser Pro Ser Ser Leu Ser Thr Ser Val Gly
1               5                   10                  15
Asp Arg Val Thr Ile Thr Cys Lys Ala Ser Gln Asp Val Ser Thr Ala
            20                  25                  30
Val Val Trp Tyr Gln Gln Lys Pro Gly Lys Ser Pro Lys Leu Leu Ile
        35                  40                  45
Tyr Ser Ala Ser Tyr Arg Tyr Thr Gly Val Pro Ser Arg Phe Ser Gly
    50                  55                  60
Ser Gly Ser Gly Thr Asp Phe Thr Phe Thr Ile Ser Ser Val Gln Pro
65                  70                  75                  80
Glu Asp Ile Ala Thr Tyr Tyr Cys His Gln Tyr Tyr Gly Ser Pro Pro
                85                  90                  95
Thr Phe Gly Gly Gly Thr Lys Leu Glu Ile Lys
            100                 105

<210>  41
<211>  360
<212>  DNA
<213>  Artificial Sequence
<220>
<223>  Artificial Sequence 41

<400>  41

gaggtgcagc tggtggagag cggcggcggc ctggtgcagc ccggcggctc cctgaggctg      60
tcttgtgccg ccagcggctt cacctttagc tcctcttaca tgtcttgggt gcggcaggca     120
cctggcaaga gactggagct ggtggccgcc atcaactcta atggcggcaa gacatactat     180
gccgacagcg tgaagggcag gtttaccatc tcccgcgata actctaagaa tacactgtat     240
ctgcagatga gctccctgag agccgaggac accgccgtgt actattgcac acggcagaga     300
ggcaactacg tgggcgccat ggattattgg ggccagggca ccagcgtgac agtgtctagc     360

<210>  42
<211>  120
<212>  PRT
<213>  Artificial Sequence
<220>
<223>  Artificial Sequence 42

<400>  42

Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Ser
            20                  25                  30
Tyr Met Ser Trp Val Arg Gln Ala Pro Gly Lys Arg Leu Glu Leu Val
        35                  40                  45
Ala Ala Ile Asn Ser Asn Gly Gly Lys Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Ser Ser Leu Arg Ala Glu Asp Thr Ala Val Tyr Tyr Cys
                85                  90                  95
Thr Arg Gln Arg Gly Asn Tyr Val Gly Ala Met Asp Tyr Trp Gly Gln
            100                 105                 110
Gly Thr Ser Val Thr Val Ser Ser
        115                 120

<210>  43
<211>  321
<212>  DNA
<213>  Artificial Sequence
<220>
<223>  Artificial Sequence 43

<400>  43

gacatccaga tgacccagtc tccaagctcc ctgtccgcct ctgtgggcga cagggtgacc      60
atcacatgtc aggcctctca ggatgtgagc acagccgtgg tgtggtacca gcagaagcca     120
ggcaagagcc ccaagctgct gatctatagc gcctcctacc tgtataccgg cgtgccttcc     180
agattctctg gcagcggctc cggcacagac ttcaccttta caatctctag cctgcagcca     240
gaggatatcg ccacctacta ttgccaccag tactatggca gccctcccac cttcggcggc     300
ggcacaaagc tggagatcaa g                                               321

<210>  44
<211>  107
<212>  PRT
<213>  Artificial Sequence
<220>
<223>  Artificial Sequence 44

<400>  44

Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Val Ser Thr Ala
            20                  25                  30
Val Val Trp Tyr Gln Gln Lys Pro Gly Lys Ser Pro Lys Leu Leu Ile
        35                  40                  45
Tyr Ser Ala Ser Tyr Leu Tyr Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys His Gln Tyr Tyr Gly Ser Pro Pro
                85                  90                  95
Thr Phe Gly Gly Gly Thr Lys Leu Glu Ile Lys
            100                 105

<210>  45
<211>  360
<212>  DNA
<213>  Artificial Sequence

<220>
<223>  Artificial Sequence 45

<400>  45

gaggtgcagc tggtggagag cggcggcggc ctggtgcagc ccggcggctc cctgcggctg      60
tcttgtgccg ccagcggctt cacctttagc tcctcttaca tgtcctgggt gaggcaggca     120
cctggcaagg gactggagta cgtgagcgcc atcaactcta atggcggcaa gacatactat     180
gccgactctg tgaagggcag gtttaccatc agccgcgata actccaagaa tacactgtac     240
ctgcagatga gctccctgag ggcagaggac accgccgtgt actattgcac acggcagaga     300
ggcaactacg tgggcgccat ggattattgg ggccagggca cctccgtgac agtgtctagc     360

<210>  46
<211>  120
<212>  PRT
<213>  Artificial Sequence
<220>
<223>  Artificial Sequence 46

<400>  46

Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Ser
            20                  25                  30
Tyr Met Ser Trp Val Arg Gln Ala Pro Gly Lys Gly Leu Glu Tyr Val
        35                  40                  45
Ser Ala Ile Asn Ser Asn Gly Gly Lys Thr Tyr Tyr Ala Asp Ser Val
    50                  55                  60
Lys Gly Arg Phe Thr Ile Ser Arg Asp Asn Ser Lys Asn Thr Leu Tyr
65                  70                  75                  80
Leu Gln Met Ser Ser Leu Arg Ala Glu Asp Thr Ala Val Tyr Tyr Cys
                85                  90                  95
Thr Arg Gln Arg Gly Asn Tyr Val Gly Ala Met Asp Tyr Trp Gly Gln
            100                 105                 110
Gly Thr Ser Val Thr Val Ser Ser
        115                 120

<210>  47
<211>  321
<212>  DNA
<213>  Artificial Sequence
<220>
<223>  Artificial Sequence 47

<400>  47

gacatccaga tgacccagtc tccaagctcc ctgtccgcct ctgtgggcga cagggtgacc      60
atcacatgtc aggcctctca ggatgtgagc acagccctgg cctggtacca gcagaagcca     120
ggcaaggccc ccaagctgct gatctatagc gcctccaacc tggagaccgg cgtgcctagc     180
agattctctg gcagcggctc cggcacagac ttcaccttta caatctctag cctgcagcca     240
gaggatatcg ccacctacta ttgccaccag tactatggca gccctcccac cttcggcggc     300
ggcacaaagc tggagatcaa g                                               321

<210>  48
<211>  107
<212>  PRT
<213>  Artificial Sequence
<220>
<223>  Artificial Sequence 48

<400>  48

Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Val Ser Thr Ala
            20                  25                  30
Leu Ala Trp Tyr Gln Gln Lys Pro Gly Lys Ala Pro Lys Leu Leu Ile
        35                  40                  45
Tyr Ser Ala Ser Asn Leu Glu Thr Gly Val Pro Ser Arg Phe Ser Gly
    50                  55                  60
Ser Gly Ser Gly Thr Asp Phe Thr Phe Thr Ile Ser Ser Leu Gln Pro
65                  70                  75                  80
Glu Asp Ile Ala Thr Tyr Tyr Cys His Gln Tyr Tyr Gly Ser Pro Pro
                85                  90                  95
Thr Phe Gly Gly Gly Thr Lys Leu Glu Ile Lys
            100                 105

<210>  49
<211>  25
<212>  PRT
<213>  Artificial Sequence
<220>
<223>  Artificial Sequence 49

<400>  49

Glu Val Gln Leu Gln Gln Ser Gly Pro Glu Leu Val Lys Pro Gly Ala
1               5                   10                  15
Ser Val Lys Ile Ser Cys Lys Thr Ser
            20                  25

<210>  50
<211>  17
<212>  PRT
<213>  Artificial Sequence
<220>
<223>  Artificial Sequence 50

<400>  50

Ile His Trp Val Lys Gln Asn His Gly Lys Ser Leu Glu Trp Ile Gly
1               5                   10                  15
Gly

<210>  51
<211>  38
<212>  PRT
<213>  Artificial Sequence
<220>
<223>  Artificial Sequence 51

<400>  51

Val Tyr Asn Gln Asn Phe Lys Gly Lys Ala Thr Leu Thr Val Asp Lys
1               5                   10                  15
Ser Ser Ser Thr Ala Tyr Met Glu Leu Arg Ser Leu Thr Ser Glu Asp
            20                  25                  30
Ser Ala Val Tyr Tyr Cys
        35

<210>  52
<211>  11
<212>  PRT
<213>  Artificial Sequence
<220>

<223>  Artificial Sequence 52

<400>  52

Trp Gly Gln Gly Thr Ser Val Thr Val Ser Ser
1               5                   10

<210>  53
<211>  26
<212>  PRT
<213>  Artificial Sequence
<220>
<223>  Artificial Sequence 53

<400>  53

Asp Ile Val Met Thr Gln Ser His Lys Phe Met Ser Thr Ser Val Gly
1               5                   10                  15
Asp Arg Val Ser Ile Thr Cys Lys Ala Ser
            20                  25

<210>  54
<211>  17
<212>  PRT
<213>  Artificial Sequence
<220>
<223>  Artificial Sequence 54

<400>  54

Val Ala Trp Tyr Gln Gln Lys Pro Gly Gln Ser Pro Lys Leu Leu Ile
1               5                   10                  15
Tyr

<210>  55
<211>  36
<212>  PRT
<213>  Artificial Sequence
<220>
<223>  Artificial Sequence 55

<400>  55

Tyr Arg Tyr Thr Gly Val Pro Asp Arg Phe Thr Gly Ser Gly Ser Gly
1               5                   10                  15
Thr Asp Phe Thr Phe Thr Ile Ser Ser Val Gln Ala Glu Asp Leu Ala
            20                  25                  30
Val Tyr Tyr Cys
        35

<210>  56
<211>  10
<212>  PRT
<213>  Artificial Sequence
<220>
<223>  Artificial Sequence 56

<400>  56

Phe Gly Gly Gly Thr Asn Leu Glu Ile Lys
1               5                   10

<210>  57

<211>  25
<212>  PRT
<213>  Artificial Sequence
<220>
<223>  Artificial Sequence 57

<400>  57

Asp Val Lys Leu Val Glu Ser Gly Gly Asp Leu Val Asn Leu Gly Gly
1               5                   10                  15
Ser Leu Lys Leu Ser Cys Ala Ala Ser
            20                  25

<210>  58
<211>  17
<212>  PRT
<213>  Artificial Sequence
<220>
<223>  Artificial Sequence 58

<400>  58

Met Ser Trp Val Arg Gln Thr Pro Glu Arg Arg Leu Glu Leu Val Ala
1               5                   10                  15
Ala

<210>  59
<211>  38
<212>  PRT
<213>  Artificial Sequence
<220>
<223>  Artificial Sequence 59

<400>  59

Tyr Tyr Pro Asp Thr Val Lys Gly Arg Phe Thr Ile Ser Arg Asp Asn
1               5                   10                  15
Ala Lys Asn Thr Leu Tyr Leu Gln Met Ser Gly Leu Lys Thr Glu Asp
            20                  25                  30
Thr Ala Leu Tyr Tyr Cys
        35

<210>  60
<211>  11
<212>  PRT
<213>  Artificial Sequence
<220>
<223>  Artificial Sequence 60

<400>  60

Trp Gly Gln Gly Thr Ser Val Thr Val Ser Ser
1               5                   10

<210>  61
<211>  26
<212>  PRT
<213>  Artificial Sequence
<220>
<223>  Artificial Sequence 61

<400>  61

Asp Ile Val Met Thr Gln Ser His Lys Phe Met Ser Thr Ser Val Gly
1               5                   10                  15
Asp Arg Val Ser Ile Thr Cys Lys Ala Ser
            20                  25

<210>  62
<211>  17
<212>  PRT
<213>  Artificial Sequence
<220>
<223>  Artificial Sequence 62

<400>  62

Val Val Trp Tyr Gln Gln Lys Pro Gly Gln Ser Pro Thr Leu Leu Ile
1               5                   10                  15
Tyr

<210>  63
<211>  36
<212>  PRT
<213>  Artificial Sequence
<220>
<223>  Artificial Sequence 63

<400>  63

Tyr Arg Tyr Thr Gly Val Pro Asp Arg Phe Thr Gly Ser Gly Ser Gly
1               5                   10                  15
Thr Asp Phe Thr Phe Thr Ile Ser Ser Val Gln Ala Glu Asp Leu Ala
            20                  25                  30
Val Tyr Tyr Cys
        35

<210>  64
<211>  10
<212>  PRT
<213>  Artificial Sequence
<220>
<223>  Artificial Sequence 64

<400>  64

Phe Gly Gly Gly Thr Lys Leu Glu Ile Lys
1               5                   10

<210>  65
<211>  25
<212>  PRT
<213>  Artificial Sequence
<220>
<223>  Artificial Sequence 65

<400>  65

Gln Val Gln Leu Gln Gln Ser Gly Ala Glu Val Val Lys Pro Gly Ala
1               5                   10                  15
Ser Val Lys Ile Ser Cys Lys Thr Ser
            20                  25

<210>  66
<211>  17
<212>  PRT

<213>  Artificial Sequence
<220>
<223>  Artificial Sequence 66

<400>  66

Ile His Trp Val Lys Gln Ala His Gly Gln Ser Leu Glu Trp Ile Gly
1               5                   10                  15
Gly

<210>  67
<211>  38
<212>  PRT
<213>  Artificial Sequence
<220>
<223>  Artificial Sequence 67

<400>  67

Val Tyr Asn Gln Lys Phe Gln Gly Lys Ala Thr Leu Thr Val Asp Lys
1               5                   10                  15
Ser Thr Ser Thr Ala Tyr Met Glu Leu Arg Ser Leu Thr Ser Glu Asp
            20                  25                  30
Thr Ala Val Tyr Tyr Cys
        35

<210>  68
<211>  11
<212>  PRT
<213>  Artificial Sequence
<220>
<223>  Artificial Sequence 68

<400>  68

Trp Gly Gln Gly Thr Ser Val Thr Val Ser Ser
1               5                   10

<210>  69
<211>  26
<212>  PRT
<213>  Artificial Sequence
<220>
<223>  Artificial Sequence 69

<400>  69

Asp Ile Gln Met Thr Gln Ser Pro Lys Ser Leu Ser Thr Ser Val Gly
1               5                   10                  15
Asp Arg Val Thr Ile Thr Cys Arg Ala Ser
            20                  25

<210>  70
<211>  17
<212>  PRT
<213>  Artificial Sequence
<220>
<223>  Artificial Sequence 70

<400>  70

Val Ala Trp Tyr Gln Gln Lys Pro Gly Lys Ser Pro Lys Leu Leu Ile
1               5                   10                  15

Tyr

<210> 71
<211> 36
<212> PRT
<213> Artificial Sequence
<220>
<223> Artificial Sequence 71

<400> 71

Tyr Arg Tyr Thr Gly Val Pro Ser Arg Phe Ser Gly Ser Gly Ser Gly
1               5                   10                  15
Thr Asp Phe Thr Phe Thr Ile Ser Ser Val Gln Pro Glu Asp Leu Ala
            20                  25                  30
Thr Tyr Tyr Cys
        35

<210> 72
<211> 10
<212> PRT
<213> Artificial Sequence
<220>
<223> Artificial Sequence 72

<400> 72

Phe Gly Gly Gly Thr Asn Leu Glu Ile Lys
1               5                   10

<210> 73
<211> 25
<212> PRT
<213> Artificial Sequence
<220>
<223> Artificial Sequence 73

<400> 73

Gln Val Gln Leu Val Gln Ser Gly Ala Glu Val Val Lys Pro Gly Ala
1               5                   10                  15
Ser Val Lys Val Ser Cys Lys Thr Ser
            20                  25

<210> 74
<211> 17
<212> PRT
<213> Artificial Sequence
<220>
<223> Artificial Sequence 74

<400> 74

Ile His Trp Val Arg Gln Ala Pro Gly Gln Ser Leu Glu Trp Ile Gly
1               5                   10                  15
Gly

<210> 75
<211> 38
<212> PRT
<213> Artificial Sequence
<220>
<223> Artificial Sequence 75

<400>  75

Asn Tyr Asn Gln Lys Phe Gln Gly Lys Val Thr Leu Thr Val Asp Lys
1               5                   10                  15
Ser Thr Ser Thr Ala Tyr Met Glu Leu Ser Ser Leu Arg Ser Glu Asp
            20                  25                  30
Thr Ala Val Tyr Tyr Cys
        35

<210>  76
<211>  11
<212>  PRT
<213>  Artificial Sequence
<220>
<223>  Artificial Sequence 76

<400>  76

Trp Gly Gln Gly Thr Ser Val Thr Val Ser Ser
1               5                   10

<210>  77
<211>  26
<212>  PRT
<213>  Artificial Sequence
<220>
<223>  Artificial Sequence 77

<400>  77

<210>  78
<211>  17
<212>  PRT
<213>  Artificial Sequence
<220>
<223>  Artificial Sequence 78

<400>  78

Val Ala Trp Tyr Gln Gln Lys Pro Gly Lys Ala Pro Lys Leu Leu Ile
1               5                   10                  15
Tyr

<210>  79
<211>  36
<212>  PRT
<213>  Artificial Sequence
<220>
<223>  Artificial Sequence 79

<400>  79

Ser Arg Tyr Thr Gly Val Pro Ser Arg Phe Ser Gly Ser Gly Ser Gly
1               5                   10                  15
Thr Asp Phe Thr Leu Thr Ile Ser Ser Val Gln Pro Glu Asp Leu Ala
            20                  25                  30
Thr Tyr Tyr Cys

        35

<210>  80
<211>  10
<212>  PRT
<213>  Artificial Sequence
<220>
<223>  Artificial Sequence 80

<400>  80

Phe Gly Gly Gly Thr Asn Leu Glu Ile Lys
1               5                   10

<210>  81
<211>  25
<212>  PRT
<213>  Artificial Sequence
<220>
<223>  Artificial Sequence 81

<400>  81

Gln Val Gln Leu Val Gln Ser Gly Ala Glu Val Val Lys Pro Gly Ala
1               5                   10                  15
Ser Val Lys Val Ser Cys Lys Ala Ser
            20                  25

<210>  82
<211>  17
<212>  PRT
<213>  Artificial Sequence
<220>
<223>  Artificial Sequence 82

<400>  82

Ile His Trp Val Arg Gln Ala Pro Gly Gln Gly Leu Glu Trp Ile Gly
1               5                   10                  15
Gly

<210>  83
<211>  38
<212>  PRT
<213>  Artificial Sequence
<220>
<223>  Artificial Sequence 83

<400>  83

Asn Tyr Ala Gln Lys Phe Gln Gly Arg Val Thr Ile Thr Val Asp Lys
1               5                   10                  15
Ser Thr Ser Thr Ala Tyr Met Glu Leu Ser Ser Leu Arg Ser Glu Asp
            20                  25                  30
Thr Ala Val Tyr Tyr Cys
        35

<210>  84
<211>  11
<212>  PRT
<213>  Artificial Sequence
<220>
<223>  Artificial Sequence 84

<400>  84

Trp Gly Gln Gly Thr Ser Val Thr Val Ser Ser
1               5                   10

<210>  85
<211>  26
<212>  PRT
<213>  Artificial Sequence
<220>
<223>  Artificial Sequence 85

<400>  85

<210>  86
<211>  17
<212>  PRT
<213>  Artificial Sequence
<220>
<223>  Artificial Sequence 86

<400>  86

Leu Ala Trp Tyr Gln Gln Lys Pro Gly Lys Ala Pro Lys Leu Leu Ile
1               5                   10                  15
Tyr

<210>  87
<211>  36
<212>  PRT
<213>  Artificial Sequence
<220>
<223>  Artificial Sequence 87

<400>  87

Ser Leu Tyr Thr Gly Val Pro Ser Arg Phe Ser Gly Ser Gly Ser Gly
1               5                   10                  15
Thr Asp Phe Thr Leu Thr Ile Ser Ser Leu Gln Pro Glu Asp Phe Ala
            20                  25                  30
Thr Tyr Tyr Cys
        35

<210>  88
<211>  10
<212>  PRT
<213>  Artificial Sequence
<220>
<223>  Artificial Sequence 88

<400>  88

Phe Gly Gly Gly Thr Asn Leu Glu Ile Lys
1               5                   10

<210>  89
<211>  25

<212>  PRT
<213>  Artificial Sequence
<220>
<223>  Artificial Sequence 89

<400>  89

Gln Val Gln Leu Val Gln Ser Gly Ala Glu Val Val Lys Pro Gly Ala
1               5                   10                  15
Ser Val Lys Val Ser Cys Lys Thr Ser
            20                  25

<210>  90
<211>  17
<212>  PRT
<213>  Artificial Sequence
<220>
<223>  Artificial Sequence 90

<400>  90

Ile His Trp Val Arg Gln Ala Pro Gly Gln Ser Leu Glu Trp Ile Gly
1               5                   10                  15
Gly

<210>  91
<211>  38
<212>  PRT
<213>  Artificial Sequence
<220>
<223>  Artificial Sequence 91

<400>  91

Val Tyr Asn Gln Lys Phe Gln Gly Lys Val Thr Leu Thr Val Asp Lys
1               5                   10                  15
Ser Thr Ser Thr Ala Tyr Met Glu Leu Ser Ser Leu Arg Ser Glu Asp
            20                  25                  30
Thr Ala Val Tyr Tyr Cys
        35

<210>  92
<211>  11
<212>  PRT
<213>  Artificial Sequence
<220>
<223>  Artificial Sequence 92

<400>  92

Trp Gly Gln Gly Thr Ser Val Thr Val Ser Ser
1               5                   10

<210>  93
<211>  26
<212>  PRT
<213>  Artificial Sequence
<220>
<223>  Artificial Sequence 93

<400>  93

<210>  94
<211>  17
<212>  PRT
<213>  Artificial Sequence
<220>
<223>  Artificial Sequence 94

<400>  94

Val Ala Trp Tyr Gln Gln Lys Pro Gly Lys Ala Pro Lys Leu Leu Ile
1               5                   10                  15
Tyr

<210>  95
<211>  36
<212>  PRT
<213>  Artificial Sequence
<220>
<223>  Artificial Sequence 95

<400>  95

Tyr Arg Tyr Thr Gly Val Pro Ser Arg Phe Ser Gly Ser Gly Ser Gly
1               5                   10                  15
Thr Asp Phe Thr Leu Thr Ile Ser Ser Val Gln Pro Glu Asp Leu Ala
            20                  25                  30
Thr Tyr Tyr Cys
        35

<210>  96
<211>  10
<212>  PRT
<213>  Artificial Sequence
<220>
<223>  Artificial Sequence 96

<400>  96

Phe Gly Gly Gly Thr Asn Leu Glu Ile Lys
1               5                   10

<210>  97
<211>  25
<212>  PRT
<213>  Artificial Sequence
<220>
<223>  Artificial Sequence 97

<400>  97

Asp Val Gln Leu Val Glu Ser Gly Gly Gly Leu Val Gln Leu Gly Gly
1               5                   10                  15
Ser Leu Arg Leu Ser Cys Ala Ala Ser
            20                  25

<210>  98
<211>  17
<212>  PRT
<213>  Artificial Sequence

<220>
<223>  Artificial Sequence 98

<400>  98

Met Ser Trp Val Arg Gln Thr Pro Glu Lys Arg Leu Glu Leu Val Ala
1               5                   10                  15
Ala

<210>  99
<211>  38
<212>  PRT
<213>  Artificial Sequence
<220>
<223>  Artificial Sequence 99

<400>  99

Tyr Tyr Ala Asp Ser Val Lys Gly Arg Phe Thr Ile Ser Arg Asp Asn
1               5                   10                  15
Ser Lys Asn Thr Leu Tyr Leu Gln Met Ser Ser Leu Arg Ala Glu Asp
            20                  25                  30
Thr Ala Val Tyr Tyr Cys
        35

<210>  100
<211>  11
<212>  PRT
<213>  Artificial Sequence
<220>
<223>  Artificial Sequence 100

<400>  100

Trp Gly Gln Gly Thr Ser Val Thr Val Ser Ser
1               5                   10

<210>  101
<211>  26
<212>  PRT
<213>  Artificial Sequence
<220>
<223>  Artificial Sequence 101

<400>  101

Asp Ile Gln Met Thr Gln Ser Pro Ser Ser Leu Ser Thr Ser Val Gly
1               5                   10                  15
Asp Arg Val Thr Ile Thr Cys Lys Ala Ser
            20                  25

<210>  102
<211>  17
<212>  PRT
<213>  Artificial Sequence
<220>
<223>  Artificial Sequence 102

<400>  102

Val Val Trp Tyr Gln Gln Lys Pro Gly Lys Ser Pro Lys Leu Leu Ile
1               5                   10                  15
Tyr

<210>  103
<211>  36
<212>  PRT
<213>  Artificial Sequence
<220>
<223>  Artificial Sequence 103

<400>  103

Tyr Arg Tyr Thr Gly Val Pro Ser Arg Phe Ser Gly Ser Gly Ser Gly
1               5                   10                  15
Thr Asp Phe Thr Phe Thr Ile Ser Ser Val Gln Pro Glu Asp Ile Ala
            20                  25                  30
Thr Tyr Tyr Cys
        35

<210>  104
<211>  10
<212>  PRT
<213>  Artificial Sequence
<220>
<223>  Artificial Sequence 104

<400>  104

Phe Gly Gly Gly Thr Lys Leu Glu Ile Lys
1               5                   10

<210>  105
<211>  25
<212>  PRT
<213>  Artificial Sequence
<220>
<223>  Artificial Sequence 105

<400>  105

<210>  106
<211>  17
<212>  PRT
<213>  Artificial Sequence
<220>
<223>  Artificial Sequence 106

<400>  106

Met Ser Trp Val Arg Gln Ala Pro Gly Lys Arg Leu Glu Leu Val Ala
1               5                   10                  15
Ala

<210>  107
<211>  38
<212>  PRT
<213>  Artificial Sequence
<220>
<223>  Artificial Sequence 107

<400>  107

Tyr Tyr Ala Asp Ser Val Lys Gly Arg Phe Thr Ile Ser Arg Asp Asn
1               5                   10                  15
Ser Lys Asn Thr Leu Tyr Leu Gln Met Ser Ser Leu Arg Ala Glu Asp
            20                  25                  30
Thr Ala Val Tyr Tyr Cys
        35

<210>  108
<211>  11
<212>  PRT
<213>  Artificial Sequence
<220>
<223>  Artificial Sequence 108

<400>  108

Trp Gly Gln Gly Thr Ser Val Thr Val Ser Ser
1               5                   10

<210>  109
<211>  26
<212>  PRT
<213>  Artificial Sequence
<220>
<223>  Artificial Sequence 109

<400>  109

Asp Ile Gln Met Thr Gln Ser Pro Ser Ser Leu Ser Ala Ser Val Gly
1               5                   10                  15
Asp Arg Val Thr Ile Thr Cys Gln Ala Ser
            20                  25

<210>  110
<211>  17
<212>  PRT
<213>  Artificial Sequence
<220>
<223>  Artificial Sequence 110

<400>  110

Val Val Trp Tyr Gln Gln Lys Pro Gly Lys Ser Pro Lys Leu Leu Ile
1               5                   10                  15
Tyr

<210>  111
<211>  36
<212>  PRT
<213>  Artificial Sequence
<220>
<223>  Artificial Sequence 111

<400>  111

Tyr Leu Tyr Thr Gly Val Pro Ser Arg Phe Ser Gly Ser Gly Ser Gly
1               5                   10                  15
Thr Asp Phe Thr Phe Thr Ile Ser Ser Leu Gln Pro Glu Asp Ile Ala
            20                  25                  30
Thr Tyr Tyr Cys
        35

<210>  112
<211>  10
<212>  PRT
<213>  Artificial Sequence
<220>
<223>  Artificial Sequence 112

<400>  112

Phe Gly Gly Gly Thr Lys Leu Glu Ile Lys
1               5                   10

<210>  113
<211>  25
<212>  PRT
<213>  Artificial Sequence
<220>
<223>  Artificial Sequence 113

<400>  113

<210>  114
<211>  17
<212>  PRT
<213>  Artificial Sequence
<220>
<223>  Artificial Sequence 114

<400>  114

Met Ser Trp Val Arg Gln Ala Pro Gly Lys Gly Leu Glu Tyr Val Ser
1               5                   10                  15
Ala

<210>  115
<211>  38
<212>  PRT
<213>  Artificial Sequence
<220>
<223>  Artificial Sequence 115

<400>  115

Tyr Tyr Ala Asp Ser Val Lys Gly Arg Phe Thr Ile Ser Arg Asp Asn
1               5                   10                  15
Ser Lys Asn Thr Leu Tyr Leu Gln Met Ser Ser Leu Arg Ala Glu Asp
            20                  25                  30
Thr Ala Val Tyr Tyr Cys
        35

<210>  116
<211>  11
<212>  PRT
<213>  Artificial Sequence
<220>
<223>  Artificial Sequence 116

<400>  116

Trp Gly Gln Gly Thr Ser Val Thr Val Ser Ser
1               5                   10

<210>  117
<211>  26
<212>  PRT
<213>  Artificial Sequence
<220>
<223>  Artificial Sequence 117

<400>  117

Asp Ile Gln Met Thr Gln Ser Pro Ser Ser Leu Ser Ala Ser Val Gly
1               5                   10                  15
Asp Arg Val Thr Ile Thr Cys Gln Ala Ser
            20                  25

<210>  118
<211>  17
<212>  PRT
<213>  Artificial Sequence
<220>
<223>  Artificial Sequence 118

<400>  118

Leu Ala Trp Tyr Gln Gln Lys Pro Gly Lys Ala Pro Lys Leu Leu Ile
1               5                   10                  15
Tyr

<210>  119
<211>  36
<212>  PRT
<213>  Artificial Sequence
<220>
<223>  Artificial Sequence 119

<400>  119

Asn Leu Glu Thr Gly Val Pro Ser Arg Phe Ser Gly Ser Gly Ser Gly
1               5                   10                  15
Thr Asp Phe Thr Phe Thr Ile Ser Ser Leu Gln Pro Glu Asp Ile Ala
            20                  25                  30
Thr Tyr Tyr Cys
        35

<210>  120
<211>  10
<212>  PRT
<213>  Artificial Sequence
<220>
<223>  Artificial Sequence 120

<400>  120

Phe Gly Gly Gly Thr Lys Leu Glu Ile Lys
1               5                   10

<210>  121
<211>  232
<212>  PRT

<213>  Artificial Sequence
<220>
<223>  Artificial Sequence 121

<400>  121

Pro Arg Gly Pro Thr Ile Lys Pro Cys Pro Pro Cys Lys Cys Pro Ala
1               5                   10                  15
Pro Asn Leu Leu Gly Gly Pro Ser Val Phe Ile Phe Pro Pro Lys Ile
            20                  25                  30
Lys Asp Val Leu Met Ile Ser Leu Ser Pro Ile Val Thr Cys Val Val
        35                  40                  45
Val Asp Val Ser Glu Asp Asp Pro Asp Val Gln Ile Ser Trp Phe Val
    50                  55                  60
Asn Asn Val Glu Val His Thr Ala Gln Thr Gln Thr His Arg Glu Asp
65                  70                  75                  80
Tyr Asn Ser Thr Leu Arg Val Val Ser Ala Leu Pro Ile Gln His Gln
                85                  90                  95
Asp Trp Met Ser Gly Lys Glu Phe Lys Cys Lys Val Asn Asn Lys Asp
            100                 105                 110
Leu Pro Ala Pro Ile Glu Arg Thr Ile Ser Lys Pro Lys Gly Ser Val
        115                 120                 125
Arg Ala Pro Gln Val Tyr Val Leu Pro Pro Pro Glu Glu Glu Met Thr
    130                 135                 140
Lys Lys Gln Val Thr Leu Thr Cys Met Val Thr Asp Phe Met Pro Glu
145                 150                 155                 160
Asp Ile Tyr Val Glu Trp Thr Asn Asn Gly Lys Thr Glu Leu Asn Tyr
                165                 170                 175
Lys Asn Thr Glu Pro Val Leu Asp Ser Asp Gly Ser Tyr Phe Met Tyr
            180                 185                 190
Ser Lys Leu Arg Val Glu Lys Lys Asn Trp Val Glu Arg Asn Ser Tyr
        195                 200                 205
Ser Cys Ser Val Val His Glu Gly Leu His Asn His His Thr Thr Lys
    210                 215                 220
Ser Phe Ser Arg Thr Pro Gly Lys
225                 230

<210>  122
<211>  443
<212>  PRT
<213>  Artificial Sequence
<220>
<223>  Artificial Sequence 122

<400>  122

Glu Val Gln Leu Glu Gln Ser Gly Ala Glu Leu Met Lys Pro Gly Ala
1               5                   10                  15
Ser Val Lys Ile Ser Cys Lys Ala Thr Gly Tyr Thr Phe Thr Thr Tyr
            20                  25                  30
Trp Ile Glu Trp Ile Lys Gln Arg Pro Gly His Ser Leu Glu Trp Ile
        35                  40                  45
Gly Glu Ile Leu Pro Gly Ser Asp Ser Thr Tyr Tyr Asn Glu Lys Val
    50                  55                  60
Lys Gly Lys Val Thr Phe Thr Ala Asp Ala Ser Ser Asn Thr Ala Tyr
65                  70                  75                  80
Met Gln Leu Ser Ser Leu Thr Ser Glu Asp Ser Ala Val Tyr Tyr Cys
                85                  90                  95
Ala Arg Gly Asp Gly Phe Tyr Val Tyr Trp Gly Gln Gly Thr Thr Leu
            100                 105                 110
Thr Val Ser Ser Ala Ser Thr Lys Gly Pro Ser Val Phe Pro Leu Ala
        115                 120                 125
Pro Cys Ser Arg Ser Thr Ser Glu Ser Thr Ala Ala Leu Gly Cys Leu

130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Lys Thr Tyr Thr Cys Asn Val Asp His Lys Pro Ser Asn Thr
        195                 200                 205
Lys Val Asp Lys Arg Val Glu Ser Lys Tyr Gly Pro Pro Cys Pro Pro
    210                 215                 220
Cys Pro Ala Pro Glu Phe Leu Gly Gly Pro Ser Val Phe Leu Phe Pro
225                 230                 235                 240
Pro Lys Pro Lys Asp Thr Leu Met Ile Ser Arg Thr Pro Glu Val Thr
                245                 250                 255
Cys Val Val Val Asp Val Ser Gln Glu Asp Pro Glu Val Gln Phe Asn
            260                 265                 270
Trp Tyr Val Asp Gly Val Glu Val His Asn Ala Lys Thr Lys Pro Arg
        275                 280                 285
Glu Glu Gln Phe Asn Ser Thr Tyr Arg Val Val Ser Val Leu Thr Val
    290                 295                 300
Leu His Gln Asp Trp Leu Asn Gly Lys Glu Tyr Lys Cys Lys Val Ser
305                 310                 315                 320
Asn Lys Gly Leu Pro Ser Ser Ile Glu Lys Thr Ile Ser Lys Ala Lys
                325                 330                 335
Gly Gln Pro Arg Glu Pro Gln Val Tyr Thr Leu Pro Pro Ser Gln Glu
            340                 345                 350
Glu Met Thr Lys Asn Gln Val Ser Leu Thr Cys Leu Val Lys Gly Phe
        355                 360                 365
Tyr Pro Ser Asp Ile Ala Val Glu Trp Glu Ser Asn Gly Gln Pro Glu
    370                 375                 380
Asn Asn Tyr Lys Thr Thr Pro Pro Val Leu Asp Ser Asp Gly Ser Phe
385                 390                 395                 400
Phe Leu Tyr Ser Arg Leu Thr Val Asp Lys Ser Arg Trp Gln Glu Gly
                405                 410                 415
Asn Val Phe Ser Cys Ser Val Met His Glu Ala Leu His Asn His Tyr
            420                 425                 430
Thr Gln Lys Ser Leu Ser Leu Ser Leu Gly Lys
        435                 440

<210>  123
<211>  214
<212>  PRT
<213>  Artificial Sequence
<220>
<223>  Artificial Sequence 123

<400>  123

Asp Ile Glu Leu Thr Gln Ser Pro Ala Thr Leu Ser Val Thr Pro Gly
1               5                   10                  15
Asp Ser Val Ser Leu Ser Cys Arg Ala Ser Gln Ser Ile Ser Asn Asn
            20                  25                  30
Leu His Trp Tyr Gln Gln Lys Ser His Glu Ser Pro Arg Leu Leu Ile
        35                  40                  45
Lys Tyr Thr Ser Gln Ser Met Ser Gly Ile Pro Ser Arg Phe Ser Gly
    50                  55                  60
Ser Gly Ser Gly Thr Asp Phe Thr Leu Ser Ile Asn Ser Val Glu Thr
65                  70                  75                  80
Glu Asp Phe Gly Val Tyr Phe Cys Gln Gln Ser Gly Ser Trp Pro Arg
                85                  90                  95
Thr Phe Gly Gly Gly Thr Lys Leu Asp Ile Lys Arg Thr Val Ala Ala
            100                 105                 110

Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210> 124
<211> 118
<212> PRT
<213> Artificial Sequence
<220>
<223> Artificial Sequence 124

<400> 124

Glu Val Gln Leu Val Glu Ser Gly Gly Gly Leu Val Gln Pro Gly Arg
1               5                   10                  15
Ser Leu Arg Leu Ser Cys Glu Ala Ser Gly Phe Thr Phe Asp Asp Tyr
            20                  25                  30
Ala Met His Trp Val Arg Gln Ala Pro Gly Lys Gly Leu Glu Trp Val
        35                  40                  45
Ser Gly Leu Ser Arg Thr Ser Val Ser Ile Gly Tyr Ala Asp Ser Val
    50                  55                  60
Lys Gly Arg Phe Thr Ile Ser Arg Asp Asn Ala Lys Asn Ser Leu Tyr
65                  70                  75                  80
Leu Glu Met Asn Ser Leu Arg Pro Glu Asp Thr Ala Leu Tyr Tyr Cys
                85                  90                  95
Ala Lys Trp Gly Thr Arg Gly Tyr Phe Asp Tyr Trp Gly Gln Gly Thr
            100                 105                 110
Leu Val Thr Val Ser Ser
        115

<210> 125
<211> 107
<212> PRT
<213> Artificial Sequence
<220>
<223> Artificial Sequence 125

<400> 125

Asp Ile Gln Met Thr Gln Ser Pro Ser Ser Val Ser Ala Ser Val Gly
1               5                   10                  15
Asp Arg Val Thr Ile Thr Cys Arg Ala Ser Gln Asp Ile Ser Ile Trp
            20                  25                  30
Leu Ala Trp Tyr Gln Gln Ser Pro Gly Lys Ala Pro Lys Leu Leu Ile
        35                  40                  45
Asn Val Ala Ser Arg Leu Gln Ser Gly Val Pro Ser Arg Phe Ser Gly
    50                  55                  60
Ser Gly Ser Gly Thr Asp Phe Thr Leu Thr Ile Asn Ser Leu Gln Pro
65                  70                  75                  80
Glu Asp Phe Val Thr Tyr Tyr Cys Gln Gln Ala Asn Ser Phe Pro Ile
                85                  90                  95
Thr Phe Gly Gln Gly Thr Arg Leu Ala Thr Lys

100                 105

<210>  126
<211>  360
<212>  DNA
<213>  Artificial Sequence
<220>
<223>  Artificial Sequence 126

<400>  126

caggtgacac tgaaggagtc cggcccaacc ctggtgaagc ccacccagac actgaccctg      60
acatgtacct tcagcggctt ttctctgagc acatccggca tgggagtggg atggatcaga     120
cagccccctg gcaaggccct ggagtggctg gccctgatct ggtgggccga cgataagagg     180
tactctacca gcctgaagtc ccgcctgaca atctccaagg acacctctaa gaaccaggtg     240
gtgctgacaa tcaccaatgt ggaccccgtg gatacagcca cctactattg cgcccggatc     300
acaagaggca actctgccat ggatttttgg ggccagggca catctgtgac cgtgagctcc     360

<210>  127
<211>  120
<212>  PRT
<213>  Artificial Sequence
<220>
<223>  Artificial Sequence 127

<400>  127

Gln Val Thr Leu Lys Glu Ser Gly Pro Thr Leu Val Lys Pro Thr Gln
1               5                   10                  15
Thr Leu Thr Leu Thr Cys Thr Phe Ser Gly Phe Ser Leu Ser Thr Ser
            20                  25                  30
Gly Met Gly Val Gly Trp Ile Arg Gln Pro Pro Gly Lys Ala Leu Glu
        35                  40                  45
Trp Leu Ala Leu Ile Trp Trp Ala Asp Asp Lys Arg Tyr Ser Thr Ser
    50                  55                  60
Leu Lys Ser Arg Leu Thr Ile Ser Lys Asp Thr Ser Lys Asn Gln Val
65                  70                  75                  80
Val Leu Thr Ile Thr Asn Val Asp Pro Val Asp Thr Ala Thr Tyr Tyr
                85                  90                  95
Cys Ala Arg Ile Thr Arg Gly Asn Ser Ala Met Asp Phe Trp Gly Gln
            100                 105                 110
Gly Thr Ser Val Thr Val Ser Ser
        115                 120

<210>  128
<211>  321
<212>  DNA
<213>  Artificial Sequence
<220>
<223>  Artificial Sequence 128

<400>  128

gacatccaga tgacccagtc tcctagctcc ctgtccgcct ctgtgggcga tagggtgacc      60
atcacatgta gagcctctga gaacgtgtac agctatctga attggtacca gcagaagccc     120
ggcaaggccc ctaagctgct gatctataac gccaagagcc tgcagtccgg agtgccaagc     180
cggttcagcg gctccggctc tggcaccgac tttaccctga caatctctag cctgcagcca     240
gaggatttcg ccacatacta ttgccagcac cactacggca tcccctggac cttcggcggc     300
ggcacaaagc tggagatcaa g                                               321

<210>  129
<211>  107
<212>  PRT

<213>  Artificial Sequence
<220>
<223>  Artificial Sequence 129

<400>  129

Asp Ile Gln Met Thr Gln Ser Pro Ser Ser Leu Ser Ala Ser Val Gly
1               5                   10                  15
Asp Arg Val Thr Ile Thr Cys Arg Ala Ser Glu Asn Val Tyr Ser Tyr
            20                  25                  30
Leu Asn Trp Tyr Gln Gln Lys Pro Gly Lys Ala Pro Lys Leu Leu Ile
        35                  40                  45
Tyr Asn Ala Lys Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln His His Tyr Gly Ile Pro Trp
                85                  90                  95
Thr Phe Gly Gly Gly Thr Lys Leu Glu Ile Lys
            100                 105

<210>  130
<211>  10
<212>  PRT
<213>  Artificial Sequence
<220>
<223>  Artificial Sequence 130

<400>  130

Gly Phe Ser Leu Ser Thr Ser Gly Met Gly
1               5                   10

<210>  131
<211>  7
<212>  PRT
<213>  Artificial Sequence
<220>
<223>  Artificial Sequence 131

<400>  131

Ile Trp Trp Ala Asp Asp Lys
1               5

<210>  132
<211>  12
<212>  PRT
<213>  Artificial Sequence
<220>
<223>  Artificial Sequence 132

<400>  132

Ala Arg Ile Thr Arg Gly Asn Ser Ala Met Asp Phe
1               5                   10

<210>  133
<211>  6
<212>  PRT
<213>  Artificial Sequence
<220>
<223>  Artificial Sequence 133

<400>  133

Glu Asn Val Tyr Ser Tyr
1               5

<210>  134
<211>  3
<212>  PRT
<213>  Artificial Sequence
<220>
<223>  Artificial Sequence 134

<400>  134

Asn Ala Lys
1

<210>  135
<211>  9
<212>  PRT
<213>  Artificial Sequence
<220>
<223>  Artificial Sequence 135

<400>  135

Gln His His Tyr Gly Ile Pro Trp Thr
1               5

<210>  136
<211>  25
<212>  PRT
<213>  Artificial Sequence
<220>
<223>  Artificial Sequence 136

<400>  136

Gln Val Thr Leu Lys Glu Ser Gly Pro Thr Leu Val Lys Pro Thr Gln
1               5                   10                  15
Thr Leu Thr Leu Thr Cys Thr Phe Ser
            20                  25

<210>  137
<211>  17
<212>  PRT
<213>  Artificial Sequence
<220>
<223>  Artificial Sequence 137

<400>  137

Val Gly Trp Ile Arg Gln Pro Pro Gly Lys Ala Leu Glu Trp Leu Ala
1               5                   10                  15
Leu

<210>  138
<211>  38
<212>  PRT
<213>  Artificial Sequence
<220>
<223>  Artificial Sequence 138

<400>  138

Arg Tyr Ser Thr Ser Leu Lys Ser Arg Leu Thr Ile Ser Lys Asp Thr
1               5                   10                  15
Ser Lys Asn Gln Val Val Leu Thr Ile Thr Asn Val Asp Pro Val Asp
            20                  25                  30
Thr Ala Thr Tyr Tyr Cys
        35

<210>  139
<211>  11
<212>  PRT
<213>  Artificial Sequence
<220>
<223>  Artificial Sequence 139

<400>  139

Trp Gly Gln Gly Thr Ser Val Thr Val Ser Ser
1               5                   10

<210>  140
<211>  26
<212>  PRT
<213>  Artificial Sequence
<220>
<223>  Artificial Sequence 140

<400>  140

<210>  141
<211>  17
<212>  PRT
<213>  Artificial Sequence
<220>
<223>  Artificial Sequence 141

<400>  141

Leu Asn Trp Tyr Gln Gln Lys Pro Gly Lys Ala Pro Lys Leu Leu Ile
1               5                   10                  15
Tyr

<210>  142
<211>  36
<212>  PRT
<213>  Artificial Sequence
<220>
<223>  Artificial Sequence 142

<400>  142

Ser Leu Gln Ser Gly Val Pro Ser Arg Phe Ser Gly Ser Gly Ser Gly
1               5                   10                  15
Thr Asp Phe Thr Leu Thr Ile Ser Ser Leu Gln Pro Glu Asp Phe Ala
            20                  25                  30
Thr Tyr Tyr Cys

        35

<210> 143
<211> 10
<212> PRT
<213> Artificial Sequence
<220>
<223> Artificial Sequence 143

<400> 143

Phe Gly Gly Gly Thr Lys Leu Glu Ile Lys
1               5                   10

## Description

### TECHNICAL FIELD

The present invention relates to the field of allergic disease treatment and molecular immunology, and particularly, to an anti-human interleukin-4-receptor A antibody, a pharmaceutical composition or a kit comprising the same, and use thereof.

### BACKGROUND

Interleukin-4-receptor (IL-4R) is a transmembrane receptor that exists in two distinct forms: type I IL-4R, which consists of an IL-4R α subunit (referred to as IL-4RA herein) with high affinity and a γ_{c} subunit with moderate affinity, and binds to interleukin-4 (IL-4) and mediates IL-4 induced cell proliferation, activation and other biological functions; and type II IL-4R, which consists of an IL-4R α subunit (IL-4RA) with high affinity and an interleukin-13-receptor α subunit (IL-13Rα), and is a homologous functional receptor for interleukin-13 (IL-13) capable of binding to IL-13 (Wang and Secombes, Cytokine, 2015, 75(1):8-13). IL-4R is expressed in T cells, B cells, hematopoietic stem cells and endothelial cells, epithelial cells, muscle, fibroblasts, hepatocytes, and brain tissue. IL-4RA, along with γ_{c} subunit or IL-13R α subunit, activates various non-receptor protein tyrosine kinases in cytoplasm after IL-4 binds to its receptor, further initiating downstream signal transduction pathways (Nelms et al., Annu. Rev. Immunol., 1999, 17:701-738; LaPorte et al., Cell, 2008, 132(2):259-272).

IL-4RA can bind to IL-4 and IL-13 with high affinity, and is a major functional subunit of the foregoing type I and type II IL-4R. The inhibition of IL-4RA can effectively block relevant biological functions mediated by IL-4 and IL-13 (Gessner et al., Immunobiology, 2000, 201:285).

IL-4 is a pleiotropic cytokine secreted by immune cells such as CD4⁺ T cell subsets, B cells, mast cells and the like; IL-13 is produced mainly by activated T cells (Th2 in mice) in human, and has various biological effects on monocytes (macrophages), B lymphocytes, NK cells, vascular endothelial cells and the like. *In vitro* studies have shown that IL-4 and IL-13 can exert corresponding effector functions in a variety of cells, such as T cells, B cells, eosinophils, mast cells, basophils, airway smooth muscle cells, respiratory tract epithelial cells, fibroblasts and endothelial cells, which are the major effector cells responsible for the development of allergic diseases such as allergic rhinitis and food allergy, and asthma (May et al., Cytokine, 2015, 75(1):89-116); furthermore, airway smooth muscle cells, respiratory tract epithelial cells, fibroblasts and endothelial cells, etc. are also involved in the development, progression and maintenance of chronic obstructive pulmonary disease (for reviews, see Steinke et al., RespRes, 2001, 2(66):66-70 and Willis-Karp et al., Immunol Rev, 2004, 202:175-190); T and B cell dysfunctions are the major mechanisms of autoimmune diseases, and IL-4 is involved in the development of autoimmune diseases by activating downstream signaling pathways through binding to IL-4R on the surface of immune cells (May et al., Cytokine, 2015, 75(1):89-116).

The IL-4/IL-13 pathways play an important role in asthma pathology (Chatila et al., Trends in Molecular Med., 2004, 10(10):493-499) as well as in other diseases outlined elsewhere herein. Airway hyperreactivity, mucus hypersecretion, and airway remodeling, etc. are the key pathological features of asthma. Studies have demonstrated that IL-4 and IL-13 are involved in the development and maintenance of the above pathological processes (May et al., Cytokine, 2015, 75(1):89-116). IL-13 is considered as a key cytokine for triggering airway hyperreactivity (AHR), and IL-4 is a major cause of Th2 immune cell polarization and IgE production (Wynn et al., Annu. Rev. Immunol., 2003, 21:425-456).

Atopic dermatitis, especially moderate and severe atopic dermatitis, is a serious chronic inflammatory skin disease, mainly characterized by severe itching, pronounced eczematous changes and dry skin. Atopic dermatitis usually starts in infants, and continues for a lifetime in some patients. It may seriously affect the quality of life of the patients due to chronic recurrent eczematous rash, severe itching, sleep deficit, dietary restrictions, and psychosocial reasons. IL-4/IL-13 are considered as a key driver of the persistent intrinsic inflammation of atopic dermatitis (Malajian et al., Cytokine, 2015, 73(2):311-318). Clinical studies have demonstrated that dupilumab, an anti-human IL-4RA monoclonal antibody, is effective in treating moderate and severe atopic dermatitis (Beck et al., N. Engl. J. Med., 2014, 371(2):130-139) and has been approved by the U.S. Food and Drug Administration (FDA) for treating moderate and severe atopic dermatitis.

In addition, basic medical researches and clinical researches also proved that IL-4, IL-13 and IL-4R pathways are involved in the development and progression of chronic obstructive pulmonary disease, rhinosinusitis and tumors, and the inhibition of IL-4RA has the potential for treating chronic obstructive pulmonary disease (Jin Lin et al., Journal of Practical Medicine, 2014, 30(22):3543-3544), rhinosinusitis, pulmonary fibrosis and tumors (May et al., Cytokine, 2015, 75(1):89-116; Guo Changkuo et al., Chemistry of Life, 2017, 37(3):413-418). In adult patients with symptomatic chronic rhinosinusitis and nasal polyposis refractory to intranasal glucocorticoid, anti-human IL-4RA monoclonal antibody dupilumab in combination with glucocorticoids can significantly ameliorate symptoms and reduce nasal polyps (Bachert et al., JAMA, 2016, 315(5):469-79).

The anti-human IL-4RA antibody medicament has wide application prospect, and can be used for treating allergic diseases, such as allergic rhinitis, asthma, allergy and atopic dermatitis, while treating rhinosinusitis, nasal polyps, chronic obstructive pulmonary disease, tissue fibrosis, tumor, autoimmune diseases and the like. Therefore, the development of antibody medicaments with high affinity to human IL-4RA for treating allergic diseases with better efficacy and less severe toxic and side effects poses great significance. However, available anti-human IL-4RA antibody medicaments showed insufficient affinities, and there's still a need for an anti-human IL-4RA antibody with high affinity.

### SUMMARY

After intensive studies and creative efforts, the inventors used mammalian cell expression systems to express recombinant IL-4RA as an antigen to immunize mice, and obtained hybridoma cells by fusion of mouse spleen cells and myeloma cells. The inventors obtained the following hybridoma cell lines by screening a large number of the samples.

The inventor found that:
The hybridoma cell line 13E5 is capable of secreting and producing a specific antibody (named as 13E5) that specifically binds to the human IL-4RA, and the antibody can block the binding of human IL-4RA to IL-4 effectively.

Furthermore, the inventors have creatively prepared humanized anti-human IL-4RA antibodies (named as 13E5 H1L1, 13E5 H2L2, 13E5 H3L3, 13E5 H4L4 and 13E5 H4L2, respectively).

The hybridoma cell line 18H7 is capable of secreting and producing a specific antibody (named as 18H7) that specifically binds to the human IL-4RA, and the antibody can block the binding of human IL-4RA to IL-4 effectively.

Furthermore, the inventors have creatively prepared humanized anti-human IL-4RA antibodies (named as 18H7 H1L1, 18H7 H2L2, 18H7 H2L3 and 18H7 H3L2, respectively).

The hybridoma cell line 20G10 is capable of secreting and producing a specific antibody (named as 20G10) that specifically binds to the human IL-4RA, and the antibody can block the binding of human IL-4RA and IL-4 effectively.

Furthermore, the inventors have creatively prepared a humanized anti-human IL-4RA antibody (named as 20G10 H3L3).

The foregoing antibodies can effectively bind to the human IL-4RA, block the binding of human IL-4RA to the ligand IL-4 or IL-13 thereof, and inhibit the activation of the downstream signaling pathway of the human IL-4 RA. The antibodies have potential in preparing a medicament for preventing and treating allergic rhinitis, asthma, allergy, atopic dermatitis, rhinosinusitis, nasal polyps, chronic obstructive pulmonary disease, tissue fibrosis and autoimmune diseases.

The present invention is detailed below.

One aspect of the present invention relates to an antibody or an antigen-binding fragment thereof, the antibody comprising:
an HCDR1, comprising or consisting of the sequence set forth in SEQ ID NO: 9, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence,
an HCDR2, comprising or consisting of the sequence set forth in SEQ ID NO: 10, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence, and
an HCDR3, comprising or consisting of the sequence set forth in SEQ ID NO: 11, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence, and the antibody further comprising:
an LCDR1, comprising or consisting of the sequence set forth in SEQ ID NO: 12, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence,
an LCDR2, comprising or consisting of the sequence set forth in SEQ ID NO: 13, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence, and
an LCDR3, comprising or consisting of the sequence set forth in SEQ ID NO: 14, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence;
   or,
the antibody comprising:
an HCDR1, comprising or consisting of the sequence set forth in SEQ ID NO: 15, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence,
an HCDR2, comprising or consisting of the sequence set forth in SEQ ID NO: 16, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence, and
an HCDR3, comprising or consisting of the sequence set forth in SEQ ID NO: 17, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence,
and the antibody further comprising:
an LCDR1, comprising or consisting of the sequence set forth in SEQ ID NO: 18, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence,
an LCDR2, comprising or consisting of the sequence set forth in SEQ ID NO: 19, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence, and
an LCDR3, comprising or consisting of the sequence set forth in SEQ ID NO: 20, a sequence having at least 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared to the sequence;
   or,
the antibody comprising:
an HCDR1, comprising or consisting of the sequence set forth in SEQ ID NO: 130, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
an HCDR2, comprising or consisting of the sequence set forth in SEQ ID NO: 131, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence, and
an HCDR3, comprising or consisting of the sequence set forth in SEQ ID NO: 132, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
and the antibody further comprising:
an LCDR1, comprising or consisting of the sequence set forth in SEQ ID NO: 133, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
an LCDR2, comprising or consisting of the sequence set forth in SEQ ID NO: 134, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence, and
an LCDR3, comprising or consisting of the sequence set forth in SEQ ID NO: 135, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence.

In one embodiment of the present invention, the antibody comprises:
(1) a heavy chain variable region comprising or consisting of:
   the amino acid sequence set forth in SEQ ID NO: 2,
   a sequence having at least 80%, 85% or 90%, preferably at least 91%, 92%, 93%, 94%, 95%, 96%, 97%, 98% or 99% sequence identity to the sequence set forth in SEQ ID NO: 2, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 2, and
   a light chain variable region comprising or consisting of:
      the amino acid sequence set forth in SEQ ID NO: 4,
      a sequence having at least 80%, 85% or 90%, preferably at least 91%, 92%, 93%, 94%, 95%, 96%, 97%, 98% or 99% sequence identity to the sequence set forth in SEQ ID NO: 4, or
      an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 4;
(2) a heavy chain variable region comprising or consisting of:
   the amino acid sequence set forth in SEQ ID NO: 6,
   a sequence having at least 80%, 85% or 90%, preferably at least 91%, 92%, 93%, 94%, 95%, 96%, 97%, 98% or 99% sequence identity to the sequence set forth in SEQ ID NO: 6, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 6, and
   a light chain variable region comprising or consisting of:
   the amino acid sequence set forth in SEQ ID NO: 8,
   a sequence having at least 80%, 85% or 90%, preferably at least 91%, 92%, 93%, 94%, 95%, 96%, 97%, 98% or 99% sequence identity to the sequence set forth in SEQ ID NO: 8, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 8;
(3) a heavy chain variable region comprising or consisting of:
   the amino acid sequence set forth in SEQ ID NO: 22,
   a sequence having at least 80%, 85% or 90%, preferably at least 91%, 92%, 93%, 94%, 95%, 96%, 97%, 98% or 99% sequence identity to the sequence set forth in SEQ ID NO: 22, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 22, and
   a light chain variable region comprising or consisting of:
      the amino acid sequence set forth in SEQ ID NO: 24,
      a sequence having at least 80%, 85% or 90%, preferably at least 91%, 92%, 93%, 94%, 95%, 96%, 97%, 98% or 99% sequence identity to the sequence set forth in SEQ ID NO: 24, or
      an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 24;
(4) a heavy chain variable region comprising or consisting of:
   the amino acid sequence set forth in SEQ ID NO: 26,
   a sequence having at least 80%, 85% or 90%, preferably at least 91%, 92%, 93%, 94%, 95%, 96%, 97%, 98% or 99% sequence identity to the sequence set forth in SEQ ID NO: 26, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 26, and
   a light chain variable region comprising or consisting of:
      the amino acid sequence set forth in SEQ ID NO: 28,
      a sequence having at least 80%, 85% or 90%, preferably at least 91%, 92%, 93%, 94%, 95%, 96%, 97%, 98% or 99% sequence identity to the sequence set forth in SEQ ID NO: 28, or
      an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 28;
(5) a heavy chain variable region comprising or consisting of:
   the amino acid sequence set forth in SEQ ID NO: 30,
   a sequence having at least 80%, 85% or 90%, preferably at least 91%, 92%, 93%, 94%, 95%, 96%, 97%, 98% or 99% sequence identity to the sequence set forth in SEQ ID NO: 30, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 30, and
   a light chain variable region comprising or consisting of:
      the amino acid sequence set forth in SEQ ID NO: 32,
      a sequence having at least 80%, 85% or 90%, preferably at least 91%, 92%, 93%, 94%, 95%, 96%, 97%, 98% or 99% sequence identity to the sequence set forth in SEQ ID NO: 32, or
      an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 32;
(6) a heavy chain variable region comprising or consisting of:
   the amino acid sequence set forth in SEQ ID NO: 34,
   a sequence having at least 80%, 85% or 90%, preferably at least 91%, 92%, 93%, 94%, 95%, 96%, 97%, 98% or 99% sequence identity to the sequence set forth in SEQ ID NO: 34, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 34, and
   a light chain variable region comprising or consisting of:
      the amino acid sequence set forth in SEQ ID NO: 36,
      a sequence having at least 80%, 85% or 90%, preferably at least 91%, 92%, 93%, 94%, 95%, 96%, 97%, 98% or 99% sequence identity to the sequence set forth in SEQ ID NO: 36, or
      an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 36;
(7) a heavy chain variable region comprising or consisting of:
   the amino acid sequence set forth in SEQ ID NO: 38,
   a sequence having at least 80%, 85% or 90%, preferably at least 91%, 92%, 93%, 94%, 95%, 96%, 97%, 98% or 99% sequence identity to the sequence set forth in SEQ ID NO: 38, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 38, and
   a light chain variable region comprising or consisting of:
      the amino acid sequence set forth in SEQ ID NO: 40,
      a sequence having at least 80%, 85% or 90%, preferably at least 91%, 92%, 93%, 94%, 95%, 96%, 97%, 98% or 99% sequence identity to the sequence set forth in SEQ ID NO: 40, or
      an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 40;
(8) a heavy chain variable region comprising or consisting of:
   the amino acid sequence set forth in SEQ ID NO: 42,
   a sequence having at least 80%, 85% or 90%, preferably at least 91%, 92%, 93%, 94%, 95%, 96%, 97%, 98% or 99% sequence identity to the sequence set forth in SEQ ID NO: 42, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 42, and
   a light chain variable region comprising or consisting of:
   the amino acid sequence set forth in SEQ ID NO: 44,
   a sequence having at least 80%, 85% or 90%, preferably at least 91%, 92%, 93%, 94%, 95%, 96%, 97%, 98% or 99% sequence identity to the sequence set forth in SEQ ID NO: 44, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 44;
(9) a heavy chain variable region comprising or consisting of:
   the amino acid sequence set forth in SEQ ID NO: 46,
   a sequence having at least 80%, 85% or 90%, preferably at least 91%, 92%, 93%, 94%, 95%, 96%, 97%, 98% or 99% sequence identity to the sequence set forth in SEQ ID NO: 46, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 46, and
   a light chain variable region comprising or consisting of:
      the amino acid sequence set forth in SEQ ID NO: 48,
      a sequence having at least 80%, 85% or 90%, preferably at least 91%, 92%, 93%, 94%, 95%, 96%, 97%, 98% or 99% sequence identity to the sequence set forth in SEQ ID NO: 48, or
      an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 48;
(10) a heavy chain variable region comprising or consisting of:
   the amino acid sequence set forth in SEQ ID NO: 42,
   a sequence having at least 80%, 85% or 90%, preferably at least 91%, 92%, 93%, 94%, 95%, 96%, 97%, 98% or 99% sequence identity to the sequence set forth in SEQ ID NO: 42, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 42, and
   a light chain variable region comprising or consisting of:
   the amino acid sequence set forth in SEQ ID NO: 48,
   a sequence having at least 80%, 85% or 90%, preferably at least 91%, 92%, 93%, 94%, 95%, 96%, 97%, 98% or 99% sequence identity to the sequence set forth in SEQ ID NO: 48, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 48;
(11) a heavy chain variable region comprising or consisting of:
   the amino acid sequence set forth in SEQ ID NO: 46,
   a sequence having at least 80%, 85% or 90%, preferably at least 91%, 92%, 93%, 94%, 95%, 96%, 97%, 98% or 99% sequence identity to the sequence set forth in SEQ ID NO: 46, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 46, and
   a light chain variable region comprising or consisting of:
      a sequence having at least 80%, 85% or 90%, preferably at least 91%, 92%, 93%, 94%, 95%, 96%, 97%, 98% or 99% sequence identity to the sequence set forth in SEQ ID NO: 44, or
      an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 44;
(12) a heavy chain variable region comprising or consisting of:
   the amino acid sequence set forth in SEQ ID NO: 34,
   a sequence having at least 80%, 85% or 90%, preferably at least 91%, 92%, 93%, 94%, 95%, 96%, 97%, 98% or 99% sequence identity to the sequence set forth in SEQ ID NO: 34, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 34, and
   a light chain variable region comprising or consisting of:
   the amino acid sequence set forth in SEQ ID NO: 28,
   a sequence having at least 80%, 85% or 90%, preferably at least 91%, 92%, 93%, 94%, 95%, 96%, 97%, 98% or 99% sequence identity to the sequence set forth in SEQ ID NO: 28, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 28; or
(13) a heavy chain variable region comprising or consisting of:
   the amino acid sequence set forth in SEQ ID NO: 127,
   a sequence having at least 80%, 85% or 90%, preferably at least 91%, 92%, 93%, 94%, 95%, 96%, 97%, 98% or 99% sequence identity to the sequence set forth in SEQ ID NO: 127, or
   an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 127, and
   a light chain variable region comprising or consisting of:
      the amino acid sequence set forth in SEQ ID NO: 129,
      a sequence having at least 80%, 85% or 90%, preferably at least 91%, 92%, 93%, 94%, 95%, 96%, 97%, 98% or 99% sequence identity to the sequence set forth in SEQ ID NO: 129, or
      an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 129.

The amino acid sequences of the CDR regions of the antibodies in (1) to (13) above are analyzed by technical means well known to those skilled in the art, for example, by a VBASE2 database.

The antibodies 13E5, 13E5 H1L1, 13E5 H2L2, 13E5 H3L3, 13E5 H4L4 and 13E5 H4L2 disclosed herein share the same HCDR1-3 and LCDR1-3.

According to the IMGT numbering system, the amino acid sequences of the 3 HCDR regions of the heavy chain variable region are as follows:
HCDR1: GYTFTEYT SEQ ID NO: 9
HCDR2: INPNNGGT SEQ ID NO: 10
HCDR3: ARVRRGMDY SEQ ID NO: 11

The amino acid sequences of the 3 CDR regions of the light chain variable region are as follows:
LCDR1: QDVTTA SEQ ID NO: 12
LCDR2: SAS SEQ ID NO: 13
LCDR3: QQHYSAPWT SEQ ID NO: 14

The antibodies 18H7, 18H7 H1L1, 18H7 H2L2, 18H7 H2L3, and 18H7 H3L2 disclosed herein share the same HCDR1-3 and LCDR1-3.

According to the IMGT numbering system, the amino acid sequences of the 3 HCDR regions of the heavy chain variable region are as follows:
HCDR1: GFTFSSSY SEQ ID NO: 15
HCDR2: INSNGGKT SEQ ID NO: 16
HCDR3: TRQRGNYVGAMDY SEQ ID NO: 17

The amino acid sequences of the 3 CDR regions of the light chain variable region are as follows:
LCDR1: QDVSTA SEQ ID NO: 18
LCDR2: SAS SEQ ID NO: 19
LCDR3: HQYYGSPPT SEQ ID NO: 20

The antibody 20G10 H3L3 disclosed herein has the following HCDR1-3 and LCDR1-3. According to the IMGT numbering system, the amino acid sequences of the 3 HCDR regions of the heavy chain variable region are as follows:
HCDR1: GFSLSTSGMG SEQ ID NO: 130
HCDR2: IWWADDK SEQ ID NO: 131
HCDR3: ARITRGNSAMDF SEQ ID NO: 132

The amino acid sequences of the 3 CDR regions of the light chain variable region are as follows:
LCDR1: ENVYSY SEQ ID NO: 133
LCDR2: NAK SEQ ID NO: 134
LCDR3: QHHYGIPWT SEQ ID NO: 135

In one embodiment of the present invention, the antibody further comprises framework regions (FRs) in the heavy chain variable region, preferably the FRs including FR-H1, FR-H2, FR-H3 and FR-H4, wherein the FR-H1 comprises or consists of the amino acid sequence set forth in SEQ ID NO: 49, a sequence having at least 80%, 85% or 90%, preferably at least 91%, 92%, 93%, 94%, 95%, 96%, 97%, 98% or 99% sequence identity to the sequence set forth in SEQ ID NO: 49, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 49; the FR-H2 comprises or consists of the amino acid sequence set forth in SEQ ID NO: 50, a sequence having at least 80%, 85% or 90%, preferably at least 91%, 92%, 93%, 94%, 95%, 96%, 97%, 98% or 99% sequence identity to the sequence set forth in SEQ ID NO: 50, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 50; the FR-H3 comprises or consists of the amino acid sequence set forth in SEQ ID NO: 51, a sequence having at least 80%, 85% or 90%, preferably at least 91%, 92%, 93%, 94%, 95%, 96%, 97%, 98% or 99% sequence identity to the sequence set forth in SEQ ID NO: 51, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 51; and the FR-H4 comprises or consists of the amino acid sequence set forth in SEQ ID NO: 52, a sequence having at least 80%, 85% or 90%, preferably at least 91%, 92%, 93%, 94%, 95%, 96%, 97%, 98% or 99% sequence identity to the sequence set forth in SEQ ID NO: 52, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 52.

In one embodiment of the present invention, the antibody further comprises FRs in the light chain variable region, preferably the FRs including FR-L1, FR-L2, FR-L3 and FR-L4, wherein the FR-L1 comprises or consists of the amino acid sequence set forth in SEQ ID NO: 53, a sequence having at least 80%, 85% or 90%, preferably at least 91%, 92%, 93%, 94%, 95%, 96%, 97%, 98% or 99% sequence identity to the sequence set forth in SEQ ID NO: 53, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 53; the FR-L2 comprises or consists of the amino acid sequence set forth in SEQ ID NO: 54, a sequence having at least 80%, 85% or 90%, preferably at least 91%, 92%, 93%, 94%, 95%, 96%, 97%, 98% or 99% sequence identity to the sequence set forth in SEQ ID NO: 54, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 54; the FR-L3 comprises or consists of the amino acid sequence set forth in SEQ ID NO: 55, a sequence having at least 80%, 85% or 90%, preferably at least 91%, 92%, 93%, 94%, 95%, 96%, 97%, 98% or 99% sequence identity to the sequence set forth in SEQ ID NO: 55, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 55; and the FR-L4 comprises or consists of the amino acid sequence set forth in SEQ ID NO: 56, a sequence having at least 80%, 85% or 90%, preferably at least 91%, 92%, 93%, 94%, 95%, 96%, 97%, 98% or 99% sequence identity to the sequence set forth in SEQ ID NO: 56, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 56.

In one embodiment of the present invention, the antibody further comprises FRs in the heavy chain variable region, preferably the FRs including FR-H1, FR-H2, FR-H3 and FR-H4, wherein the FR-H1 comprises or consists of the amino acid sequence set forth in SEQ ID NO: 57, a sequence having at least 80%, 85% or 90%, preferably at least 91%, 92%, 93%, 94%, 95%, 96%, 97%, 98% or 99% sequence identity to the sequence set forth in SEQ ID NO: 57, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 57; the FR-H2 comprises or consists of the amino acid sequence set forth in SEQ ID NO: 58, a sequence having at least 80%, 85% or 90%, preferably at least 91%, 92%, 93%, 94%, 95%, 96%, 97%, 98% or 99% sequence identity to the sequence set forth in SEQ ID NO: 58, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 58; the FR-H3 comprises or consists of the amino acid sequence set forth in SEQ ID NO: 59, a sequence having at least 80%, 85% or 90%, preferably at least 91%, 92%, 93%, 94%, 95%, 96%, 97%, 98% or 99% sequence identity to the sequence set forth in SEQ ID NO: 59, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 59; and the FR-H4 comprises or consists of the amino acid sequence set forth in SEQ ID NO: 60, a sequence having at least 80%, 85% or 90%, preferably at least 91%, 92%, 93%, 94%, 95%, 96%, 97%, 98% or 99% sequence identity to the sequence set forth in SEQ ID NO: 60, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 60.

In one embodiment of the present invention, the antibody further comprises FRs in the light chain variable region, preferably the FRs including FR-L1, FR-L2, FR-L3 and FR-L4, wherein the FR-L1 comprises or consists of the amino acid sequence set forth in SEQ ID NO: 61, a sequence having at least 80%, 85% or 90%, preferably at least 91%, 92%, 93%, 94%, 95%, 96%, 97%, 98% or 99% sequence identity to the sequence set forth in SEQ ID NO: 61, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 61; the FR-L2 comprises or consists of the amino acid sequence set forth in SEQ ID NO: 62, a sequence having at least 80%, 85% or 90%, preferably at least 91%, 92%, 93%, 94%, 95%, 96%, 97%, 98% or 99% sequence identity to the sequence set forth in SEQ ID NO: 62, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 62; the FR-L3 comprises or consists of the amino acid sequence set forth in SEQ ID NO: 63, a sequence having at least 80%, 85% or 90%, preferably at least 91%, 92%, 93%, 94%, 95%, 96%, 97%, 98% or 99% sequence identity to the sequence set forth in SEQ ID NO: 63, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 63; and the FR-L4 comprises or consists of the amino acid sequence set forth in SEQ ID NO: 64, a sequence having at least 80%, 85% or 90%, preferably at least 91%, 92%, 93%, 94%, 95%, 96%, 97%, 98% or 99% sequence identity to the sequence set forth in SEQ ID NO: 64, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 64.

In one embodiment of the present invention, the antibody further comprises FRs in the heavy chain variable region, preferably the FRs including FR-H1, FR-H2, FR-H3 and FR-H4, wherein the FR-H1 comprises or consists of the amino acid sequence set forth in SEQ ID NO: 65, a sequence having at least 80%, 85% or 90%, preferably at least 91%, 92%, 93%, 94%, 95%, 96%, 97%, 98% or 99% sequence identity to the sequence set forth in SEQ ID NO: 65, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 65; the FR-H2 comprises or consists of the amino acid sequence set forth in SEQ ID NO: 66, a sequence having at least 80%, 85% or 90%, preferably at least 91%, 92%, 93%, 94%, 95%, 96%, 97%, 98% or 99% sequence identity to the sequence set forth in SEQ ID NO: 66, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 66; the FR-H3 comprises or consists of the amino acid sequence set forth in SEQ ID NO: 67, a sequence having at least 80%, 85% or 90%, preferably at least 91%, 92%, 93%, 94%, 95%, 96%, 97%, 98% or 99% sequence identity to the sequence set forth in SEQ ID NO: 67, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 67; and the FR-H4 comprises or consists of the amino acid sequence set forth in SEQ ID NO: 68, a sequence having at least 80%, 85% or 90%, preferably at least 91%, 92%, 93%, 94%, 95%, 96%, 97%, 98% or 99% sequence identity to the sequence set forth in SEQ ID NO: 68, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 68.

In one embodiment of the present invention, the antibody further comprises FRs in the light chain variable region, preferably the FRs including FR-L1, FR-L2, FR-L3 and FR-L4, wherein the FR-L1 comprises or consists of the amino acid sequence set forth in SEQ ID NO: 69, a sequence having at least 80%, 85% or 90%, preferably at least 91%, 92%, 93%, 94%, 95%, 96%, 97%, 98% or 99% sequence identity to the sequence set forth in SEQ ID NO: 69, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 69; the FR-L2 comprises or consists of the amino acid sequence set forth in SEQ ID NO: 70, a sequence having at least 80%, 85% or 90%, preferably at least 91%, 92%, 93%, 94%, 95%, 96%, 97%, 98% or 99% sequence identity to the sequence set forth in SEQ ID NO: 70, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 70; the FR-L3 comprises or consists of the amino acid sequence set forth in SEQ ID NO: 71, a sequence having at least 80%, 85% or 90%, preferably at least 91%, 92%, 93%, 94%, 95%, 96%, 97%, 98% or 99% sequence identity to the sequence set forth in SEQ ID NO: 71, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 71; and the FR-L4 comprises or consists of the amino acid sequence set forth in SEQ ID NO: 72, a sequence having at least 80%, 85% or 90%, preferably at least 91%, 92%, 93%, 94%, 95%, 96%, 97%, 98% or 99% sequence identity to the sequence set forth in SEQ ID NO: 72, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 72.

In one embodiment of the present invention, the antibody further comprises FRs in the heavy chain variable region, preferably the FRs including FR-H1, FR-H2, FR-H3 and FR-H4, wherein the FR-H1 comprises or consists of the amino acid sequence set forth in SEQ ID NO: 73, a sequence having at least 80%, 85% or 90%, preferably at least 91%, 92%, 93%, 94%, 95%, 96%, 97%, 98% or 99% sequence identity to the sequence set forth in SEQ ID NO: 73, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 73; the FR-H2 comprises or consists of the amino acid sequence set forth in SEQ ID NO: 74, a sequence having at least 80%, 85% or 90%, preferably at least 91%, 92%, 93%, 94%, 95%, 96%, 97%, 98% or 99% sequence identity to the sequence set forth in SEQ ID NO: 74, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 74; the FR-H3 comprises or consists of the amino acid sequence set forth in SEQ ID NO: 75, a sequence having at least 80%, 85% or 90%, preferably at least 91%, 92%, 93%, 94%, 95%, 96%, 97%, 98% or 99% sequence identity to the sequence set forth in SEQ ID NO: 75, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 75; and the FR-H4 comprises or consists of the amino acid sequence set forth in SEQ ID NO: 76, a sequence having at least 80%, 85% or 90%, preferably at least 91%, 92%, 93%, 94%, 95%, 96%, 97%, 98% or 99% sequence identity to the sequence set forth in SEQ ID NO: 76, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 76.

In one embodiment of the present invention, the antibody further comprises FRs in the light chain variable region, preferably the FRs including FR-L1, FR-L2, FR-L3 and FR-L4, wherein the FR-L1 comprises or consists of the amino acid sequence set forth in SEQ ID NO: 77, a sequence having at least 80%, 85% or 90%, preferably at least 91%, 92%, 93%, 94%, 95%, 96%, 97%, 98% or 99% sequence identity to the sequence set forth in SEQ ID NO: 77, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 77; the FR-L2 comprises or consists of the amino acid sequence set forth in SEQ ID NO: 78, a sequence having at least 80%, 85% or 90%, preferably at least 91%, 92%, 93%, 94%, 95%, 96%, 97%, 98% or 99% sequence identity to the sequence set forth in SEQ ID NO: 78, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 78; the FR-L3 comprises or consists of the amino acid sequence set forth in SEQ ID NO: 79, a sequence having at least 80%, 85% or 90%, preferably at least 91%, 92%, 93%, 94%, 95%, 96%, 97%, 98% or 99% sequence identity to the sequence set forth in SEQ ID NO: 79, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 79; and the FR-L4 comprises or consists of the amino acid sequence set forth in SEQ ID NO: 80, a sequence having at least 80%, 85% or 90%, preferably at least 91%, 92%, 93%, 94%, 95%, 96%, 97%, 98% or 99% sequence identity to the sequence set forth in SEQ ID NO: 80, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 80.

In one embodiment of the present invention, the antibody further comprises FRs in the heavy chain variable region, preferably the FRs including FR-H1, FR-H2, FR-H3 and FR-H4, wherein the FR-H1 comprises or consists of the amino acid sequence set forth in SEQ ID NO: 81, a sequence having at least 80%, 85% or 90%, preferably at least 91%, 92%, 93%, 94%, 95%, 96%, 97%, 98% or 99% sequence identity to the sequence set forth in SEQ ID NO: 81, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 81; the FR-H2 comprises or consists of the amino acid sequence set forth in SEQ ID NO: 82, a sequence having at least 80%, 85% or 90%, preferably at least 91%, 92%, 93%, 94%, 95%, 96%, 97%, 98% or 99% sequence identity to the sequence set forth in SEQ ID NO: 82, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 82; the FR-H3 comprises or consists of the amino acid sequence set forth in SEQ ID NO: 83, a sequence having at least 80%, 85% or 90%, preferably at least 91%, 92%, 93%, 94%, 95%, 96%, 97%, 98% or 99% sequence identity to the sequence set forth in SEQ ID NO: 83, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 83; and the FR-H4 comprises or consists of the amino acid sequence set forth in SEQ ID NO: 84, a sequence having at least 80%, 85% or 90%, preferably at least 91%, 92%, 93%, 94%, 95%, 96%, 97%, 98% or 99% sequence identity to the sequence set forth in SEQ ID NO: 84, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 84.

In one embodiment of the present invention, the antibody further comprises FRs in the light chain variable region, preferably the FRs including FR-L1, FR-L2, FR-L3 and FR-L4, wherein the FR-L1 comprises or consists of the amino acid sequence set froth in SEQ ID NO: 85, a sequence having at least 80%, 85% or 90%, preferably at least 91%, 92%, 93%, 94%, 95%, 96%, 97%, 98% or 99% sequence identity to the sequence set forth in SEQ ID NO: 85, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 85; the FR-L2 comprises or consists of the amino acid sequence set forth in SEQ ID NO: 86, a sequence having at least 80%, 85% or 90%, preferably at least 91%, 92%, 93%, 94%, 95%, 96%, 97%, 98% or 99% sequence identity to the sequence set forth in SEQ ID NO: 86, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 86; the FR-L3 comprises or consists of the amino acid sequence set forth in SEQ ID NO: 87, a sequence having at least 80%, 85% or 90%, preferably at least 91%, 92%, 93%, 94%, 95%, 96%, 97%, 98% or 99% sequence identity to the sequence set forth in SEQ ID NO: 87, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 87; and the FR-L4 comprises or consists of the amino acid sequence set forth in SEQ ID NO: 88, a sequence having at least 80%, 85% or 90%, preferably at least 91%, 92%, 93%, 94%, 95%, 96%, 97%, 98% or 99% sequence identity to the sequence set forth in SEQ ID NO: 88, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 88.

In one embodiment of the present invention, the antibody further comprises FRs in the heavy chain variable region, preferably the FRs including FR-H1, FR-H2, FR-H3 and FR-H4, wherein the FR-H1 comprises or consists of the amino acid sequence set forth in SEQ ID NO: 89, a sequence having at least 80%, 85% or 90%, preferably at least 91%, 92%, 93%, 94%, 95%, 96%, 97%, 98% or 99% sequence identity to the sequence set forth in SEQ ID NO: 89, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 89; the FR-H2 comprises or consists of the amino acid sequence set forth in SEQ ID NO: 90, a sequence having at least 80%, 85% or 90%, preferably at least 91%, 92%, 93%, 94%, 95%, 96%, 97%, 98% or 99% sequence identity to the sequence set forth in SEQ ID NO: 90, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 90; the FR-H3 comprises or consists of the amino acid sequence set forth in SEQ ID NO: 91, a sequence having at least 80%, 85% or 90%, preferably at least 91%, 92%, 93%, 94%, 95%, 96%, 97%, 98% or 99% sequence identity to the sequence set forth in SEQ ID NO: 91, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 91; and the FR-H4 comprises or consists of the amino acid sequence set forth in SEQ ID NO: 92, a sequence having at least 80%, 85% or 90%, preferably at least 91%, 92%, 93%, 94%, 95%, 96%, 97%, 98% or 99% sequence identity to the sequence set forth in SEQ ID NO: 92, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 92.

In one embodiment of the present invention, the antibody further comprises FRs in the light chain variable region, preferably the FRs including FR-L1, FR-L2, FR-L3 and FR-L4, wherein the FR-L1 comprises or consists of the amino acid sequence set forth in SEQ ID NO: 93, a sequence having at least 80%, 85% or 90%, preferably at least 91%, 92%, 93%, 94%, 95%, 96%, 97%, 98% or 99% sequence identity to the sequence set forth in SEQ ID NO: 93, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 93; the FR-L2 comprises or consists of the amino acid sequence set forth in SEQ ID NO: 94, a sequence having at least 80%, 85% or 90%, preferably at least 91%, 92%, 93%, 94%, 95%, 96%, 97%, 98% or 99% sequence identity to the sequence set forth in SEQ ID NO: 94, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 94; the FR-L3 comprises or consists of the amino acid sequence set forth in SEQ ID NO: 95, a sequence having at least 80%, 85% or 90%, preferably at least 91%, 92%, 93%, 94%, 95%, 96%, 97%, 98% or 99% sequence identity to the sequence set forth in SEQ ID NO: 95, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 95; and the FR-L4 comprises or consists of the amino acid sequence set forth in SEQ ID NO: 96, a sequence having at least 80%, 85% or 90%, preferably at least 91%, 92%, 93%, 94%, 95%, 96%, 97%, 98% or 99% sequence identity to the sequence set forth in SEQ ID NO: 96, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 96.

In one embodiment of the present invention, the antibody further comprises FRs in the heavy chain variable region, preferably the FRs including FR-H1, FR-H2, FR-H3 and FR-H4, wherein the FR-H1 comprises or consists of the amino acid sequence set forth in SEQ ID NO: 97, a sequence having at least 80%, 85% or 90%, preferably at least 91%, 92%, 93%, 94%, 95%, 96%, 97%, 98% or 99% sequence identity to the sequence set forth in SEQ ID NO: 97, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 97; the FR-H2 comprises or consists of the amino acid sequence set forth in SEQ ID NO: 98, a sequence having at least 80%, 85% or 90%, preferably at least 91%, 92%, 93%, 94%, 95%, 96%, 97%, 98% or 99% sequence identity to the sequence set forth in SEQ ID NO: 98, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 98; the FR-H3 comprises or consists of the amino acid sequence set forth in SEQ ID NO: 99, a sequence having at least 80%, 85% or 90%, preferably at least 91%, 92%, 93%, 94%, 95%, 96%, 97%, 98% or 99% sequence identity to the sequence set forth in SEQ ID NO: 99, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 99; and the FR-H4 comprises or consists of the amino acid sequence set forth in SEQ ID NO: 100, a sequence having at least 80%, 85% or 90%, preferably at least 91%, 92%, 93%, 94%, 95%, 96%, 97%, 98% or 99% sequence identity to the sequence set forth in SEQ ID NO: 100, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 100.

In one embodiment of the present invention, the antibody further comprises FRs in the light chain variable region, preferably the FRs including FR-L1, FR-L2, FR-L3 and FR-L4, wherein the FR-L1 comprises or consists of the amino acid sequence set forth in SEQ ID NO: 101, a sequence having at least 80%, 85% or 90%, preferably at least 91%, 92%, 93%, 94%, 95%, 96%, 97%, 98% or 99% sequence identity to the sequence set forth in SEQ ID NO: 101, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 101; the FR-L2 comprises or consists of the amino acid sequence set forth in SEQ ID NO: 102, a sequence having at least 80%, 85% or 90%, preferably at least 91%, 92%, 93%, 94%, 95%, 96%, 97%, 98% or 99% sequence identity to the sequence set forth in SEQ ID NO: 102, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 102; the FR-L3 comprises or consists of the amino acid sequence set forth in SEQ ID NO: 103, a sequence having at least 80%, 85% or 90%, preferably at least 91%, 92%, 93%, 94%, 95%, 96%, 97%, 98% or 99% sequence identity to the sequence set forth in SEQ ID NO: 103, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 103; and the FR-L4 comprises or consists of the amino acid sequence set forth in SEQ ID NO: 104, a sequence having at least 80%, 85% or 90%, preferably at least 91%, 92%, 93%, 94%, 95%, 96%, 97%, 98% or 99% sequence identity to the sequence set forth in SEQ ID NO: 104, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 104.

In one embodiment of the present invention, the antibody further comprises FRs in the heavy chain variable region, preferably the FRs including FR-H1, FR-H2, FR-H3 and FR-H4, wherein the FR-H1 comprises or consists of the amino acid sequence set forth in SEQ ID NO: 105, a sequence having at least 80%, 85% or 90%, preferably at least 91%, 92%, 93%, 94%, 95%, 96%, 97%, 98% or 99% sequence identity to the sequence set forth in SEQ ID NO: 105, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 105; the FR-H2 comprises or consists of the amino acid sequence set forth in SEQ ID NO: 106, a sequence having at least 80%, 85% or 90%, preferably at least 91%, 92%, 93%, 94%, 95%, 96%, 97%, 98% or 99% sequence identity to the sequence set forth in SEQ ID NO: 106, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 106; the FR-H3 comprises or consists of the amino acid sequence set forth in SEQ ID NO: 107, a sequence having at least 80%, 85% or 90%, preferably at least 91%, 92%, 93%, 94%, 95%, 96%, 97%, 98% or 99% sequence identity to the sequence set forth in SEQ ID NO: 107, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 107; and the FR-H4 comprises or consists of the amino acid sequence set forth in SEQ ID NO: 108, a sequence having at least 80%, 85% or 90%, preferably at least 91%, 92%, 93%, 94%, 95%, 96%, 97%, 98% or 99% sequence identity to the sequence set forth in SEQ ID NO: 108, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 108.

In one embodiment of the present invention, the antibody further comprises FRs in the light chain variable region, preferably the FRs including FR-L1, FR-L2, FR-L3 and FR-L4, wherein the FR-L1 comprises or consists of the amino acid sequence set forth in SEQ ID NO: 109, a sequence having at least 80%, 85% or 90%, preferably at least 91%, 92%, 93%, 94%, 95%, 96%, 97%, 98% or 99% sequence identity to the sequence set forth in SEQ ID NO: 109, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 109; the FR-L2 comprises or consists of the amino acid sequence set forth in SEQ ID NO: 110, a sequence having at least 80%, 85% or 90%, preferably at least 91%, 92%, 93%, 94%, 95%, 96%, 97%, 98% or 99% sequence identity to the sequence set forth in SEQ ID NO: 110, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 110; the FR-L3 comprises or consists of the amino acid sequence set forth in SEQ ID NO: 111, a sequence having at least 80%, 85% or 90%, preferably at least 91%, 92%, 93%, 94%, 95%, 96%, 97%, 98% or 99% sequence identity to the sequence set forth in SEQ ID NO: 111, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 111; and the FR-L4 comprises or consists of the amino acid sequence set forth in SEQ ID NO: 112, a sequence having at least 80%, 85% or 90%, preferably at least 91%, 92%, 93%, 94%, 95%, 96%, 97%, 98% or 99% sequence identity to the sequence set forth in SEQ ID NO: 112, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 112.

In one embodiment of the present invention, the antibody further comprises FRs in the heavy chain variable region, preferably the FRs including FR-H1, FR-H2, FR-H3 and FR-H4, wherein the FR-H1 comprises or consists of the amino acid sequence set forth in SEQ ID NO: 113, a sequence having at least 80%, 85% or 90%, preferably at least 91%, 92%, 93%, 94%, 95%, 96%, 97%, 98% or 99% sequence identity to the sequence set forth in SEQ ID NO: 113, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 113; the FR-H2 comprises or consists of the amino acid sequence set forth in SEQ ID NO: 114, a sequence having at least 80%, 85% or 90%, preferably at least 91%, 92%, 93%, 94%, 95%, 96%, 97%, 98% or 99% sequence identity to the sequence set forth in SEQ ID NO: 114, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 114; the FR-H3 comprises or consists of the amino acid sequence set forth in SEQ ID NO: 115, a sequence having at least 80%, 85% or 90%, preferably at least 91%, 92%, 93%, 94%, 95%, 96%, 97%, 98% or 99% sequence identity to the sequence set forth in SEQ ID NO: 115, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 115; and the FR-H4 comprises or consists of the amino acid sequence set forth in SEQ ID NO: 116, a sequence having at least 80%, 85% or 90%, preferably at least 91%, 92%, 93%, 94%, 95%, 96%, 97%, 98% or 99% sequence identity to the sequence set forth in SEQ ID NO: 116, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 116.

In one embodiment of the present invention, the antibody further comprises FRs in the light chain variable region, preferably the FRs including FR-L1, FR-L2, FR-L3 and FR-L4, wherein the FR-L1 comprises or consists of the amino acid sequence set forth in SEQ ID NO: 117, a sequence having at least 80%, 85% or 90%, preferably at least 91%, 92%, 93%, 94%, 95%, 96%, 97%, 98% or 99% sequence identity to the sequence set forth in SEQ ID NO: 117, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 117; the FR-L2 comprises or consists of the amino acid sequence set forth in SEQ ID NO: 118, a sequence having at least 80%, 85% or 90%, preferably at least 91%, 92%, 93%, 94%, 95%, 96%, 97%, 98% or 99% sequence identity to the sequence set forth in SEQ ID NO: 118, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 118; the FR-L3 comprises or consists of the amino acid sequence set forth in SEQ ID NO: 119, a sequence having at least 80%, 85% or 90%, preferably at least 91%, 92%, 93%, 94%, 95%, 96%, 97%, 98% or 99% sequence identity to the sequence set forth in SEQ ID NO: 119, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 119; and the FR-L4 comprises or consists of the amino acid sequence set forth in SEQ ID NO: 120, a sequence having at least 80%, 85% or 90%, preferably at least 91%, 92%, 93%, 94%, 95%, 96%, 97%, 98% or 99% sequence identity to the sequence set forth in SEQ ID NO: 120, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 120.

In one embodiment of the present invention, the antibody further comprises FRs in the heavy chain variable region, preferably the FRs including FR-H1, FR-H2, FR-H3 and FR-H4, wherein the FR-H1 comprises or consists of the amino acid sequence set forth in SEQ ID NO: 136, a sequence having at least 80%, 85% or 90%, preferably at least 91%, 92%, 93%, 94%, 95%, 96%, 97%, 98% or 99% sequence identity to the sequence set forth in SEQ ID NO: 136, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 136; the FR-H2 comprises or consists of the amino acid sequence set forth in SEQ ID NO: 137, a sequence having at least 80%, 85% or 90%, preferably at least 91%, 92%, 93%, 94%, 95%, 96%, 97%, 98% or 99% sequence identity to the sequence set forth in SEQ ID NO: 137, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 137; the FR-H3 comprises or consists of the amino acid sequence set forth in SEQ ID NO: 138, a sequence having at least 80%, 85% or 90%, preferably at least 91%, 92%, 93%, 94%, 95%, 96%, 97%, 98% or 99% sequence identity to the sequence set forth in SEQ ID NO: 138, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 138; and the FR-H4 comprises or consists of the amino acid sequence set forth in SEQ ID NO: 139, a sequence having at least 80%, 85% or 90%, preferably at least 91%, 92%, 93%, 94%, 95%, 96%, 97%, 98% or 99% sequence identity to the sequence set forth in SEQ ID NO: 139, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 139.

In one embodiment of the present invention, the antibody further comprises FRs in the light chain variable region, preferably the FRs including FR-L1, FR-L2, FR-L3 and FR-L4, wherein the FR-L1 comprises or consists of the amino acid sequence set forth in SEQ ID NO: 140, a sequence having at least 80%, 85% or 90%, preferably at least 91%, 92%, 93%, 94%, 95%, 96%, 97%, 98% or 99% sequence identity to the sequence set forth in SEQ ID NO: 140, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 140; the FR-L2 comprises or consists of the amino acid sequence set forth in SEQ ID NO: 141, a sequence having at least 80%, 85% or 90%, preferably at least 91%, 92%, 93%, 94%, 95%, 96%, 97%, 98% or 99% sequence identity to the sequence set forth in SEQ ID NO: 141, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 141; the FR-L3 comprises or consists of the amino acid sequence set forth in SEQ ID NO: 142, a sequence having at least 80%, 85% or 90%, preferably at least 91%, 92%, 93%, 94%, 95%, 96%, 97%, 98% or 99% sequence identity to the sequence set forth in SEQ ID NO: 142, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 142; and the FR-L4 comprises or consists of the amino acid sequence set forth in SEQ ID NO: 143, a sequence having at least 80%, 85% or 90%, preferably at least 91%, 92%, 93%, 94%, 95%, 96%, 97%, 98% or 99% sequence identity to the sequence set forth in SEQ ID NO: 143, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 143.

One aspect of the present invention relates to an isolated polypeptide comprising the sequences set forth in SEQ ID NOs: 9, 10 and 11, wherein the polypeptide specifically binds to human IL-4RA as part of an anti-human IL-4RA antibody, the antibody further comprising the sequences set forth in SEQ ID NOs: 12, 13 and 14.

One aspect of the present invention relates to an isolated polypeptide comprising the sequences set forth in SEQ ID NOs: 15, 16 and 17, wherein the polypeptide specifically binds to human IL-4RA as part of an anti-human IL-4RA antibody, the antibody further comprising the sequences set forth in SEQ ID NOs: 18, 19 and 20.

One aspect of the present invention relates to an isolated polypeptide comprising the sequences set forth in SEQ ID NOs: 12, 13 and 14, wherein the polypeptide specifically binds to human IL-4RA as part of an anti-human IL-4RA antibody, the antibody further comprising the sequences set forth in SEQ ID NOs: 9, 10 and 11.

One aspect of the present invention relates to an isolated polypeptide comprising the sequences set forth in SEQ ID NOs: 18, 19 and 20, wherein the polypeptide specifically binds to human IL-4RA as part of an anti-human IL-4RA antibody, the antibody further comprising the sequences set forth in SEQ ID NOs: 15, 16 and 17.

One aspect of the present invention relates to an isolated polypeptide comprising the sequences set forth in SEQ ID NOs: 130, 131 and 132, wherein the polypeptide specifically binds to human IL-4RA as part of an anti-human IL-4RA antibody, the antibody further comprising the sequences set forth in SEQ ID NOs: 133, 134 and 135.

One aspect of the present invention relates to an isolated polypeptide comprising the sequences set forth in SEQ ID NOs: 133, 134 and 135, wherein the polypeptide specifically binds to human IL-4RA as part of an anti-human IL-4RA antibody, the antibody further comprising the sequences set forth in SEQ ID NOs: 130, 131 and 132.

One aspect of the present invention relates to an isolated polypeptide comprising a sequence selected from the sequences set forth in SEQ ID NOs: 2, 6, 22, 26, 30, 34 and 38, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences, wherein the polypeptide specifically binds to human IL-4RA as part of an anti-human IL-4RA antibody, the antibody further comprising a sequence selected from the sequences set forth in SEQ ID NOs: 4, 8, 24, 28, 32, 36 and 40, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences;
an isolated polypeptide comprising a sequence selected from the sequence set forth in SEQ ID NO: 42, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 44, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polypeptide comprising a sequence selected from the sequence set forth in SEQ ID NO: 42, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 48, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polypeptide comprising a sequence selected from the sequence set forth in SEQ ID NO: 46, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 48, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence; or
an isolated polypeptide comprising a sequence selected from the sequence set forth in SEQ ID NO: 34, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 28, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence.

One aspect of the present invention relates to an isolated polypeptide comprising a sequence selected from the sequences set forth in SEQ ID NOs: 4, 8, 24, 28, 32, 36 and 40, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences, wherein the polypeptide specifically binds to human IL-4RA as part of an anti-human IL-4RA antibody, the antibody further comprising a sequence respectively selected from the sequences set forth in SEQ ID NOs: 2, 6, 22, 26, 30, 34 and 38, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences;
an isolated polypeptide comprising a sequence selected from the sequence set forth in SEQ ID NO: 44, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 42, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polypeptide comprising a sequence selected from the sequence set forth in SEQ ID NO: 48, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 42, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polypeptide comprising a sequence selected from the sequence set forth in SEQ ID NO: 48, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 46, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polypeptide comprising a sequence selected from the sequence set forth in SEQ ID NO: 28, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 34, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polypeptide comprising a sequence selected from the sequence set forth in SEQ ID NO: 127, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 129, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence; or
an isolated polypeptide comprising a sequence selected from the sequence set forth in SEQ ID NO: 129, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 127, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence.

In one embodiment of the present invention, the antigen-binding fragment is selected from Fab, Fab', F(ab')₂, Fd, Fv, dAb, Fab/c, complementarity determining region (CDR) fragment, single chain antibody (e.g., scFv), bivalent antibody and domain antibody.

In one embodiment of the present invention, the antibody is a humanized antibody, a chimeric antibody or a multispecific antibody (e.g., bispecific antibody).

In one embodiment of the present invention, the antibody binds to human IL-4RA protein with a K_{D} less than about 10⁻⁵ M, e.g., less than about 10⁻⁶ M, 10⁻⁷ M, 10⁻⁸ M, 10⁻⁹ M or 10⁻¹⁰ M or less. Preferably, the K_{D} is measured by a Fortebio molecular interaction instrument. In one embodiment of the present invention, the antibody binds to human IL-4RA protein with an EC₅₀ less than about 100 nM, e.g., less than about
10 nM, 1 nM, 0.9 nM, 0.8 nM, 0.7 nM, 0.6 nM, 0.5 nM, 0.4 nM, 0.3 nM,
0.2 nM, 0.1 nM or less. Specifically, the EC₅₀ is measured by indirect ELISA.

In one embodiment of the present invention, the antibody comprises a constant region, and the constant region is derived from a species other than murine, e.g., from a human antibody, preferably from a human IgG, more preferably from IgG4.

In one embodiment of the present invention, the constant region of the antibody is humanized, e.g., the heavy chain constant regions are Ig gamma-4 chain C regions, preferably the Ig gamma-4 chain C region of GenBank ACCESSION No: P01861.1; the light chain constant regions are Ig kappa chain C regions, preferably the Ig kappa chain C region of GenBank ACCESSION No: P01834.

Another aspect of the present invention relates to an isolated polynucleotide encoding a polypeptide comprising the sequences set forth in SEQ ID NOs: 9, 10 and 11, wherein the polypeptide specifically binds to human IL-4RA as part of an anti-human IL-4RA antibody, the antibody further comprising the sequences set forth in SEQ ID NOs: 12, 13 and 14. One aspect of the present invention relates to an isolated polynucleotide encoding a polypeptide comprising the sequences set forth in SEQ ID NOs: 15, 16 and 17, wherein the polypeptide specifically binds to human IL-4RA as part of an anti-human IL-4RA antibody, the antibody further comprising the sequences set forth in SEQ ID NOs: 18, 19 and 20. One aspect of the present invention relates to an isolated polynucleotide encoding a polypeptide comprising the sequences set forth in SEQ ID NOs: 12, 13 and 14, wherein the polypeptide specifically binds to human IL-4RA as part of an anti-human IL-4RA antibody, the antibody further comprising the sequences set forth in SEQ ID NOs: 9, 10 and 11.

One aspect of the present invention relates to an isolated polynucleotide encoding a polypeptide comprising the sequences set forth in SEQ ID NOs: 18, 19 and 20, wherein the polypeptide specifically binds to human IL-4RA as part of an anti-human IL-4RA antibody, the antibody further comprising the sequences set forth in SEQ ID NOs: 15, 16 and 17. One aspect of the present invention relates to an isolated polynucleotide encoding a polypeptide comprising the sequences set forth in SEQ ID NOs: 130, 131 and 132, wherein the polypeptide specifically binds to human IL-4RA as part of an anti-human IL-4RA antibody, the antibody further comprising the sequences set forth in SEQ ID NOs: 133, 134 and 135.

One aspect of the present invention relates to an isolated polynucleotide encoding a polypeptide comprising the sequences set forth in SEQ ID NOs: 133, 134 and 135, wherein the polypeptide specifically binds to human IL-4RA as part of an anti-human IL-4RA antibody, the antibody further comprising the sequences set forth in SEQ ID NOs: 130, 131 and 132.

One aspect of the present invention relates to an isolated polynucleotide encoding a polypeptide comprising a sequence selected from the sequences set forth in SEQ ID NOs: 2, 6, 22, 26, 30, 34 and 38, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences, wherein the polypeptide specifically binds to human IL-4RA as part of an anti-human IL-4RA antibody, the antibody further comprising a sequence respectively selected from the sequences set forth in SEQ ID NOs: 4, 8, 24, 28, 32, 36 and 40, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences; an isolated polynucleotide encoding a polypeptide comprising a sequence selected from the sequence set forth in SEQ ID NO: 42, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 44, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polynucleotide encoding a polypeptide comprising a sequence selected from the sequence set forth in SEQ ID NO: 42, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 48, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polynucleotide encoding a polypeptide comprising a sequence selected from the sequence set forth in SEQ ID NO: 46, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as
part of an anti-human IL-4RA antibody, the antibody further comprising a sequence selected from the sequence set forth in SEQ ID NO: 48, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence; or
an isolated polynucleotide encoding a polypeptide comprising a sequence selected from the sequence set forth in SEQ ID NO: 34, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as
part of an anti-human IL-4RA antibody, the antibody further comprising a sequence selected from the sequence set forth in SEQ ID NO: 28, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence.

One aspect of the present invention relates to an isolated polynucleotide encoding a polypeptide comprising a sequence selected from the sequences set forth in SEQ ID NOs: 4, 8, 24, 28, 32, 36 and 40, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences, wherein the polypeptide specifically binds to human IL-4RA as part of an anti-human IL-4RA antibody, the antibody further comprising a sequence respectively selected from the sequences set forth in SEQ ID NOs: 2, 6, 22, 26, 30, 34 and 38, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences;
an isolated polynucleotide encoding a polypeptide comprising a sequence selected from the sequence set forth in SEQ ID NO: 44, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as
part of an anti-human IL-4RA antibody, the antibody further comprising a sequence selected from the sequence set forth in SEQ ID NO: 42, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polynucleotide encoding a polypeptide comprising a sequence selected from the sequence set forth in SEQ ID NO: 48, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as
part of an anti-human IL-4RA antibody, the antibody further comprising a sequence selected from the sequence set forth in SEQ ID NO: 42, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polynucleotide encoding a polypeptide comprising a sequence selected from the sequence set forth in SEQ ID NO: 48, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 46, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polynucleotide encoding a polypeptide comprising a sequence selected from the sequence set forth in SEQ ID NO: 28, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as
part of an anti-human IL-4RA antibody, the antibody further comprising a sequence selected from the sequence set forth in SEQ ID NO: 34, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
an isolated polynucleotide encoding a polypeptide comprising a sequence selected from the sequence set forth in SEQ ID NO: 127, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as
part of an anti-human IL-4RA antibody, the antibody further comprising a sequence selected from the sequence set forth in SEQ ID NO: 129, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence; or
an isolated polynucleotide encoding a polypeptide comprising a sequence selected from the sequence set forth in SEQ ID NO: 129, a sequence having at least 80%, 85% or 90%,
preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as
part of an anti-human IL-4RA antibody, the antibody further comprising a sequence selected from the sequence set forth in SEQ ID NO: 127, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence.

In particular, the polynucleotide molecule comprises or consists of the sequences set forth in SEQ ID NO: 1, SEQ ID NO: 5, SEQ ID NO: 21, SEQ ID NO: 25, SEQ ID NO: 29, SEQ ID NO: 33, SEQ ID NO: 37, SEQ ID NO: 41, SEQ ID NO: 45 or SEQ ID NO: 126, or a sequence having at least 80%, 85% or 90%, preferably at least 91%, 92%, 93%, 94%, 95%, 96%, 97%, 98% or 99% sequence identity to the sequences.

In particular, the polynucleotide molecule comprises or consists of the sequences set forth in SEQ ID NO: 3, SEQ ID NO: 7, SEQ ID NO: 23, SEQ ID NO: 27, SEQ ID NO: 31, SEQ ID NO: 35, SEQ ID NO: 39, SEQ ID NO: 43, SEQ ID NO: 47 or SEQ ID NO: 128, or a sequence having at least 80%, 85% or 90%, preferably at least 91%, 92%, 93%, 94%, 95%, 96%, 97%, 98% or 99% sequence identity to the sequences.

Yet another aspect of the present invention relates to a vector comprising any one of the polynucleotide molecules disclosed herein.

Yet another aspect of the present invention relates to a host cell comprising any one of the polynucleotide molecules disclosed herein, or the vector disclosed herein.

Yet another aspect of the present invention relates to a method for preparing any one of the antibodies or the antigen-binding fragments thereof disclosed herein, comprising culturing the host cell disclosed herein in a suitable condition, and isolating the antibody or the antigen-binding fragment thereof from the cell cultures.

One aspect of the present invention further provides an antibody conjugate comprising the anti-human IL-4RA antibody or the antigen-binding fragment thereof, and a conjugated moiety coupled thereto, wherein the conjugated moiety is a purification tag (e.g., a His tag), a cytotoxic agent or a detectable label. Preferably, the conjugated moiety is a radioisotope, a luminescent substance, a colored substance, an enzyme or polyethylene glycol.

One aspect of the present invention further provides a multispecific antibody, preferably a bispecific antibody, comprising the anti-human IL-4RA antibody or the antigen-binding fragment thereof, and an antibody or an antigen-binding fragment against another antigen and/or another antigenic epitope.

One aspect the present invention further provides a fusion protein comprising any one of the anti-human IL-4RA antibodies or the antigen-binding fragments thereof disclosed herein. One aspect the present invention further provides a kit comprising any one of the antibodies or the antigen-binding fragments thereof disclosed herein, or the antibody conjugate or the multispecific antibody disclosed herein.

Preferably, the kit further comprises a second antibody that specifically recognizes the antibody or the antigen-binding fragment thereof; optionally, the second antibody further comprises a detectable label, such as a radioisotope, a luminescent substance, a colored substance, an enzyme or polyethylene glycol.

Yet another aspect of the present invention relates to use of any one of the antibodies or the antigen-binding fragments thereof disclosed herein, or the antibody conjugate or the multispecific antibody disclosed herein in preparing a kit for detecting the presence or level of human IL-4RA in a sample or for the prevention and/or treatment and/or adjuvant treatment and/or diagnosis of an allergic disease, a tumor, an autoimmune disease, a skin infection, tissue fibrosis, rhinosinusitis, nasal polyps and chronic obstructive pulmonary disease, wherein preferably the allergic disease is selected from atopic dermatitis, allergic rhinitis, asthma and allergy, and more preferably, atopic dermatitis includes moderate and severe atopic dermatitis.

Yet another aspect of the present invention relates to a pharmaceutical composition comprising any one of the antibodies or the antigen-binding fragments thereof disclosed herein or the antibody conjugate, the multispecific antibody or the fusion protein disclosed herein, and optionally, a pharmaceutically acceptable carrier and/or excipient. Preferably, the pharmaceutical composition is used alone or in combination with one or more medicaments. When the pharmaceutical composition cannot be directly mixed with the combined medicament, the pharmaceutical composition and the combined medicament are separately present in the kit.

Yet another aspect of the present invention relates to use of any one of the antibodies or the antigen binding fragments thereof disclosed herein or the antibody conjugate, the multispecific antibody or the fusion protein disclosed herein in preparing:
a medicament for blocking the binding of human IL-4RA to IL-4 or IL-13,
a medicament for blocking the activity of human IL-4RA or down-regulating the level of human IL-4RA, and
a medicament for blocking a cellular biological response mediated by the binding of human IL-4 or human IL-13 to IL-4RA;
wherein preferably, the ligand of human IL-4RA is human IL-4 or human IL-13, more preferably, human IL-4.

One aspect of the present invention relates to use of any one of the antibodies or the antigen binding fragments thereof disclosed herein or the antibody conjugate, the multispecific antibody or the fusion protein disclosed herein in preparing a medicament for treating a disease selected from: an atopic dermatitis including moderate and severe atopic dermatitis; nasal polyps; asthma; a skin infection; an autoimmune disease, and the like.

Yet another aspect of the present invention relates to an *in vivo* or *in vitro* method comprising administering a cell comprising the antibody or the antigen-binding fragment thereof disclosed herein, the antibody conjugate, the multispecific antibody or the fusion protein disclosed herein, or administering to a subject in need an effective amount of any one of the antibodies or the antigen-binding fragments thereof or the antibody conjugate, the multispecific antibody or the fusion protein disclosed herein. The method is selected from:
a method for blocking the binding of IL-4RA to IL-4 or IL-13,
a method for down-regulating IL-4RA activity or level, or
a method for blocking a cellular biological response mediated by the binding of human IL-4 or human IL-13 to IL-4RA;
wherein preferably, the ligand of IL-4RA is IL-4 or IL-13, more preferably, IL-4.

In one embodiment of the present invention, the *in vitro* method is non-therapeutic and/or non-diagnostic.

Yet another aspect of the present invention relates to use of any one of the antibodies or the antigen-binding fragments thereof or the antibody conjugate or the multispecific antibody disclosed herein in preparing a medicament for the prevention and/or treatment and/or adjuvant treatment and/or diagnosis of an allergic disease, a tumor, a skin infection, an autoimmune disease, tissue fibrosis, rhinosinusitis, nasal polyps and chronic obstructive pulmonary disease. In particular, the allergic disease is selected from atopic dermatitis, allergic rhinitis, asthma and allergy.

In one embodiment of the present invention, the medicament is in a dosage form suitable for oral administration to the gastrointestinal (GI) tract. Preferably the dosage form is selected from tablets, capsules, pills, powders, granules, emulsions, microemulsions, solutions, suspensions, syrups and elixirs.

In one embodiment of the present invention, the medicament is in a form suitable for administration by subcutaneous injection, intradermal injection, intravenous injection, intramuscular injection or intralesional injection.

Yet another aspect of the present invention relates to a method for the prevention and/or treatment and/or adjuvant treatment and/or diagnosis of an allergic disease, a tumor, an autoimmune disease, tissue fibrosis, a skin infection, rhinosinusitis, nasal polyps, chronic obstructive pulmonary disease, comprising administering any of the antibody or the antigen-binding fragment thereof, the antibody conjugate, or the multispecific antibody disclosed herein to a subject in need. In particular, the allergic disease is selected from atopic dermatitis, allergic rhinitis, asthma and allergy.

In the present invention, unless otherwise defined, the scientific and technical terms used herein have the meanings generally understood by those skilled in the art. In addition, the laboratory operations of cell culture, molecular genetics, nucleic acid chemistry and immunology used in the present invention are the routine operations widely used in the corresponding fields. Meanwhile, in order to better understand the present invention, the definitions and explanations of the relevant terms are provided below.

As used herein, the term "antigen-binding fragment" means a protein or portion of a protein that specifically binds to a given antigen. For example, a portion of an antibody comprising amino acid residues that interact with an antigen and confer the antibody the specificity and affinity for the antigen is referred to as an "antigen-binding fragment". The antigen-binding fragment generally comprises one or more complementary-determining regions (CDRs). Certain antigen-binding fragments further comprise one or more framework regions (FRs). CDRs are amino acid sequences that contribute to antigen binding specificity and affinity. As used herein, the term "antibody" refers to an intact immunoglobulin of any isotype or an antigen-binding fragment thereof that can compete with an intact antibody for specifically binding to a target antigen, and includes, for example, chimeric, humanized, fully human, and bispecific antibodies or antigen-binding fragments thereof. Such "antibodies" are antigen-binding proteins. An intact antibody generally comprises at least two full-length heavy chains and two full-length light chains, but, in some cases, may comprise fewer chains, such as an antibody naturally occurring in Camelidae species that may comprise only a heavy chain. An antibody or antigen-binding fragment thereof may be derived from a single source only, or may be "chimeric", i.e., different portions of an antibody may be derived from two different sources as further described below. Antibodies or antigen-binding fragments thereof can be produced in hybridomas by recombinant DNA techniques, or by enzymatic or chemical cleavage of intact antibodies. Unless otherwise indicated, the term "antibody", in addition to antibodies comprising two full-length heavy chains and two full-length light chains, also includes derivatives, variants and fragments thereof.

As used herein, the term "antigen-binding fragment" (or simply "fragment") of an "antibody" or "immunoglobulin chain" (heavy or light chain) includes a portion of an antibody (whether obtained or synthesized) that lacks at least some of the amino acid residues present in the full length of the antibody but is capable of specifically binding the antigen. Such fragments are biologically active as they specifically bind to a target antigen and can compete with other antibodies or antigen binding fragments thereof for specifically binding to a given epitope. In one aspect, such fragments will retain at least one CDR present in the full-length light or heavy chain of the antibody, and in some embodiments, will comprise a single heavy and/or light chain or a portion thereof. Such biologically active fragments can be produced by recombinant DNA techniques, or, for example, by enzymatic or chemical cleavage of intact antibodies. Immunologically functional immunoglobulin fragments include, but are not limited to, Fab, Fab', F(ab')₂, Fv, domain antibodies, and single-chain antibodies, and can be derived from any mammalian source, including, but not limited to, human, mouse, rat, Camelidae species, and rabbit. It is further contemplated that a functional portion of an antibody disclosed herein, such as one or more CDRs, can be covalently bound to a second protein or a small molecule to generate a therapeutic agent that targets a particular target in the body, thereby having bifunctional therapeutic properties or having an extended serum half-life, such as a fusion protein.

As used herein, the terms "antibody full-length chain", "full-length antibody", "intact antibody" and "whole antibody" are used interchangeably herein to refer to such an antibody having a substantially similar structure to a natural antibody structure or having a heavy chain that comprises an Fc region as defined herein.

The term "light chain" includes full-length light chains and fragments thereof having a sufficient variable region sequence to confer the binding specificity. The full-length light chain comprises a variable region domain VL and a constant region domain CL. The variable region domain of the light chain is at the amino terminus of the polypeptide. Light chains include kappa (κ) and lambda (λ) chains.

The term "heavy chain" includes full-length heavy chains and fragments thereof having a sufficient variable region sequence to confer the binding specificity. The full-length heavy chain comprises a variable region domain VH and 3 constant region domains CHI, CH2 and CH3. The VH domain is at the amino terminus of the polypeptide and the CH domains are at the carboxyl terminus, in which CH3 is closest to the carboxyl terminus of the polypeptide. The heavy chain may be of any isotype, including IgG (including IgG1, IgG2, IgG3 and IgG4 subtypes), IgA (including IgA1 and IgA2 subtypes), IgM and IgE.

As used herein, the term "Fab" fragment consists of one light chain, CH1 and the variable region of one heavy chain. The heavy chain of a Fab molecule cannot form disulfide bonds with another heavy chain molecule.

As used herein, the term "Fc" region comprises two heavy chain fragments comprising the CH1 and CH2 domains of an antibody. The two heavy chain fragments are held together by two or more disulfide bonds and the hydrophobic interaction of the CH3 domains.

As used herein, the term "Fab'" fragment comprises portions of one light chain and one heavy chain including the VH domain and the CH1 domain and the region between the CH1 and CH2 domains) such that interchain disulfide bonds can be formed between the two heavy chains of two Fab' fragments to give an F(ab')₂ molecule.

As used herein, the term "F(ab')₂" fragment comprises two light chains and two heavy chains containing portions of the constant region between the CH1 and CH2 domains such that interchain disulfide bonds are formed between the two heavy chains. Thus the F(ab')₂ fragment consists of two Fab' fragments held together by disulfide bonds between the two heavy chains.

As used herein, the term "Fv" region comprises the variable regions from the heavy and light chains, but lacks the constant regions.

As used herein, the term "Fd" fragment refers to an antibody fragment consisting of VH and CH1 domains (Ward et al., Nature, 341:544-546 (1989)).

As used herein, the term "dAb" fragment consists of a VH domain (Ward et al., Nature 341:544-546 (1989)).

As used herein, the term "Fab'-SH" is the designation herein for Fab', wherein one or more cysteine residues of the constant domain carry a free thiol group.

As used herein, the term "Fab/c" fragment is an intermediate formed by pepsin digestion of an immunoglobulin, which combines the advantages of Fab and Fc regions, i.e., strong diffusibility and low metabolic clearance *in vivo,* while retaining high affinity (Liu Jianjun, Chinese Journal of Cellular and Molecular Immunology, 1989(4):29-29).

As used herein, the term "single-chain antibody" is an Fv molecule in which the heavy and light chain variable regions are connected by a flexible linker to form a single polypeptide chain (which forms an antigen binding region) (see, e.g., Bird et al., Science, 242:423-426 (1988), and Huston et al., Proc. Natl. Acad. Sci., USA, 90:5879-5883 (1988)). Single-chain antibodies are described in detail in International Patent Publication No. WO88/01649 and U.S. Pat. Nos. 4,946,778 and 5,260,203, the disclosures of which are incorporated herein by reference.

As used herein, the term "domain antibody" is an immunofunctional immunoglobulin fragment that comprises only the variable region of the heavy chain or the light chain. In some cases, two or more VH regions are covalently linked by a peptide linker to generate a multivalent domain antibody (particularly a bivalent domain antibody). The two VH regions of the bivalent domain antibody may target the same or different antigens.

As used herein, the term "bivalent antigen-binding protein" or "bivalent antibody" comprises two antigen-binding sites. In some cases, the two binding sites have specificity against the same antigen. The bivalent antibody may be bispecific.

As used herein, the term "multispecific antigen-binding protein" or "multispecific antibody" is an antigen-binding protein or antibody that targets more than one antigen or epitope.

As used herein, the term "bispecific", "dual-specificity" or "bifunctional" antigen-binding protein or antibody is a hybrid antigen-binding protein or antibody having two different antigen-binding sites, respectively. A bispecific antibody is a multispecific antigen-binding protein or multispecific antibody, and can be produced by a variety of methods, including but not limited to, fusion of hybridomas or linkage of Fab' fragments. See, e.g., Songsivilai and Lachmann, 1990, Clin. Exp. Immunol., 79:315-321; Kostelny et al., 1992, J. Immunol., 148:1547-1553. The two binding sites of a bispecific antigen-binding protein or antibody will bind to two different epitopes that are present in the same or different protein targets. As used in the present invention, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody has a high specificity for a single epitope on an antigen. The polyclonal antibody, relative to the monoclonal antibody, generally comprises at least two or more different antibodies which generally recognize different epitopes on an antigen. Monoclonal antibodies can generally be obtained using hybridoma technique first reported by Kohler et al. (Nature, 256:495, 1975), but can also be obtained using recombinant DNA technique (see, e.g., U.S. Pat. No. 4,816,567).

As used in the present invention, the term "humanized antibody" refers to an antibody or antibody fragment obtained when all or a part of CDR regions of a human immunoglobulin (receptor antibody) are replaced by the CDR regions of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, for example, Jones et al., Nature, 321:522-525 (1986); Reichmann et al., Nature, 332:323-329 (1988); Presta, Curr. Op. Struct. Biol., 2:593-596 (1992); and Clark, Immunol. Today, 21:397-402 (2000).

As used herein, the term "epitope" refers to a site on an antigen at which an immunoglobulin or antibody specifically binds. "Epitope" is also called in the field as an "antigenic determinant". The epitope or antigenic determinant generally consists of chemically active surface groups of molecules such as amino acids, carbohydrates or sugar side chains, and usually has specific three-dimensional structural characteristics and specific charge characteristics. For example, the epitope generally includes at least 3, 4, 5, 6, 7, 8, 9, 10, 11, 12, 13, 14, or 15 consecutive or non-consecutive amino acids in a unique spatial conformation, which can be "linear" or "conformational". See, for example, Epitope Mapping Protocols in Methods in Molecular Biology, Vol. 66, G. E. Morris, Ed. (1996). In a linear epitope, all interaction sites between a protein and an interaction molecule (e.g., an antibody) are located adjacently along the primary amino acid sequence of the protein. In a conformational epitope, the interaction sites are located across sections of the amino acid residue.

The terms "polypeptide" or "protein" are used interchangeably herein to refer to a polymer of amino acid residues. The term is also used to refer to an amino acid polymer in which one or more amino acid residues are analogs or mimetics of naturally existing amino acids, and for naturally existing amino acid polymers. The term may also include, for example, amino acid polymers that have been modified by addition of saccharide residues to form glycoproteins, or have been phosphorylated. Polypeptides and proteins can be produced by naturally existing cells and non-recombinant cells; or they may be produced by genetically engineered or recombinant cells, and comprise a molecule having the amino acid sequence of a native protein or a molecule having deletions, insertions and/or substitutions in one or more amino acids of the native sequence.

In particular, the terms "polypeptide" and "protein" include antibodies, such as anti-human IL-4RA antibodies (also referred to as IL-4RA antibodies), IL-4RA-binding proteins, antibodies or sequences having deletions, insertions, and/or substitutions in one or more amino acids of an antigen-binding protein.

The term "polypeptide fragment" refers to a polypeptide having amino-terminal deletions, carboxyl-terminal deletions, and/or internal deletions as compared to a full-length protein. Such fragments may also contain modified amino acids as compared to the full-length protein. In certain embodiments, such fragments are about 5 to 500 amino acids in length. For example, a fragment can be at least 5, 6, 8, 10, 14, 20, 50, 70, 100, 110, 150, 200, 250, 300, 350, 400 or 450 amino acids in length. Useful polypeptide fragments include immunologically functional fragments of antibodies, including binding domains. In the case of human IL-4RA antibodies, useful fragments include but are not limited to, CDR regions, variable domains of heavy or light chains, portions of antibody chains, variable domains just comprising 2 CDRs, or the like.

The terms "human IL-4RA", "hIL-4RA", "human IL-4 receptor A", "human IL-4 receptor alpha subunit" are used interchangeably to refer to human interleukin-4 receptor alpha subunit. Human IL-4RA refers to its mature peptide (Genbank ID: NP_001244336.1). IL-4 and IL-13 are the main endogenous agonists of IL-4RA. Unless otherwise indicated or clear from the context in which the term is used, "IL-4RA" refers to human IL-4RA

A "derivative" of a polypeptide is a polypeptide (e.g., an antigen-binding protein or antibody) that is chemically modified in other manners than insertion, deletion or substitution, e.g., by conjugation with another chemical moiety, e.g., a PEG-conjugated polypeptide.

As used herein, the term "isolated" refers to "obtained by artificial means from a natural state". If a certain "isolated" substance or component appears in nature, it may be that change occurs in its natural environment, or that it is isolated from the natural environment, or both. For example, a certain non-isolated polynucleotide or polypeptide naturally exists in a certain living animal, and the same polynucleotide or polypeptide with a high purity isolated from such a natural state is called isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows for the expression of the protein encoded by the inserted polynucleotide, the vector is called an expression vector. A vector can be introduced into a host cell by transformation, transduction, or transfection so that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or PI-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector can contain a variety of elements that control expression, including, but not limited to promoter sequences, transcription initiation sequences, enhancer sequences, selection elements, and reporter genes. In addition, the vector may further contain a replication initiation site.

As used herein, the term "host cell" refers to cells that can be introduced with vectors, including, but not limited to, prokaryotic cells such as *E. coli* or *bacillus subtilis,* fungal cells such as yeast cells or *aspergillus,* insect cells such as S2 drosophila cells or Sf9, or animal cells such as fibroblasts, CHO cells, COS cells, NSO cells, HeLa cells, BHK cells, HEK 293 cells, or human cells.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) refers to that the antibody binds to the antigen with an affinity (K_{D}) of less than about 10⁻⁵ M, such as less than about 10⁻⁶ M, 10⁻⁷ M, 10⁻⁸ M, 10⁻⁹ M or 10⁻¹⁰ M or less.

As used herein, the term "K_{D}" refers to a dissociation equilibrium constant for a specific antibody-antigen interaction, which is used to describe the binding affinity between the antibody and the antigen. Among the several parameters measured by molecular binding kinetics, the K_{D} value is the dissociation equilibrium constant. In an antibody drug research, it is the parameter characterizing the intensity of the affinity effect of an antibody of interest and the target antigen molecule, and is calculated by the formula: K_{D} = k_{dis}/kₒₙ. A smaller equilibrium dissociation constant indicates a more intensive antibody-antigen binding and a higher affinity between the antibody and the antigen. kₒₙ (association rate constant) is the rate of antigen-antibody complex formation, and a smaller kₒₙ suggests a faster binding of an antibody to an antigen. k_{dis} (dissociation rate constant) is the rate at which an antibody dissociates from an antigen-antibody complex, and a smaller k_{dis} suggests a slower rate for the antibody dissociating from the antigen and a firmer binding between the antibody and the antigen. Generally, an antibody binds to an antigen (e.g., L1 protein) with a dissociation equilibrium constant (K_{D}) of less than about 10⁻⁵ M, such as less than about 10⁻⁶ M, 10⁻⁷ M, 10⁻⁸ M, 10⁻⁹ M or 10⁻¹⁰ M or less, for example, as determined on a BIACORE surface plasmon resonance (SPR) instrument or a ForteBio molecular interaction instrument.

As used herein, the terms "monoclonal antibody" and "McAb" have the same meaning and can be used interchangeably; the terms "polyclonal antibody" and "PcAb" have the same meaning and can be used interchangeably; the terms "polypeptide" and "protein" have the same meaning and can be used interchangeably. Besides, herein, amino acids are generally represented by single-letter and three-letter abbreviations known in the art. For example, alanine can be represented by A or Ala.

As used herein, the terms "hybridoma" and "hybridoma cells" can be used interchangeably, and when referring to the terms "hybridoma" and "hybridoma cells", subclones and progeny cells of the hybridoma are also included.

As used herein, the terms "percent sequence identity" and "percent sequence homology" are used interchangeably.

As used herein, the terms "similarity", "sequence similarity" and "identity" refers to the correlation of the sequences of two or more protein or polypeptide molecules, as determined by aligning and comparing the sequences. "Percent identity" refers to the percentage of identical amino acid residues in the molecules compared, and can be calculated based on the size of the smallest molecule for comparison. For such calculations, gaps in the alignment (if any) must be addressed by a particular mathematical model or computer program (i.e., an "algorithm"). The term "substantial identity", when used for polypeptides, refers to that two peptide sequences, when optimally aligned, for example using the programs GAP or BESTFIT, using default gap weights provided by the programs, have at least 70%, 75% or 80% sequence identity, at least 90% or 95% sequence identity, or at least 97%, 98% or 99% sequence identity. In some cases, residue positions that are not identical differ by conservative amino acid substitutions. "Conservative amino acid substitution" is one in which the amino acid residue is replaced with another amino acid residue having a side chain R group that possesses similar chemical properties (e.g., charge or hydrophilicity). Generally, conservative amino acid substitutions will substantially retain the functions and properties of the protein. In cases where two or more amino acid sequences differ from each other by conservative substitutions, the percent sequence identity may be elevated to correct for the conservative nature of the substitution. Methods for making this adjustment are well known to those skilled in the art. See, e.g., Pearson, Methods Mol. Biol., 243:307-31 (1994). Examples of groups of amino acids having side chains with similar chemical properties include: 1) aliphatic hydroxy side chain: glycine, alanine, valine, leucine and isoleucine, 2) aliphatic hydroxy side chain: serine and threonine, 3) amide-containing side chain: asparagine and glutamine, 4) aromatic side chain: phenylalanine, tyrosine and tryptophan, 5) basic side chain: lysine, arginine and histidine, 6) acidic side chain: aspartic acid and glutamic acid, and 7) sulfur-containing side chain: cysteine and methionine. The conservative amino acid substitution groups are valine-leucine-isoleucine, phenylalanine-tyrosine, lysine-arginine, alanine-valine, glutamic acid-aspartic acid and asparagine-glutamine

Optionally, a conservative substitution is any change with a positive value in the PAM250 log-likelihood matrix disclosed in Gonnet et al., Science, 256:1443-45 (1992), which is incorporated herein by reference. A "moderately conservative" substitution is any change with a non-negative value in the PAM250 log-likelihood matrix.

Sequence identity of polypeptides is usually measured by sequence analysis software. Protein analysis software matches sequences using a measure of similarity assigned to different substitutions, deletions and other modifications (including conservative amino acid substitutions). For example, GCG, including programs such as "Gap" and "Bestfit" which (using default parameters specified by the program) can be used to determine sequence homology or sequence identity between closely related polypeptides (e.g., homologous polypeptides from different biological species) or between a wild-type protein and its mutant protein. See, for example, GCG Version 6.1 (University of Wisconsin, WI). Polypeptide sequences can also be compared using FASTA with default or recommended parameters. See GCG Version 6.10 FASTA (e.g., FASTA2 and FASTA3) which provides alignments for regions of optimal overlap between the challenge and query sequences and percent sequence identities (Pearson, Methods Enzymol. 183:63-98 (1990); Pearson, Methods Mol. Biol., 132:185-219 (2000)). Another preferred algorithm when comparing sequences to a database containing massive sequences from different organisms is the computer program BLAST, in particular, blastp or tblastn (using default parameters provided by the program). See, for example, Altschul et al., Mol. Biol., 215:403-410 (1990); Al tschul et al., Nucleic Acids Res., 25:3389-402 (1997).

Compared with the prior art, the present invention has the following advantages:
The anti-human IL-4RA antibody disclosed herein can bind to human IL-4RA with high affinity, and inhibit relevant cellular biological effects mediated by human IL-4 and human IL-13, such as cell proliferation, IL-4 and IL-13 induced CD23 expression level up-regulation and the like, by blocking the binding of human IL-4 and human IL-13 to human IL-4RA. The antibody has advantages of high activity, exclusion of species difference and the like, and can be used in preparing medicaments for blocking the binding of human IL-4 and IL-13 to human IL-4RA and in preparing medicaments for treating or preventing allergic diseases such as allergic rhinitis, asthma, allergy and atopic dermatitis (including moderate and severe atopic dermatitis), sinusitis, nasal polyps, chronic obstructive pulmonary disease, tissue fibrosis, and autoimmune diseases, thus having prospect for application and marketing.

### BRIEF DESCRIPTION OF THE DRAWINGS

FIG. 1: Binding activity of 13E5 and dupilumab to antigen IL4RA-mFc.
FIG. 2: Binding activity of 13E5 H1L1, 13E5 H2L2, 13E5 H3L3 and dupilumab to antigen IL4RA-mFc.
FIG. 3: Binding activity of 13E5 H4L2, 13E5 H4L4 and dupilumab to antigen IL4RA-mFc.
FIG. 4: Activity of 13E5 and dupilumab in competing with human IL4-N-His for binding to human IL4RA-mFc.
FIG. 5: Activity of 13E5 H1L1, 13E5 H2L2, 13E5 H3L3 and dupilumab in competing with human IL4-N-His for binding to human IL4RA-mFc.
FIG. 6: Activity of 13E5 H4L2, 13E5 H4L4 and dupilumab in competing with human IL4-N-His for binding to human IL4RA-mFc.
FIG. 7: Binding activity of 18H7 and dupilumab to antigen IL4RA-mFc.
FIG. 8: Binding activity of 18H7 H1L1, 18H7 H2L2, 18H7 H2L3, 18H7 H3L2, 18H7 H3L3 and dupilumab to antigen IL4RA-mFc.
FIG. 9: Activity assay of 18H7 and dupilumab in competing with human IL4-N-His for binding to human IL4RA-mFc.
FIG. 10: Activity assay of 18H7 H1L1, 18H7 H2L2, 18H7 H2L3, 18H7 H3L2 and dupilumab in competing with human IL4-N-His for binding to human IL4RA-mFc.
FIG. 11: Activity assay of 20G10 H3L3, 13E5 H4L4 and dupilumab in competing with human IL4-N-His for binding to human IL4RA-mFc.
FIG. 12: Affinity constant assay of 13E5 H4L4 to human IL4RA. The antibody concentrations for the curve pairs from top to bottom were 25 nM, 6.25 nM, 3.13 nM, 1.56 nM, 0.78 nM and 0.39 nM, respectively.
FIG. 13: Affinity constant assay of 18H7 H1L1 to human IL4RA. The antibody concentrations for the curve pairs from top to bottom were 25 nM, 6.25 nM, 3.13 nM, 1.56 nM, 0.78 nM and 0.39 nM, respectively.
FIG. 14: Affinity constant assay of dupilumab to human IL4RA. The antibody concentrations for the curve pairs from top to bottom were 12.5 nM, 6.25 nM, 3.13 nM, 1.56 nM, 0.78 nM and 0.39 nM, respectively.
FIG. 15: Affinity constant assay of 13E5 H1L1 to human IL4RA. The antibody concentrations for the curve pairs from top to bottom were 25 nM, 12.5 nM, 6.25 nM, 3.13 nM, 1.56 nM, 0.78 nM and 0.39 nM, respectively.
FIG. 16: Affinity constant assay of 13E5 H2L2 to human IL4RA. The antibody concentrations for the curve pairs from top to bottom were 25 nM, 12.5 nM, 6.25 nM, 3.13 nM, 1.56 nM, 0.78 nM and 0.39 nM, respectively.
FIG. 17: Affinity constant assay of 13E5 H3L3 to human IL4RA. The antibody concentrations for the curve pairs from top to bottom were 25 nM, 12.5 nM, 6.25 nM, 3.13 nM, 1.56 nM and 0.39 nM, respectively.
FIG. 18: Affinity constant assay of 13E5 H4L2 to human IL4RA. The antibody concentrations for the curve pairs from top to bottom were 12.5 nM, 6.25 nM, 3.13 nM, 1.56 nM, 0.78 nM and 0.39 nM, respectively.
FIG. 19: Affinity constant assay of 18H7 H2L2 to human IL4RA. The antibody concentrations for the curve pairs from top to bottom were 6.25 nM, 3.13 nM, 1.56 nM, 0.78 nM and 0.39 nM, respectively.
FIG. 20: Affinity constant assay of 18H7 H3L2 to human IL4RA. The antibody concentrations for the curve pairs from top to bottom were 25 nM, 12.5 nM, 6.25 nM, 3.13 nM, 1.56 nM, 0.78 nM and 0.39 nM, respectively.
FIG. 21: Affinity constant assay of dupilumab to human IL4RA. The antibody concentrations for the curve pairs from top to bottom were 6.25 nM, 3.13 nM, 1.56 nM, 0.78 nM and 0.39 nM, respectively.
FIG. 22: 13E5 H1L1, 13E5 H2L2 and dupilumab inhibiting IL-4-induced TF-1 cell proliferation.
FIG. 23: 13E5 H1L1, 13E5 H2L2 and dupilumab inhibiting IL-13-induced TF-1 cell proliferation.
FIG. 24: 13E5 H4L2, 13E5 H4L4 and dupilumab inhibiting IL-4-induced TF-1 cell proliferation.
FIG. 25: 13E5 H4L2, 13E5 H4L4 and dupilumab inhibiting IL-13-induced TF-1 cell proliferation.
FIG. 26: 18H7 H1L1, 18H7 H2L2 and dupilumab inhibiting IL-4-induced TF-1 cell proliferation.
FIG. 27: 18H7 H1L1, 18H7 H2L2 and dupilumab inhibiting IL-13-induced TF-1 cell proliferation.
FIG. 28: 18H7 H1L1, 13E5 H4L4 and dupilumab inhibiting IL-4-induced CD23 expression up-regulation in monocytes.
FIG. 29: 18H7 H1L1, 13E5 H4L4 and dupilumab inhibiting IL-13-induced CD23 expression up-regulation in monocytes.
FIG. 30: 13E5 H4L4 inhibiting increase of epidermal thickness in B-hIL4Ra mouse skin inflammation model.
FIG. 31: Pathological sections showing epidermal thickness in mice of all experimental groups (HE staining, 400×). a. control (normal saline); b. dupilumab (80 mg/kg); c. dupilumab (20 mg/kg); d. hIgG4 (80 mg/kg); e. 13E5 H4L4 (80 mg/kg); f. 13E5 H4L4 (20 mg/kg).

### DETAILED DESCRIPTION

The embodiments of the present invention will be described in detail below with reference to the examples. Those skilled in the art will understand that the following examples are only used to illustrate the present invention, and should not be regarded as limiting the scope of the present invention. In the cases where the techniques or conditions are not specified, the examples were carried out according to the techniques or conditions described in the literature in the art (e.g., see, Molecular Cloning: A Laboratory Manual, authored by J. Sambrook et al., and translated by Huang Peitang et al., Third Edition, Science Press) or according to the product manual. Reagents or instruments used are commercially available conventional products if the manufacturers thereof are not specified.

In the following examples of the present invention, BALB/C mice were purchased from Guangdong Medical Laboratory Animal Center.

In the following examples of the present invention, the reference antibody dupilumab VAB 16F3-1 (hereinafter referred to as dupilumab) was produced by Akeso, Inc., the sequence of which can be found in Patent Application No. PCT/US2007/021210 granted to Regeneron Pharmaceuticals, Inc. The heavy chain variable region of antibody VAB 16F3-1 is set forth in SEQ ID NO: 124, with the constant region being an Ig gamma-1 chain C region, ACCESSION No. P01857, while the coding sequence of the light chain variable region of antibody VAB 16F3-1 is set forth in SEQ ID NO: 125, with the constant region being an Ig kappa chain C region, ACCESSION No. P01834).

### Example 1. Preparation of anti-human IL-4RA antibodies 20G10, 13E5 and 18H7

### 1. Preparation of hybridoma cell lines 20G10, 13E5 and 18H7

The antigen IL-4RA-mFc for producing anti-IL-4RA antibody was a fusion protein of human IL-4RA mature peptide (Genbank ID: NP_001244336.1) and mFc tag (SEQ ID NO: 121) synthesized by Akeso, Inc., and was used for immunizing BALB/C mice (purchased from Guangdong Medical Laboratory Animal Center). Spleen cells of immunized BALB/C mice (purchased from Guangdong center for medicine laboratory animals) and mouse myeloma cells were fused to form hybridoma cells with reference to existing cell fusion techniques (e.g., Stewart, S.J., "Monoclonal Antibody Production", in Basic Methods in Antibody Production and Characterization, Eds. G.C. Howard and D.R. Bethell, Boca Raton: CRC Press, 2000). The plate was coated with IL 4RA-hFc protein (IL-4RA is described above, and hFc is a human IgG Fc purification tag, specifically Ig gamma-1 chain C region, Genbank ID: P01857, positions 114-330) for indirect ELISA. By screening, hybridoma cell lines secreting antibodies specifically binding to IL 4RA-hFc were obtained. The hybridoma cell lines obtained by indirect ELISA screening were subjected to competitive ELISA to select hybridoma cell lines secreting monoclonal antibodies that compete with ligand IL4-N-his (IL4 NCBI Gene ID: AAH70123.1) for binding to IL 4RA-hFc. Two hybridoma cell lines stably secreting anti-human IL-4RA antibodies were obtained by limiting dilution. The hybridoma cell lines were named as LT018, LT008 and LT009, and the secreted monoclonal antibodies were named as 20G10, 13E5 and 18H7. Hybridoma cell line LT018 (IL4RA-20G10) was deposited at China Center for Type Culture Collection (CCTCC) on Dec. 25, 2019 with an accession number CCTCC NO: C202010, and a preservation address of Wuhan University, Wuhan, China, postal code: 430072. Hybridoma cell line LT008 (IL4RA-13E5) was deposited at China Center for Type Culture Collection (CCTCC) on Jun. 21, 2018 with an accession number CCTCC NO: C2018131, and a preservation address of Wuhan University, Wuhan, China, postal code: 430072. Hybridoma cell line LT009 (IL4RA-18H7) was deposited at China Center for Type Culture Collection (CCTCC) on Jun. 21, 2018 with an accession number CCTCC NO: C2018132, and a preservation address of Wuhan University, Wuhan, China, postal code: 430072.

### 2. Preparation of anti-human IL-4RA antibodies 20G10, 13E5 and 18H7

The cell lines LT018, LT008 and LT009 prepared as described above were separately cultured with a chemical defined medium (CD medium; containing 1% streptomycin) in a 5% CO₂, 37 °C incubator. After 7 days, the supernatants were collected and purified by high-speed centrifugation, vacuum filtration through a microfiltration membrane and a HiTrap protein A HP column to give antibodies 20G10, 13E5 and 18H7.

### Example 2. Sequence analysis of anti-human IL-4RA antibody 13E5

mRNA was extracted from the cell line LT008 cultured in Example 1 according to the method described in the manual of RNAprep pure Cell/Bacteria Kit (Tiangen, Cat. No. DP430).

cDNA was synthesized according to the manual of Invitrogen SuperScript® III First-Strand Synthesis System for RT-PCR and amplified by PCR.

The PCR-amplified products were directly subjected to TA cloning according to the manual of the pEASY-T1 Cloning Kit (Transgen CT101).

The TA-cloned products were directly sequenced, and the sequencing results are as follows: The nucleic acid sequence of the heavy chain variable region is set forth in SEQ ID NO: 1 with a length of 348 bp. The coded amino acid sequence is set forth in SEQ ID NO: 2 with a length of 116 amino acids, while the sequences of heavy chain CDR1, CDR2 and CDR3 are set forth in SEQ ID NOs: 9, 10 and 11, respectively.

The nucleic acid sequence of the light chain variable region is set forth in SEQ ID NO: 3 with a length of 321 bp. The coded amino acid sequence is set forth in SEQ ID NO: 4 with a length of 107 amino acids, while the sequences of light chain CDR1, CDR2 and CDR3 are set forth in SEQ ID NOs: 12, 13 and 14, respectively.

### Example 3. Design and preparation of humanized anti-human IL-4RA antibodies 13E5 H1L1, 13E5 H2L2, 13E5 H3L3, 13E5 H4L2 and 13E5 H4L4

### 1. Design of light and heavy chain sequences of humanized anti-IL4RA antibodies 13E5 H1L1, 13E5 H2L2, 13E5 H3L3, 13E5 H4L2, and 13E5 H4L4

Based on the three-dimensional crystal structure of human IL-4RA protein (Hage T, Reinemer P, Sebald W., Crystals of a 1:1 Complex Between Human Interleukin-4 and the Extracellular Domain of Its Receptor Alpha Chain, Eur. J. Biochem., 1998; 258(2):831-6.) and the sequence of antibody 13E5 obtained in Example 2, the variable region sequences of antibodies 13E5 H1L1, 13E5 H2L2, 13E5 H3L3, 13E5 H4L2 and 13E5 H4L4 were given by computer modeling and mutation design (antibody constant region sequences from NCBI database: the heavy chain constant region is Ig gamma-4 chain C region, ACCESSION No. P01861.1; the light chain constant region is Ig kappa chain C region, ACCESSION No. P01834).

The designed variable region sequences are as follows:
(1) Heavy and light chain sequences of humanized monoclonal antibody 13E5 H1L1
   The nucleic acid sequence of the heavy chain variable region is set forth in SEQ ID NO: 21 with a length of 348 bp. The coded amino acid sequence is set forth in SEQ ID NO: 22 with a length of 116 amino acids, while the sequences of heavy chain CDR1, CDR2 and CDR3 are set forth in SEQ ID NOs: 9, 10 and 11, respectively.
   The nucleic acid sequence of the light chain variable region is set forth in SEQ ID NO: 23 with a length of 321 bp. The coded amino acid sequence is set forth in SEQ ID NO: 24 with a length of 107 amino acids, while the sequences of light chain CDR1, CDR2 and CDR3 are set forth in SEQ ID NOs: 12, 13 and 14, respectively.
(2) Heavy and light chain sequences of humanized monoclonal antibody 13E5 H2L2
   The nucleic acid sequence of the heavy chain variable region is set forth in SEQ ID NO: 25 with a length of 348 bp. The coded amino acid sequence is set forth in SEQ ID NO: 26 with a length of 116 amino acids, while the sequences of heavy chain CDR1, CDR2 and CDR3 are set forth in SEQ ID NOs: 9, 10 and 11, respectively.
   The nucleic acid sequence of the light chain variable region is set forth in SEQ ID NO: 27 with a length of 321 bp. The coded amino acid sequence is set forth in SEQ ID NO: 28 with a length of 107 amino acids, while the sequences of light chain CDR1, CDR2 and CDR3 are set forth in SEQ ID NOs: 12, 13 and 14, respectively.
(3) Heavy and light chain sequences of humanized monoclonal antibody 13E5 H3L3
   The nucleic acid sequence of the heavy chain variable region is set forth in SEQ ID NO: 29 with a length of 348 bp. The coded amino acid sequence is set forth in SEQ ID NO: 30 with a length of 116 amino acids, while the sequences of heavy chain CDR1, CDR2 and CDR3 are set forth in SEQ ID NOs: 9, 10 and 11, respectively.
   The nucleic acid sequence of the light chain variable region is set forth in SEQ ID NO: 31 with a length of 321 bp. The coded amino acid sequence is set forth in SEQ ID NO: 32 with a length of 107 amino acids, while the sequences of light chain CDR1, CDR2 and CDR3 are set forth in SEQ ID NOs: 12, 13 and 14, respectively.
(4) Heavy and light chain sequences of humanized monoclonal antibody 13E5 H4L4
   The nucleic acid sequence of the heavy chain variable region is set forth in SEQ ID NO: 33 with a length of 348 bp. The coded amino acid sequence is set forth in SEQ ID NO: 34 with a length of 116 amino acids, while the sequences of heavy chain CDR1, CDR2 and CDR3 are set forth in SEQ ID NOs: 9, 10 and 11, respectively.
   The nucleic acid sequence of the light chain variable region is set forth in SEQ ID NO: 35 with a length of 321 bp. The coded amino acid sequence is set forth in SEQ ID NO: 36 with a length of 107 amino acids, while the sequences of light chain CDR1, CDR2 and CDR3 are set forth in SEQ ID NOs: 12, 13 and 14, respectively.
(5) Heavy and light chain sequences of humanized monoclonal antibody 13E5 H4L2
   The nucleic acid sequence of the heavy chain variable region is set forth in SEQ ID NO: 33, and
      the coded amino acid sequence is set forth in SEQ ID NO: 34.
   The nucleic acid sequence of the light chain variable region is set forth in SEQ ID NO: 27, and
      the coded amino acid sequence is set forth in SEQ ID NO: 28.

### 2. Preparation of humanized antibodies 13E5 H1L1, 13E5 H2L2, 13E5 H3L3, 13E5 H4L2 and 13E5 H4L4

The heavy chain constant regions are Ig gamma-4 chain C region, ACCESSION: P01861.1; the light chain constant regions are Ig kappa chain C region, ACCESSION: P01834. The heavy and light chain variable region cDNA of 13E5 H1L1, heavy and light chain variable region cDNA of 13E5 H2L2, heavy and light chain variable region cDNA of 13E5 H3L3, heavy and light chain variable region cDNA of 13E5 H4L2, and heavy and light chain variable region cDNA of 13E5 H4L4 were separately cloned into pUC57 simple vector (provided by Genscript Biotech Corporation) to give pUC57simple-13E5H1, pUC57simple-13E5L1, pUC57simple-13E5H2, pUC57simple-13E5L2, pUC57simple-13E5H3, pUC57simple-13E5L3, pUC57simple-13E5H4 and pUC57simple-13E5L4. The variable region fragments were acquired by digestion according to the standard techniques described in *Molecular Cloning: A Laboratory Manual* (Second Edition), and subcloned into the pcDNA3.1 vectors containing the corresponding heavy or light chain constant region fragment (for the pcDNA3.1 vectors containing the heavy and light chain constant regions, the restriction enzymes were HindIII & EcoRI) to give pcDNA3.1-13E5H1, pcDNA3.1-13E5L1, pcDNA3.1-13E5H2, pcDNA3.1-13E5L2, pcDNA3.1-13E5H3, pcDNA3.1-13E5L3, pcDNA3.1-13E5H4 and pcDNA3.1-13E5L4. The recombinant plasmid pair containing the corresponding light and heavy chains (pcDNA3.1-13E5H1 and pcDNA3.1-13E5L1, pcDNA3.1-13E5H2 and pcDNA3.1-13E5L2, pcDNA3.1-13E5H3 and pcDNA3.1-13E5L3, pcDNA3.1-13E5H4 and pcDNA3.1-13E5L4, and pcDNA3.1-13E5H4 and pcDNA3.1-13E5L2) were co-transfected into 293F cells. The cultures were collected and purified. After the sequences were verified, endotoxin-free expression plasmids were prepared, and were transiently transfected into HEK293 cells for antibody expression. The cultures were collected after 7 days, and purified on a Protein A column to give humanized antibodies 13E5 H1L1, 13E5 H2L2, 13E5 H3L3, 13E5 H4L2 and 13E5 H4L4.

### Example 4. Sequence analysis of anti-human IL-4RA antibody 18H7

mRNA was extracted from the cell line LT009 cultured in Example 1 according to the method described in the manual of RNAprep pure Cell/Bacteria Kit (Tiangen, Cat. No. DP430).

cDNA was synthesized according to the manual of Invitrogen SuperScript® III First-Strand Synthesis System for RT-PCR and amplified by PCR.

The PCR-amplified products were directly subjected to TA cloning according to the manual of the pEASY-T1 Cloning Kit (Transgen CT101).

The TA-cloned products were directly sequenced, and the sequencing results are as follows:

The nucleic acid sequence of the heavy chain variable region is set forth in SEQ ID NO: 5 with a length of 360 bp. The coded amino acid sequence is set forth in SEQ ID NO: 6 with a length of 120 amino acids, while the sequences of heavy chain CDR1, CDR2 and CDR3 are set forth in SEQ ID NOs: 15, 16 and 17, respectively.

The nucleic acid sequence of the light chain variable region is set forth in SEQ ID NO: 7 with a length of 333 bp. The coded amino acid sequence is set forth in SEQ ID NO: 8 with a length of 111 amino acids, while the sequences of light chain CDR1, CDR2 and CDR3 are set forth in SEQ ID NOs: 18, 19 and 20, respectively.

### Example 5. Design and preparation of humanized anti-human IL-4RA antibodies 18H7 H1L1, 18H7 H2L2, 18H7 H2L3, 18H7 H3L2 and 18H7 H3L3

### 1. Design of light and heavy chain sequences of humanized anti-IL-4RA antibodies 18H7 H1L1, 18H7 H2L2, 18H7 H2L3, 18H7 H3L2, and 18H7 H3L3

Based on the three-dimensional crystal structure of human IL-4RA protein (Nat Hage T, Reinemer P, Sebald W., Crystals of a 1:1 Complex Between human Interleukin-4 and the Extracellular Domain of Its Receptor Alpha Chain, Eur. J. Biochem., 1998; 258(2):831-6.) and the sequence of antibody 18H7 obtained in Example 4, the variable region sequences of antibodies 18H7 H1L1, 18H7 H2L2, 18H7 H2L3, 18H7 H3L2 and 18H7 H3L3 were given by computer modeling and mutation design (antibody constant region sequences from NCBI database: the heavy chain constant region is Ig gamma-4 chain C region, ACCESSION No. P01861.1; the light chain constant region is Ig kappa chain C region, ACCESSION No. P01834).

The designed variable region sequences are as follows:
(1) Heavy and light chain sequences of humanized monoclonal antibody 18H7 H1L1
   The nucleic acid sequence of the heavy chain variable region is set forth in SEQ ID NO: 37 with a length of 360 bp. The coded amino acid sequence is set forth in SEQ ID NO: 38 with a length of 120 amino acids, while the sequences of heavy chain CDR1, CDR2 and CDR3 are set forth in SEQ ID NOs: 15, 16 and 17, respectively.
   The nucleic acid sequence of the light chain variable region is set forth in SEQ ID NO: 39 with a length of 333 bp. The coded amino acid sequence is set forth in SEQ ID NO: 40 with a length of 111 amino acids, while the amino acid sequences of light chain CDR1, CDR2 and CDR3 are set forth in SEQ ID NOs: 18, 19 and 20, respectively.
(2) Heavy and light chain sequences of humanized monoclonal antibody 18H7 H2L2
   The nucleic acid sequence of the heavy chain variable region is set forth in SEQ ID NO: 41 with a length of 360 bp. The coded amino acid sequence is set forth in SEQ ID NO: 42 with a length of 120 amino acids, while the sequences of heavy chain CDR1, CDR2 and CDR3 are set forth in SEQ ID NOs: 15, 16 and 17, respectively.
   The nucleic acid sequence of the light chain variable region is set forth in SEQ ID NO: 43 with a length of 333 bp. The coded amino acid sequence is set forth in SEQ ID NO: 44 with a length of 111 amino acids, while the sequences of light chain CDR1, CDR2 and CDR3 are set forth in SEQ ID NOs: 18, 19 and 20, respectively.
(3) Heavy and light chain sequences of humanized monoclonal antibody 18H7 H3L3
   The nucleic acid sequence of the heavy chain variable region is set forth in SEQ ID NO: 45 with a length of 360 bp. The coded amino acid sequence is set forth in SEQ ID NO: 46 with a length of 120 amino acids, while the sequences of heavy chain CDR1, CDR2 and CDR3 are set forth in SEQ ID NOs: 15, 16 and 17, respectively.
   The nucleic acid sequence of the light chain variable region is set forth in SEQ ID NO: 47 with a length of 333 bp. The coded amino acid sequence is set forth in SEQ ID NO: 48 with a length of 111 amino acids, while the sequences of light chain CDR1, CDR2 and CDR3 are set forth in SEQ ID NOs: 18, 19 and 20, respectively.
(4) Heavy and light chain sequences of humanized monoclonal antibody 18H7 H2L3
   The nucleic acid sequence of the heavy chain variable region is set forth in SEQ ID NO: 41, and
      the coded amino acid sequence is set forth in SEQ ID NO: 42.
   The nucleic acid sequence of the light chain variable region is set forth in SEQ ID NO: 47, and
      the coded amino acid sequence is set forth in SEQ ID NO: 48.
(5) Heavy and light chain sequences of humanized monoclonal antibody 18H7 H3L2
   The nucleic acid sequence of the heavy chain variable region is set forth in SEQ ID NO: 45, and
      the coded amino acid sequence is set forth in SEQ ID NO: 46.
   The nucleic acid sequence of the light chain variable region is set forth in SEQ ID NO: 43, and
      the coded amino acid sequence is set forth in SEQ ID NO: 44.

### 2. Preparation of humanized antibodies 18H7 H1L1, 18H7 H2L2, 18H7 H2L3, 18H7 H3L3 and 18H7 H3L2

The heavy chain constant regions are Ig gamma-4 chain C region, ACCESSION: P01861.1; the light chain constant regions are Ig kappa chain C region, ACCESSION: P01834.

The heavy and light chain variable region cDNA of 18H7 H1L1, heavy and light chain variable region cDNA of 18H7 H2L2, heavy and light chain variable region cDNA of 18H7 H2L3, heavy and light chain variable region cDNA of 18H7 H3L3, and heavy and light chain variable region cDNA of 18H7 H3L2 were separately cloned into pUC57 simple vector (provided by Genscript Biotech Corporation) to give pUC57simple-18H7H1, pUC57simple-18H7L1, pUC57simple-18H7H2, pUC57simple-18H7L2, pUC57simple-18H7H3 and pUC57simple-18H7L3. The variable region fragments were acquired by digestion according to the standard techniques described in Molecular Cloning: A Laboratory Manual (Second Edition), and subcloned into the pcDNA3.1 vectors containing the corresponding heavy or light chain constant region fragment to give pcDNA3.1-18H7H1, pcDNA3.1-18H7L1, pcDNA3.1-18H7H2, pcDNA3.1-18H7L2, pcDNA3.1-18H7H3 and pcDNA3.1-18H7L3. The recombinant plasmid pair containing the corresponding light and heavy chains (pcDNA3.1-18H7H1 and pcDNA3.1-18H7L1, pcDNA3.1-18H7H2 and pcDNA3.1-18H7L2, pcDNA3.1-18H7H3 and pcDNA3.1-18H7L3, pcDNA3.1-18H7H2 and pcDNA3.1-18H7L3, and pcDNA3.1-18H7H3 and pcDNA3.1 - 18H7L2) were co-transfected into 293F cells. The cultures were collected and purified. After the sequences were verified, endotoxin-free expression plasmids were prepared, and were transiently transfected into HEK293 cells for antibody expression. The cultures were collected after 7 days, and purified on a Protein A column (GE) to give the humanized antibodies 18H7 H1L1, 18H7 H2L2, 18H7 H2L3, 18H7 H3L2 and 18H7 H3L3.

### Example 6. Design and preparation of humanized anti-human IL-4RA antibody 20G10 H3L3

### 1. Design of light and heavy chain sequences of humanized anti-IL-4RA antibody 20G10 H3L3

Based on the three-dimensional crystal structure of human IL-4RA protein (Nat Hage T, Reinemer P, Sebald W., Crystals of a 1:1 Complex Between human Interleukin-4 and the Extracellular Domain of Its Receptor Alpha Chain, Eur. J. Biochem., 1998; 258(2):831-6.), the variable region sequences of antibody 20G10 H3L3 were given by computer modeling and mutation design (antibody constant region sequences from NCBI database: the heavy chain constant region is Ig gamma-4 chain C region, ACCESSION No. P01861.1; the light chain constant region is Ig kappa chain C region, ACCESSION No. P01834).

The designed variable region sequences are as follows:
(1) Heavy and light chain sequences of humanized monoclonal antibody 20G10 H3L3
   The nucleic acid sequence of the heavy chain variable region is set forth in SEQ ID NO: 126 with a length of 360 bp. The coded amino acid sequence is set forth in SEQ ID NO: 127 with a length of 120 amino acids, while the sequences of heavy chain CDR1, CDR2 and CDR3 are set forth in SEQ ID NO: 130, 131 and 132, respectively.
   The nucleic acid sequence of the light chain variable region is set forth in SEQ ID NO: 128 with a length of 321 bp. The coded amino acid sequence is set forth in SEQ ID NO: 129 with a length of 107 amino acids, while the amino acid sequences of light chain CDR1, CDR2 and CDR3 are set forth in SEQ ID NOs: 133, 134 and 135, respectively.
2. Preparation of humanized antibody 20G10 H3L3
   The heavy chain constant regions are Ig gamma-4 chain C region, ACCESSION: P01861.1; the light chain constant regions are Ig kappa chain C region, ACCESSION: P01834.
   The heavy and light chain variable region cDNA of 20G10 H3L3 were cloned into pUC57simple vector (provided by Genscript Biotech Corporation) to give pUC57simple-20G10H3 and pUC57simple-20G10L3. The variable region fragments were acquired by digestion according to the standard techniques described in Molecular Cloning: A Laboratory Manual (Second Edition), and subcloned into the pcDNA3.1 vectors containing the corresponding heavy or light chain constant region fragment to give pcDNA3.1-20G10H3 and pcDNA3.1-20G10L3. The recombinant plasmid pair containing the corresponding light and heavy chains (pcDNA3.1-20G10H3 and pcDNA3.1-20G10L3) were co-transfected into 293F cells. The cultures were collected and purified. After the sequences were verified, endotoxin-free expression plasmids were prepared, and were transiently transfected into HEK293 cells for antibody expression. The cultures were collected after 7 days, and purified on a Protein A column (GE) to give the humanized antibody 20G10 H3L3.

### Example 7. Binding activity of antibodies to antigens by ELISA

### 1. Binding activity of murine antibody 13E5 and humanized antibodies 13E5 H1L1, 13E5 H2L2, 13E5 H3L3, 13E5 H4L2 and 13E5 H4L4 to antigens human IL4RA-hFc or human IL-4RA-mFc

### 1.1 The binding activity of antibodies 13E5, 13E5 H1L1, 13E5 H2L2, 13E5 H3L3, 13E5 H4L2 and 13E5 H4L4 to the antigens human IL4RA-hFc or human IL4RA-mFc was measured by indirect ELISA.

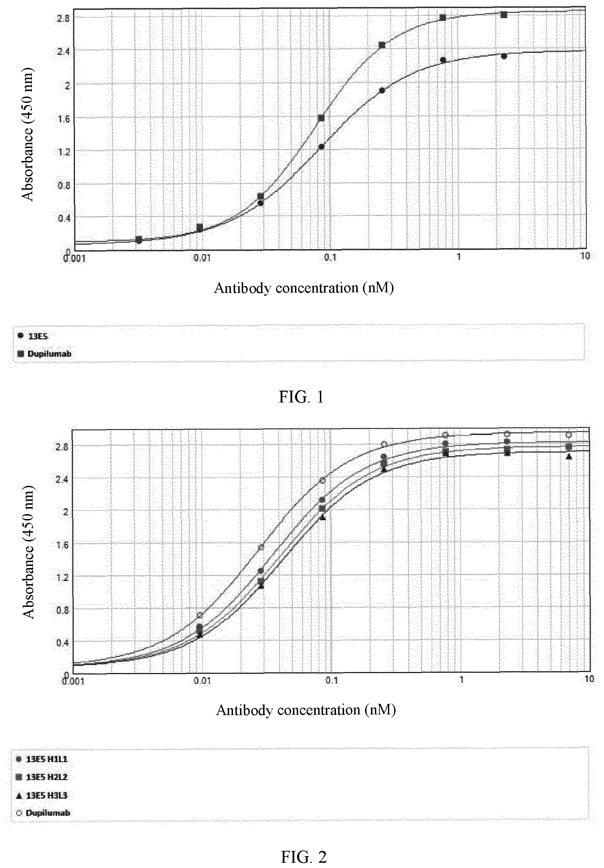

Procedures: The ELISA plates were coated with human IL4RA-hFc or human IL4RA-mFc and were incubated, and target antibodies were added after blocking. Goat anti-mouse IgG (H+L) and HRP (purchased from Jackson ImmunoResearch Inc., Cat No. 109-035-062), or goat anti-human IgG and HRP (purchased from Jackson ImmunoResearch Inc., Cat No. 109-035-088) were added. The plates were incubated and washed before TMB (Neogen, 308177) was added for chromogenic reaction. At the end of the reaction, the absorbance at 450 nm was measured by a plate reader. The data were analyzed by SoftMax Pro 6.2.1. The readings at 450 nm show that antibodies 13E5, 13E5 H1L1, 13E5 H2L2, 13E5 H3L3, 13E5 H4L2 and 13E5 H4L4 are all capable of effectively binding to antigens human IL4RA-hFc or human IL4RA-mFc in a dose-dependent manner. By 4-parameter logistic regression of absorbance vs. antibody concentration, as shown in Tables 1, 2 and 3, 13E5, 13E5 H1L1, 13E5 H2L2, 13E5 H3L3, 13E5 H4L2 and 13E5 H4L4 are all capable of binding to antigens human IL4RA-hFc or human IL4RA-mFc effectively, and demonstrate comparable binding activities to that of the reference antibody dupilumab against the same target (FIGs. 1, 2 and 3).

**Table 1. Binding activity of 13E5 and dupilumab to antigens human IL4RA-hFc or human IL4RA-mFc**

| Antibody dilution (µg/mL) | Antigen (1 µg/mL) | | | |
|---|---|---|---|---|
| | IL4RA-hFc | | IL4RA-mFc | |
| | 13E5 | | Dupilumab | |
| 0.333 | 2.300 | 2.313 | 2.758 | 2.858 |
| 1:3 | 2.318 | 2.215 | 2.748 | 2.801 |
| 1:9 | 1.923 | 1.878 | 2.388 | 2.500 |
| 1:27 | 1.251 | 1.207 | 1.469 | 1.679 |
| 1:81 | 0.558 | 0.563 | 0.599 | 0.673 |
| 1:243 | 0.244 | 0.237 | 0.250 | 0.281 |
| 1:729 | 0.112 | 0.113 | 0.126 | 0.137 |
| 0 | 0.047 | 0.053 | 0.076 | 0.069 |
| Second antibody | Goat anti-mouse IgG (H+L), HRP (1:5000) | | Goat anti-human IgG (H+L), HRP (1:5000) | |
| EC₅₀ (nM) | 0.084 | | 0.077 | |

**Table 2. Binding activity of 13E5 H1L1, 13E5 H2L2, 13E5 H3L3 and dupilumab to antigen IL4RA-mFc**

| Antibody dilution (µg/mL) | Antigen: IL4RA-mFc | | | | | | | |
|---|---|---|---|---|---|---|---|---|
| | 13E5 H1L1 | | 13E5 H2L2 | | 13E5 H3L3 | | Dupilumab | |
| 1 | 2.716 | 2.797 | 2.768 | 2.754 | 2.642 | 2.656 | 2.901 | 2.921 |
| 1:3 | 2.834 | 2.825 | 2.686 | 2.770 | 2.679 | 2.688 | 2.928 | 2.923 |
| 1:9 | 2.802 | 2.798 | 2.692 | 2.735 | 2.658 | 2.717 | 2.909 | 2.921 |
| 1:27 | 2.684 | 2.613 | 2.551 | 2.578 | 2.492 | 2.498 | 2.799 | 2.801 |
| 1:81 | 2.116 | 2.122 | 1.972 | 2.043 | 1.918 | 1.891 | 2.358 | 2.358 |
| 1:243 | 1.214 | 1.297 | 1.104 | 1.153 | 1.069 | 1.080 | 1.551 | 1.544 |
| 1:729 | 0.571 | 0.566 | 0.499 | 0.528 | 0.469 | 0.464 | 0.717 | 0.705 |
| 0 | 0.056 | 0.059 | 0.055 | 0.056 | 0.056 | 0.056 | 0.056 | 0.093 |
| Second antibody | Goat anti-human IgG (H+L), HRP (1:5000) | | | | | | | |
| EC₅₀ (nM) | 0.035 | | 0.040 | | 0.042 | | 0.028 | |

**Table 3. Binding activity of 13E5 H4L2, 13E5 H4L4 and dupilumab to antigen IL4RA-mFc**

| Antibody dilution (µg/mL) | Antigen: IL4RA-mFc | | | | | |
|---|---|---|---|---|---|---|
| | 13E5 H4L2 | | 13E5 H4L4 | | Dupilumab | |
| 0.333 | 2.733 | 2.772 | 2.798 | 2.879 | 2.850 | 2.893 |
| 1:3 | 2.730 | 2.775 | 2.766 | 2.730 | 2.676 | 2.673 |
| 1:9 | 2.359 | 2.406 | 2.370 | 2.392 | 2.482 | 2.408 |
| 1:27 | 1.492 | 1.523 | 1.474 | 1.608 | 1.637 | 1.722 |
| 1:81 | 0.673 | 0.707 | 0.709 | 0.778 | 0.861 | 0.816 |
| 1:243 | 0.305 | 0.293 | 0.317 | 0.313 | 0.335 | 0.336 |
| 1:729 | 0.145 | 0.134 | 0.146 | 0.147 | 0.147 | 0.147 |
| 0 | 0.063 | 0.063 | 0.064 | 0.064 | 0.067 | 0.064 |
| Second antibody | Goat anti-human IgG (H+L), HRP (1:5000) | | | | | |
| EC₅₀ (nM) | 0.079 | | 0.079 | | 0.066 | |

1.2 The binding activity of antibodies 13E5, 13E5 H1L1, 13E5 H2L2, 13E5 H3L3, 13E5 H4L2 and 13E5 H4L4 for blocking the binding between IL4-N-his and the antigen IL4RA-hFc was measured by competitive ELISA.

The EC₅₀ (median effect concentration) of 13E5, 13E5 H1L1, 13E5 H2L2, 13E5 H3L3, 13E5 H4L2 and 13E5 H4L4 for competing with ligand human IL4-N-His for binding to the antigen human IL4RA-hFc was assayed by ELISA, so as to investigate the activity of 13E5, 13E5 H1L1, 13E5 H2L2, 13E5 H3L3, 13E5 H4L2 and 13E5 H4L4 for blocking the binding of the ligand to the target antigen human IL4RA-hFc.

The ELISA plates were coated with human IL4RA-hFc and blocked before the antibodies were added. An equal volume of human IL4-N-His (synthesized by Akeso, Inc.) was added, and the mixture was well mixed and incubated. After the plates were washed, mouse anti-His, HRP (purchased from CoWin Biosciences, Cat No. CW0285A) was added for incubation. The plates were subjected to another wash. Chromogenic reaction was started by adding TMB (Neogen, 308177) and was then terminated. At the end of the reaction, the OD value at 450 nm wavelength was measured by a plate reader, and was analyzed and processed by SoftMax Pro 6.2.1. The results are shown in Tables 4, 5 and 6.

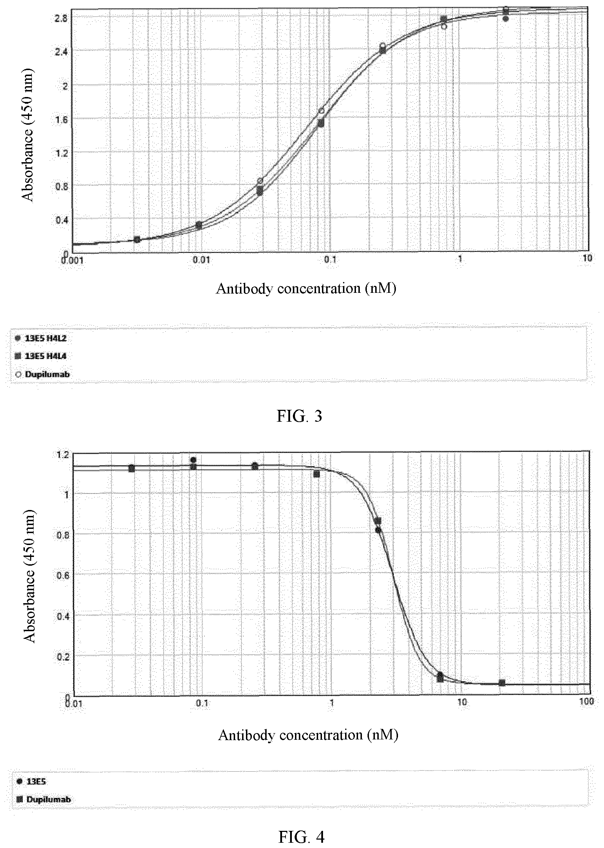

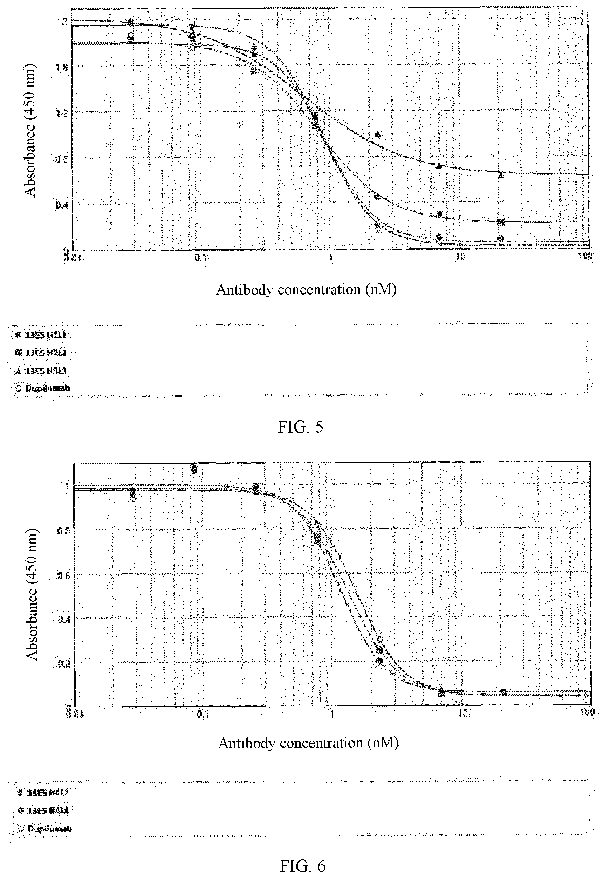

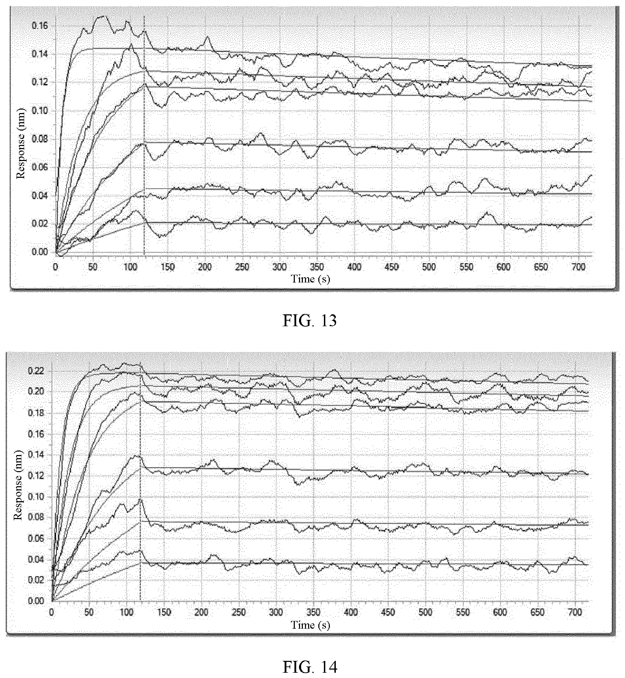

By 4-parameter logistic regression of absorbance vs. antibody concentration, the blocking EC₅₀ of the antibodies was calculated. As shown in FIGs. 4, 5 and 6, 13E5, 13E5 H1L1, 13E5 H2L2, 13E5 H4L2 and 13E5 H4L4 are all capable of effectively blocking the binding of the ligand human IL4-N-His to the antigen human IL4RA-hFc in a dose-dependent manner. Antibodies 13E5 H1L1, 13E5 H2L2, 13E5 H4L2 and 13E5 H4L4 demonstrate superior activities for competing with human IL4-N-His for binding to human IL4RA-hFc as compared to that of the reference antibody dupilumab for the same antigen.

**Table 4. Activity assay of 13E5 and dupilumab for competing with human IL4-N-His for binding to human IL4RA-hFc**

| Antibody dilution (µg/mL) | Antigen: IL4RA-hFc | | | |
|---|---|---|---|---|
| | 13E5 | | Dupilumab | |
| 3 | 0.053 | 0.053 | 0.054 | 0.052 |
| 1:3 | 0.093 | 0.100 | 0.074 | 0.076 |
| 1:9 | 0.790 | 0.832 | 0.837 | 0.877 |
| 1:27 | 1.080 | 1.095 | 1.056 | 1.123 |
| 1:81 | 1.108 | 1.164 | 1.100 | 1.149 |
| 1:243 | 1.120 | 1.199 | 1.126 | 1.126 |
| 1:729 | 1.125 | 1.129 | 1.076 | 1.158 |
| 0 | 1.136 | 1.156 | 1.123 | 1.093 |
| IL4-N-his | | | | |
| Second antibody | Mouse anti-His, HRP (1:4000) | | | |
| EC₅₀ (nM) | 3.000 | | 3.033 | |

**Table 5. Activity assay of 13E5 H1L1, 13E5 H2L2, 13E5 H3L3 and dupilumab for competing with human IL4-N-His for binding to human IL4RA-hFc**

| Antibody dilution (µg/mL) | Antigen: IL4RA-hFc | | | | | | | | |
|---|---|---|---|---|---|---|---|---|---|
| | 13E5 H1L1 | | | 13E5 H2L2 | | 13E5 H3L3 | | Dupilumab | |
| 3 | 0.070 | | 0.078 | 0.230 | 0.216 | 0.645 | 0.620 | 0.050 | 0.049 |
| 1:3 | 0.095 | | 0.103 | 0.282 | 0.306 | 0.727 | 0.708 | 0.059 | 0.057 |
| 1:9 | 0.179 | | 0.225 | 0.443 | 0.455 | 1.047 | 0.955 | 0.186 | 0.159 |
| 1:27 | 0.921 | | 1.415 | 1.048 | 1.096 | 1.450 | 0.845 | 1.230 | 1.053 |
| 1:81 | 1.468 | | 2.037 | 1.562 | 1.543 | 1.705 | 1.686 | 1.680 | 1.546 |
| 1:243 | 1.705 | | 2.157 | 1.776 | 1.888 | 1.932 | 1.846 | 1.774 | 1.743 |
| 1:729 | 1.746 | | 2.182 | 1.792 | 1.845 | 2.060 | 1.933 | 1.889 | 1.841 |
| 0 | 1.793 | | 2.243 | 1.741 | 1.776 | 2.056 | 1.912 | 1.828 | 1.806 |
| IL4-N-his | | | | | | | | | |
| Second antibody | | Mouse anti-His, HRP (1:4000) | | | | | | | |
| EC₅₀ (nM) | | 0.875 | | 0.819 | | 0.636 | | 0.943 | |

**Table 6. Activity assay of 13E5 H4L2, 13E5 H4L4 and dupilumab for competing with human IL4-N-His for binding to human IL4RA-hFc**

| Antibody dilution (µg/mL) | Antigen: IL4RA-hFc | | | | | |
|---|---|---|---|---|---|---|
| | 13E5 H4L2 | | 13E5 H4L4 | | Dupilumab | |
| 3 | 0.056 | 0.059 | 0.054 | 0.051 | 0.054 | 0.057 |
| 1:3 | 0.068 | 0.065 | 0.053 | 0.052 | 0.052 | 0.053 |
| 1:9 | 0.202 | 0.197 | 0.228 | 0.274 | 0.292 | 0.308 |
| 1:27 | 0.697 | 0.780 | 0.752 | 0.786 | 0.771 | 0.871 |
| 1:81 | 0.996 | 0.991 | 0.903 | 1.030 | 0.971 | 0.966 |
| 1:243 | 1.082 | 1.047 | 1.030 | 1.135 | 1.050 | 1.073 |
| 1:729 | 0.964 | 0.988 | 0.950 | 0.978 | 0.918 | 0.964 |
| 0 | 0.935 | 0.960 | 0.932 | 0.898 | 0.896 | 0.956 |
| IL4-N-his | | | | | | |
| Second antibody | Mouse anti-His, HRP (1:4000) | | | | | |
| EC₅₀ (nM) | 1.150 | | 1.328 | | 1.546 | |

### 2. Binding activity of murine antibody 18H7 and humanized antibodies 18H7 H1L1, 18H7 H2L2, 18H7 H2L3, 18H7 H3L2 and 18H7 H3L3 to antigens IL-4RA-hFc

### 2.1 The binding activity of the antibodies 18H7, 18H7 H1L1, 18H7 H2L2, 18H7 H2L3, 18H7 H3L2 and 18H7 H3L3 to the antigens human IL4RA-hFc or human IL4RA-mFc was measured by indirect ELISA.

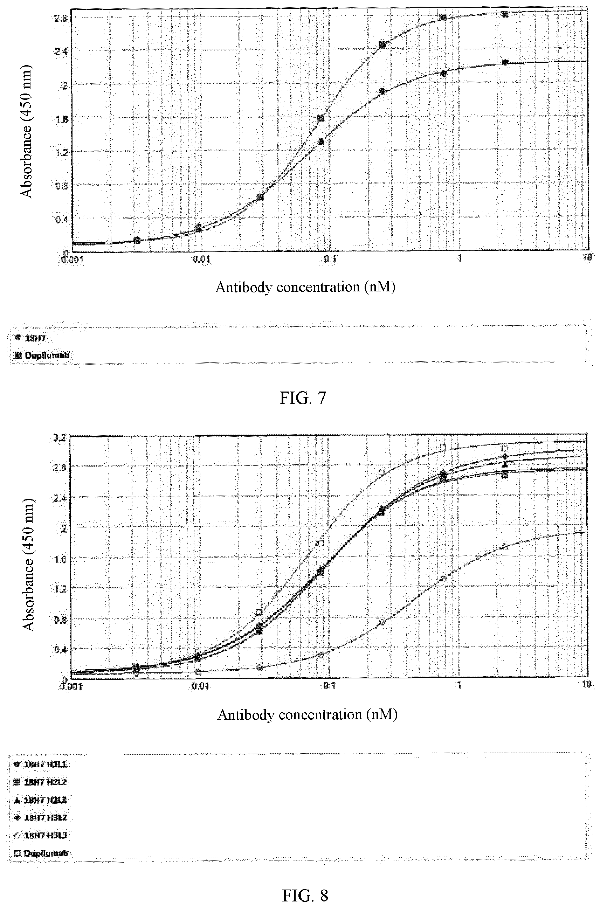

Procedures: The ELISA plates were coated with human IL4RA-hFc or human IL4RA-mFc and were incubated, and target antibodies were added after blocking. Goat anti-mouse IgG (H+L) and HRP (purchased from Jackson ImmunoResearch Inc., Cat No. 109-035-062), or goat anti-human IgG and HRP (purchased from Jackson ImmunoResearch Inc., Cat No. 109-035-088) were added. The plates were incubated and washed before TMB (Neogen, 308177) was added for chromogenic reaction. At the end of the reaction, the absorbance at 450 nm was measured by a plate reader. The data were analyzed by SoftMax Pro 6.2.1. The readings at 450 nm show that antibodies 18H7, 18H7 H1L1, 18H7 H2L2, 18H7 H2L3, 18H7 H3L2 and 18H7 H3L3 are all capable of effectively binding to antigens human IL4RA-hFc or human IL4RA-mFc in a dose-dependent manner. By 4-parameter logistic regression of absorbance vs. antibody concentration, as shown in Tables 7 and 8, 18H7, 18H7 H1L1, 18H7 H2L2, 18H7 H2L3, 18H7 H3L2 and 18H7 H3L3 are all capable of binding to antigens human IL4RA-hFc or human IL4RA-mFc effectively, and antibodies 18H7 H1L1, 18H7 H2L2 and 18H7 H2L3 demonstrate comparable binding activities to that of the reference antibody dupilumab against the same target (FIGs. 7 and 8).

**Table 7. Binding activity of 18H7 and dupilumab to antigens human IL4RA-hFc or human IL-4 RA-mFc**

| Antibody dilution (µg/mL) | Antigen (1 µg/mL) | | | |
|---|---|---|---|---|
| | IL-4RA-hFc | | IL-4RA-mFc | |
| | 18H7 | | Dupilumab | |
| 0.333 | 2.239 | 2.219 | 2.758 | 2.858 |
| 1:3 | 2.065 | 2.128 | 2.748 | 2.801 |
| 1:9 | 1.913 | 1.867 | 2.388 | 2.500 |
| 1:27 | 1.278 | 1.333 | 1.469 | 1.679 |
| 1:81 | 0.640 | 0.650 | 0.599 | 0.673 |
| 1:243 | 0.276 | 0.302 | 0.250 | 0.281 |
| 1:729 | 0.132 | 0.137 | 0.126 | 0.137 |
| 0 | 0.051 | 0.053 | 0.076 | 0.069 |
| Second antibody | Goat anti-mouse IgG (H+L), HRP (1:5000) | | Goat anti-human IgG (H+L), HRP (1:5000) | |
| EC₅₀ (nM) | 0.068 | | 0.077 | |

**Table 8: Binding activity of 18H7 H1L1, 18H7 H2L2, 18H7 H2L3, 18H7 H3L2, 18H7 H3L3 and dupilumab to antigen IL4RA-mFc**

| Antibody dilution (µg/mL) | Antigen: IL-4RA-mFc | | | | | | | | | | | |
|---|---|---|---|---|---|---|---|---|---|---|---|---|
| | 18H7 H1L1 | | 18H7 H2L2 | | 18H7 H2L3 | | 18H7 H3L2 | | 18H7 H3L3 | | Dupilumab | |
| 0.333 | 2.683 | 2.666 | 2.580 | 2.720 | 2.750 | 2.848 | 2.862 | 2.943 | 1.729 | 1.704 | 2.972 | 3.042 |
| 1:3 | 2.630 | 2.616 | 2.611 | 2.595 | 2.726 | 2.675 | 2.696 | 2.673 | 1.314 | 1.279 | 2.989 | 3.057 |
| 1:9 | 2.226 | 2.101 | 2.153 | 2.174 | 2.150 | 2.185 | 2.197 | 2.235 | 0.719 | 0.744 | 2.654 | 2.740 |
| 1:27 | 1.441 | 1.373 | 1.401 | 1.382 | 1.439 | 1.437 | 1.429 | 1.410 | 0.291 | 0.308 | 1.743 | 1.815 |
| 1:81 | 0.682 | 0.575 | 0.626 | 0.613 | 0.677 | 0.679 | 0.685 | 0.708 | 0.139 | 0.134 | 0.847 | 0.896 |
| 1:243 | 0.282 | 0.223 | 0.257 | 0.253 | 0.288 | 0.292 | 0.295 | 0.300 | 0.085 | 0.086 | 0.329 | 0.358 |
| 1:729 | 0.133 | 0.115 | 0.127 | 0.124 | 0.138 | 0.143 | 0.146 | 0.150 | 0.078 | 0.068 | 0.147 | 0.165 |
| 0 | 0.059 | 0.062 | 0.060 | 0.062 | 0.059 | 0.063 | 0.062 | 0.059 | 0.065 | 0.063 | 0.068 | 0.063 |
| Second antibody | Goat anti-human IgG (H+L), HRP (1:5000) | | | | | | | | | | | |
| EC₅₀ (nM) | 0.087 | | 0.087 | | 0.094 | | 0.100 | | 0.446 | | 0.067 | |

### 2.2 Activity of antibodies 18H7, 18H7 H1L1, 18H7 H2L2, 18H7 H2L3 and 18H7 H3L2 for blocking the binding between IL4-N-his and the antigen IL4RA-hFc by competitive ELISA The EC₅₀ (median effect concentration) of 18H7, 18H7 H1L1, 18H7 H2L2, 18H7 H2L3 and 18H7 H3L2 for competing with ligand human IL4-N-His for binding to the antigen human IL4RA-hFc was assayed by ELISA, so as to investigate the activity of 18H7, 18H7 H1L1, 18H7 H2L2, 18H7 H2L3 and 18H7 H3L2 for blocking the binding of the ligand to the target antigen human IL4RA-hFc.

The ELISA plates were coated with human IL4RA-hFc and blocked before the antibodies were added. An equal volume of human IL4-N-His (synthesized by Akeso, Inc., an IL4 connected with 6 His tags at the N terminus) was added, and the mixture was well mixed and incubated. After the plates were washed, mouse anti-His, HRP (purchased from CoWin Biosciences, Cat No. CW0285M) was added for incubation. The plates were subjected to another wash. Chromogenic reaction was started by adding TMB (Neogen, 308177) and was then terminated. At the end of the reaction, the OD value at 450 nm wavelength was measured by a plate reader, and was analyzed and processed by SoftMax Pro 6.2.1. The results are shown in Tables 9 and 10.

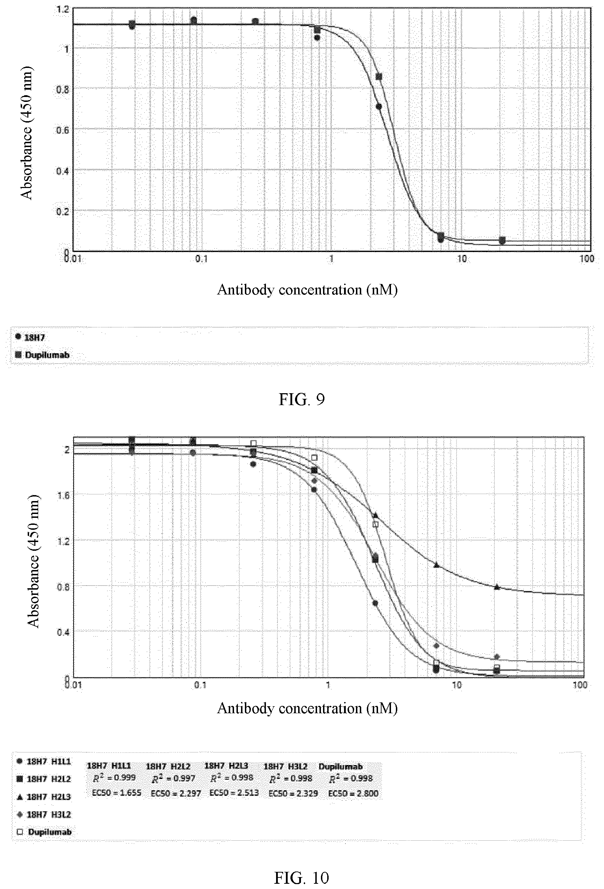

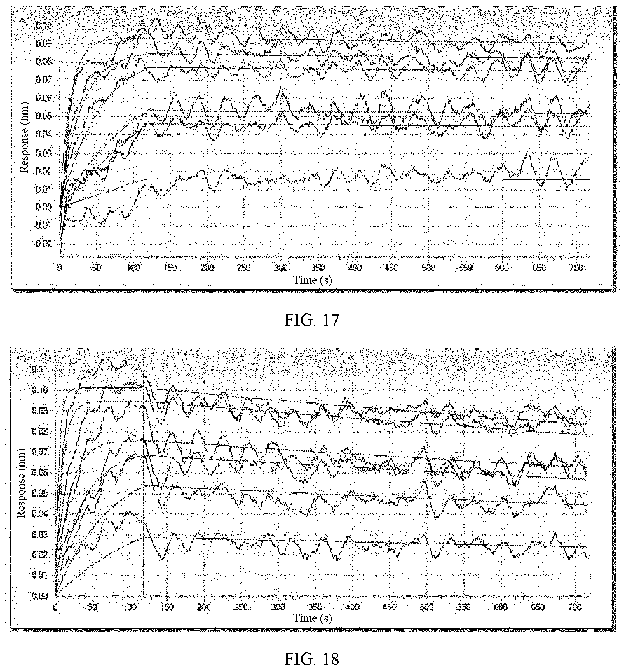

By 4-parameter logistic regression of absorbance vs. antibody concentration, the blocking EC₅₀ of the antibodies was calculated. As shown in FIGs. 9 and 10, 18H7, 18H7 H1L1, 18H7 H2L2 and 18H7 H3L2 are all capable of effectively blocking the binding of the ligand human IL4-N-His to the antigen human IL4RA-hFc in a dose-dependent manner. Antibodies 18H7 H1L1, 18H7 H2L2 and 18H7 H3L2 demonstrate superior activities for competing with human IL4-N-His for binding to human IL4RA-hFc as compared to that of the reference antibody dupilumab for the same antigen.

**Table 9. Activity assay of 18H7 and dupilumab in competing with human IL4-N-His for binding to human IL4RA-hFc**

| Antibody dilution (µg/mL) | Antigen: IL4RA-hFc (2 µg/mL) | | | |
|---|---|---|---|---|
| | 18H7 | | Dupilumab | |
| 3 | 0.046 | 0.045 | 0.054 | 0.052 |
| 1:3 | 0.051 | 0.053 | 0.074 | 0.076 |
| 1:9 | 0.704 | 0.722 | 0.837 | 0.877 |
| 1:27 | 1.047 | 1.055 | 1.056 | 1.123 |
| 1:81 | 1.117 | 1.144 | 1.100 | 1.149 |
| 1:243 | 1.113 | 1.162 | 1.126 | 1.126 |
| 1:729 | 1.097 | 1.115 | 1.076 | 1.158 |
| 0 | 1.157 | 1.127 | 1.123 | 1.093 |

| IL-4-N-his:0.15µg/ml | | | | |
|---|---|---|---|---|
| Second antibody | Mouse anti-His, HRP (1:4000) | | | |
| EC₅₀ (nM) | 2.714 | | 3.033 | |

**Table 10. Activity assay of 18H7 H1L1, 18H7 H2L2, 18H7 H2L3, 18H7 H3L2 and dupilumab in competing with human IL4-N-His for binding to human IL4RA-hFc**

| Antibody dilution (µg/mL) | Antigen: IL4RA-hFc (2 µg/mL) | | | | | | | | | |
|---|---|---|---|---|---|---|---|---|---|---|
| | 18H7 H1L1 | | 18H7 H2L2 | | 18H7 H2L3 | | 18H7 H3L2 | | Dupilumab | |
| 3 | 0.05 | 0.05 | 0.05 | 0.06 | 0.78 | 0.80 | 0.18 | 0.18 | 0.08 | 0.07 |
| | 3 | 1 | 7 | 1 | 1 | 6 | 3 | 4 | 6 | 9 |
| 1:3 | 0.05 | 0.05 | 0.07 | 0.08 | 0.95 | 1.01 | 0.28 | 0.27 | 0.12 | 0.12 |
| | 1 | 4 | 4 | 3 | 6 | 1 | 0 | 9 | 7 | 6 |
| 1:9 | 0.56 | 0.73 | 0.98 | 1.07 | 1.43 | 1.38 | 1.03 | 1.10 | 1.32 | 1.34 |
| | 4 | 5 | 5 | 2 | 9 | 8 | 6 | 0 | 6 | 1 |
| 1:27 | 1.59 | 1.67 | 1.83 | 1.78 | 1.84 | 1.77 | 1.76 | 1.67 | 1.88 | 1.94 |
| | 9 | 1 | 5 | 5 | 0 | 5 | 1 | 3 | 7 | 7 |
| 1:81 | 1.83 | 1.88 | 1.93 | 2.00 | 1.97 | 1.92 | 1.94 | 1.96 | 2.02 | 2.05 |
| | 9 | 4 | 9 | 9 | 1 | 6 | 7 | 4 | 9 | 9 |
| 1:243 | 1.93 | 1.99 | 2.03 | 2.05 | 2.08 | 2.05 | 1.96 | 1.94 | 2.07 | 2.04 |
| | 8 | 3 | 7 | 5 | 6 | 7 | 0 | 6 | 9 | 9 |
| 1:729 | 1.97 | 1.99 | 2.10 | 2.04 | 2.04 | 2.00 | 1.97 | 1.94 | 2.03 | 2.02 |
| | 9 | 5 | 1 | 3 | 8 | 2 | 5 | 8 | 9 | 9 |
| 0 | 1.91 | 1.99 | 2.06 | 2.02 | 2.09 | 1.97 | 1.92 | 1.93 | 2.03 | 1.97 |
| | 9 | 1 | 3 | 4 | 6 | 3 | 8 | 8 | 4 | 5 |

| IL-4-N-his:0.15µg/ml | | | | | | | | | | |
|---|---|---|---|---|---|---|---|---|---|---|
| Second antibody | Mouse anti-His, HRP (1:4000) | | | | | | | | | |
| EC₅₀ (nM) | 1.655 | | 2.297 | | 2.513 | | 2.329 | | 2.800 | |

### 3. Binding activity of humanized antibodies 20G10 H3L3 and 13E5 H4L4 to antigen IL-4RA-hFc

### 3.1 The activity of antibodies 20G10 H3L3 and 13E5 H4L4 for blocking the binding of IL4-N-His to the antigen IL4RA-hFc was measured by competitive ELISA.

The EC₅₀ (median effect concentration) of 20G10 H3L3 and 13E5 H4L4 for competing with ligand human IL4-N-His for binding to the antigen human IL4RA-hFc was assayed by ELISA, so as to investigate the activity of 20G10 H3L3 and 13E5 H4L4 for blocking the binding of the ligand to the target antigen human IL4RA-hFc.

The ELISA plates were coated with human IL4RA-hFc and blocked before the antibodies were added. An equal volume of human IL4-N-His (synthesized by Akeso, Inc.) was added, and the mixture was well mixed and incubated. After the plates were washed, mouse anti-His, HRP (purchased from CoWin Biosciences, Cat No. CW0285A) was added for incubation. The plates were subjected to another wash. Chromogenic reaction was started by adding TMB (Neogen, 308177) and was then terminated. At the end of the reaction, the OD value at 450 nm wavelength was measured by a plate reader, and was analyzed and processed by SoftMax Pro 6.2.1. The results are shown in Table 11.

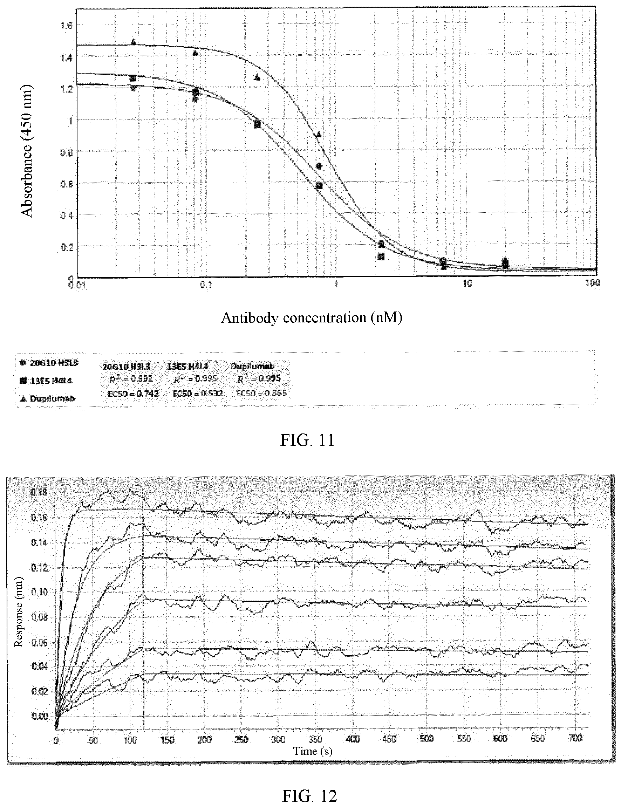

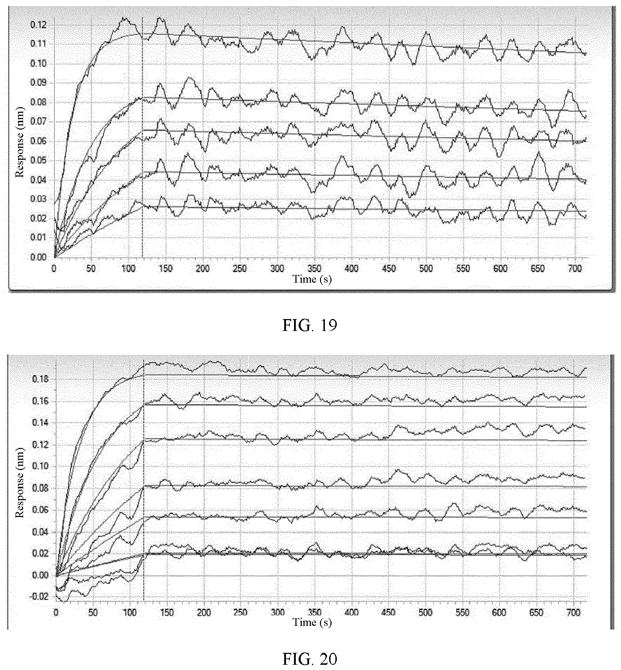

By 4-parameter logistic regression of absorbance vs. antibody concentration, the blocking EC₅₀ of the antibodies was calculated. As shown in FIG. 11, 20G10 H3L3 and 13E5 H4L4 are all capable of effectively blocking the binding of the ligand human IL4-N-His to the antigen human IL4RA-hFc in a dose-dependent manner. The antibodies 20G10 H3L3 and 13E5 H4L4 demonstrate superior activities for competing with human IL4-N-His for binding to human IL4RA-hFc as compared to that of the reference antibody dupilumab for the same antigen.

**Table 11. Activity assay of 20G10 H3L3, 13E5 H4L4 and dupilumab in competing with human IL4-N-His for binding to human IL4RA-hFc**

| Antibody dilution (µg/mL) | Antigen: IL4RA-hFc (2 µg/mL) | | | | | |
|---|---|---|---|---|---|---|
| | 20G10 H3L3 | | 13E5 H4L4 | | Dupilumab | |
| 3 | 0.093 | 0.082 | 0.065 | 0.074 | 0.059 | 0.059 |
| 1:3 | 0.099 | 0.094 | 0.097 | 0.071 | 0.059 | 0.060 |
| 1:9 | 0.199 | 0.212 | 0.123 | 0.119 | 0.201 | 0.197 |
| 1:27 | 0.701 | 0.693 | 0.554 | 0.594 | 0.853 | 0.948 |
| 1:81 | 0.948 | 1.001 | 0.944 | 0.976 | 1.241 | 1.287 |
| 1:243 | 1.118 | 1.131 | 1.137 | 1.200 | 1.443 | 1.400 |
| 1:729 | 1.210 | 1.180 | 1.252 | 1.269 | 1.534 | 1.441 |
| 0 | 1.295 | 1.274 | 1.328 | 1.349 | 1.559 | 1.482 |

| IL4-N-his:0.15µg/ml | | | | | | |
|---|---|---|---|---|---|---|
| Second antibody | Mouse anti-His, HRP (1:4000) | | | | | |
| EC50(nM) | 0.742 | | 0.532 | | 0.865 | |

### Example 8. Affinity constant assay of humanized antibodies 13E5 H1L1, 13E5 H2L2, 13E5 H3L3, 13E5 H4L2, 13E5 H4L4, 18H7 H1L1, 18H7 H2L2 and 18H7 H3L2 for human IL-4RA

By Fortebio molecular interaction instrument, the affinity constant of humanized antibodies 13E5H 1L1, 13E5H 2L2, 13E5 H3L3, 13E5H 4L2, 13E5H 4L4, 18H7 H1L1, 18H7 H2L2, 18H7 H3L2 and dupilumab for binding human IL-4RA were measured. 5 µg/mL antigen was immobilized on AMC sensor by 60 s of incubation. The sensor was then equilibrated in PBST for 300 s and incubated for 120 s in the antibodies at 0.39-25 nM (two-fold serial dilution) for binding the antibodies to the antigen immobilized on the sensor. The remaining antibodies were dissociated with the antigen by a 600-second incubation in PBST. The sensor was refreshed in 10 mM glycine, pH 1.7. The data were analyzed by 1:1 model fitting to obtain affinity constants.

The affinity constants of humanized antibodies 13E5 H4L4, 18H7 H1L1 and dupilumab (reference) for human IL4RA are shown in Table 12 and FIGs. 12-14.

**Table 12. Affinity constant assay of 13E5 H4L4, 18H7 H1L1 and dupilumab for human IL4RA**

| Name | K_{D} (M) | kon(1/Ms) | kon Error | kdis(1/s) | kdis Error | Rmax(nm) |
|---|---|---|---|---|---|---|
| 13E5 H4L4 | 2.63E-11 | 5.56E+06 | 1.60E+05 | 1.46E-04 | 9.74E-06 | 0.1370-0.1667 |
| 18H7 H1L1 | 3.09E-11 | 4.97E+06 | 1. 94E+05 | 1.54E-04 | 1.33E-05 | 0.1026-0.1445 |
| Dupilumab | 1.14E-11 | 7.08E+06 | 2.15E+05 | 8.10E-05 | 1.13E-05 | 0.1325-0.2184 |

| | | | | | | |
|---|---|---|---|---|---|---|
| K_{D} is the affinity constant; K_{D} = kdis/kon. | | | | | | |

The results show that the humanized antibodies 13E5 H4L4, 18H7 H1L1 and human IL4RA have high binding capacities to the target antigen, which are comparable to that of the reference antibody dupilumab.

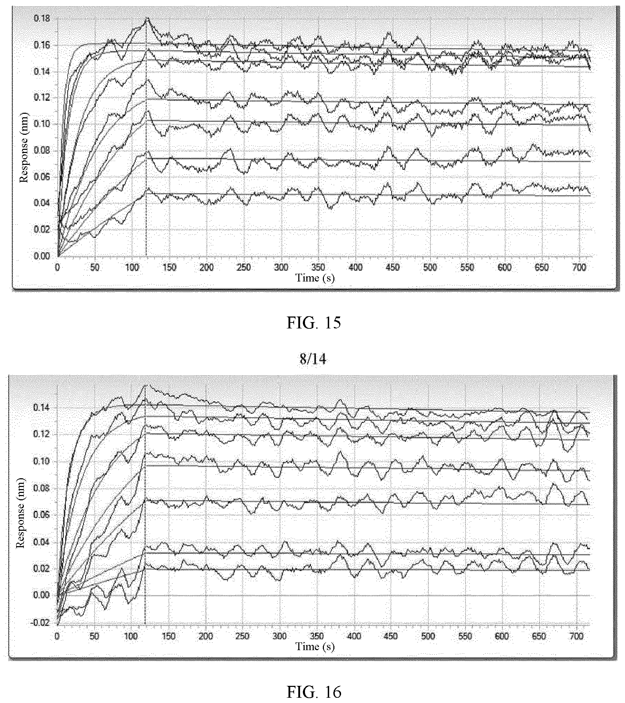

The affinity constants of humanized antibodies 13E5 H1L1, 13E5 H2L2, 13E5 H3L3, 13E5 H4L2, 18H7 H2L2, 18H7 H3L2 and dupilumab (reference) for human IL-4RA are shown in Table 13 and FIGs. 15-21.

**Table 13. Affinity constant assay of 13E5 H1L1, 13E5 H2L2, 13E5 H3L3, 13E5 H4L2, 18H7 H2L2, 18H7 H3L2 and dupilumab for human IL4RA**

| Antibody | K_{D} (M) | kon(1/Ms) | kon Error | kdis(1/s) | kdis Error | Rmax(nm) |
|---|---|---|---|---|---|---|
| 13E5 H1L1 | 1.00E-11 | 5.78E+06 | 1.94E+05 | 5.79E-05 | 1.12E-05 | 0.1347-0.2008 |
| 13E5 H2L2 | 2.83E-11 | 2.44E+06 | 9.77E+04 | 6.90E-05 | 1.44E-05 | 0.1377-0.1944 |
| 13E5 H3L3 | 1.82E-11 | 2.79E+06 | 1.46E+05 | 5.09E-05 | 1.90E-05 | 0.0824-0.1332 |
| 13E5 H4L2 | 1.79E-11 | 1.78E+07 | 1.16E+06 | 3.18E-04 | 2.15E-05 | 0.0521-0.1012 |
| 18H7 H2L2 | 2.39E-11 | 6.47E+06 | 2.63E+05 | 1.54E-04 | 1.60E-05 | 0.0913-0.117 |
| 18H7 H3L2 | 1.71E-11 | 1.27E+06 | 5.38E+04 | 2.16E-05 | 1.44E-05 | 0.1731-0.3601 |
| Dupilumab | 1.17E-11 | 2.35E+06 | 1.68E+05 | 2.75E-05 | 1.73E-05 | 0.2131-0.3131 |

| | | | | | | |
|---|---|---|---|---|---|---|
| K_{D} is the affinity constant; K_{D} = kdis/kon. | | | | | | |

The result shows that the affinity of the humanized antibody 13E5 H1L1 for the antigen is superior to that of the reference antibody dupilumab; the dissociation rate constants kdis of 13E5 H1L1, 13E5 H2L2, 18H7 H2L2 and 18H7 H3L2 with human IL-4RA were less than that of the reference antibody dupilumab, suggesting more stable binding of 13E5 H2L2, 18H7 H2L2 and 18H7 H3L2 to human IL-4RA. The association rate constants of 13E5 H1L1 and 13E5 H4L2 with the antigen human IL-4RA are greater than that of the reference antibody dupilumab, suggesting faster binding of 13E5 H1L1 and 13E5 H4L2 to the antigen human IL-4RA than dupilumab.

### Example 9. Cellular bioactivity assay

Cellular bioactivity of 13E5 H1L1, 13E5 H2L2, 13E5 H4L2, 13E5 H4L4, 18H7 H1L1 and 18H7 H2L2 for inhibiting human IL-4/IL-13-induced TF-1 cell proliferation was analyzed. The procedures are as follows:
TF-1 cells (purchased from American Type Culture Collection, Cat. No. CRL-2003) were cultivated (complete medium: RPMI 1640 + 10% FBS +
2.5 g/L glucose + 2 ng/mL GM-CSF). On the day of assay, TF-1 cells were separated by centrifugation, resuspended in a medium free of GM-CSF, and counted. The cells were inoculated into 96-well plates at 20,000 cells/well. The treatment was given as designed: the final concentrations of the antibodies were 0.05, 0.5 and 5 nM; three concentrations 0.041, 0.41 and 4.1 nM were set for IL-4, and the antibody group employed 0.41 nM IL-4; three concentrations 1.58, 15.8 and 79 nM were set for IL-13, and the antibody panel employed 15.8 nM IL-13. After administration, the 96-well plates were incubated in a 5% carbon dioxide incubator at 37 °C for 72 h. After the 72 hours, CCK8 reagent was added according to the instruction of CCK8 kit (purchased from Dojindo Laboratories, Japan, Cat No. CK04). The mixture was mixed well, incubated at 37 °C for 3-4 hours in a 5% carbon dioxide incubator, and the OD value at
450 nm was read. A curve of OD vs. cell number was plotted by seeding serially diluted TF-1 cells into 96-well plates, adding CCK8 reagent, incubating the plates in a 5% carbon dioxide incubator at 37 °C for 3-4 h and reading the OD value at 450 nm. The cell numbers of the groups were calculated according to the OD value, and GraphPad Prism 5 were used for plotting.

The isotype reference antibody was human anti-hen egg lysozyme IgG (anti-HEL) derived from the variable region of the Fab F10.6.6 sequence in Affinity Maturation Increases the Stability and Plasticity of the Fv Domain of Anti-Protein Antibodies (Acierno et al., J Mol Biol., 2007, 374(1): 130-46). The isotype was synthesized by Akeso, Inc.

Nanjing Genscript Biology was entrusted for codon optimization and gene synthesis for the heavy and light chain (complete sequence or variable region) genes of the human IgG antibodies. With reference to the Molecular Cloning: A Laboratory Manual (Second Edition), the heavy and light chain genes were subcloned into the heavy and antibody light chain expression vectors (both were pcDNA3.1 vector) for mammalian expression system by standard molecular cloning techniques such as PCR, enzymatic digestion, DNA gel electrophoresis, ligation and transformation, colony PCR or digestion and identification. The heavy and light chain genes of the recombinant expression vector were further sequenced and analyzed (the heavy and light chain sequences are set forth in SEQ ID NOs: 122 and 123). After the sequence was verified to be correct, endotoxin-free expression plasmids were prepared in a large scale, and the heavy and light chain plasmids were transiently co-transfected into HEK293 cells for expression of recombinant antibody. After 7 days of culture, the cell culture medium was collected and affinity purified using a Protein A column (GE), and the quality of the resulting antibody sample was determined using SDS-PAGE and SEC-HPLC standard analysis techniques.

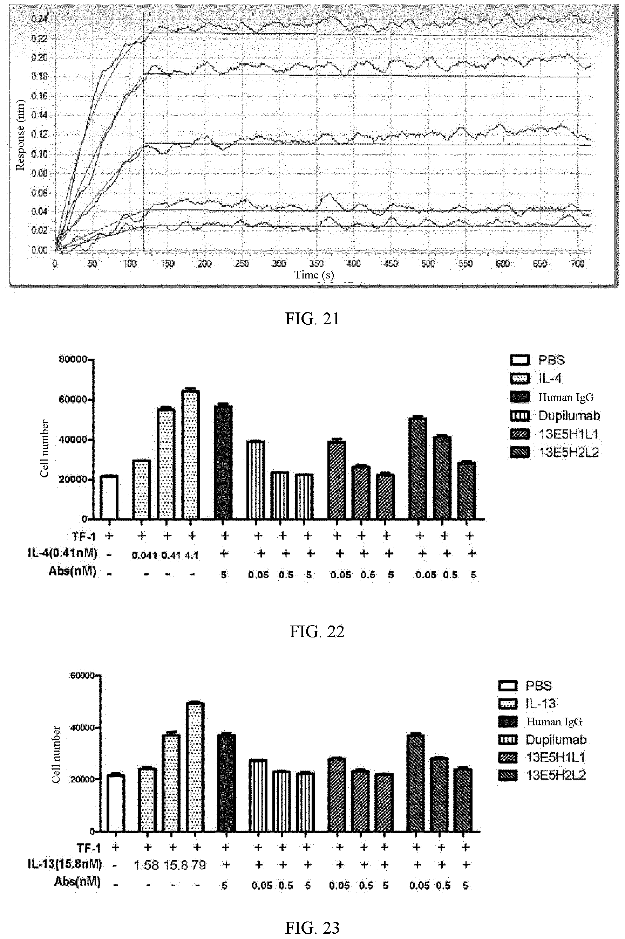

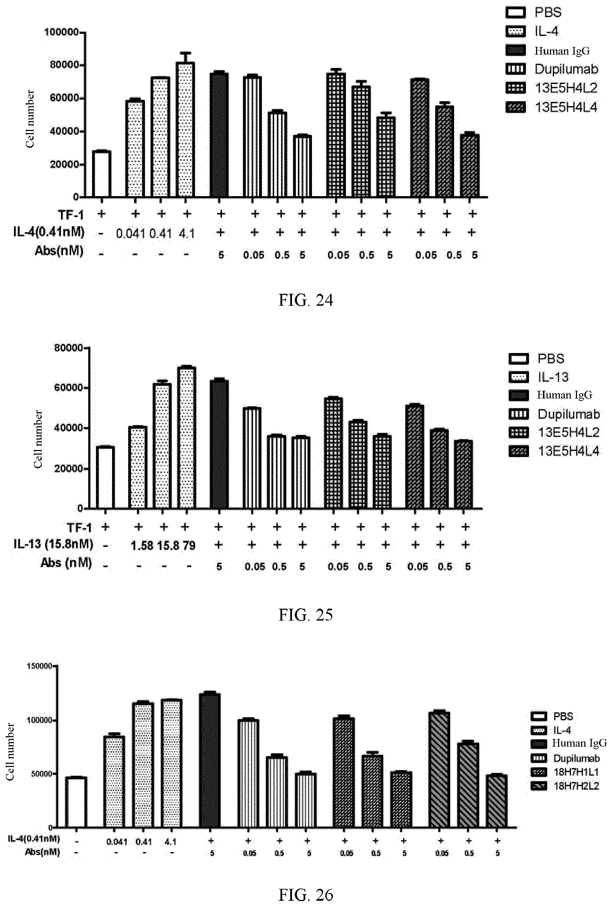

The results are shown in FIGs. 22-27. As can be seen in FIGs. 22-27, both human IL-4 and human IL-13 are able to effectively promote the proliferation of TF-1 cells in a dose-dependent manner; as compared to isotype reference antibodies (human IgG), dupilumab, 13E5 H1L1, 13E5 H2L2, 13E5 H4L2, 13E5 H4L4, 18H7 H1L1 and 18H7 H2L2 can specifically inhibit TF-1 cell proliferation induced by IL-4 and IL-13 in a dose-dependent manner.

The results show that 13E5 H1L1, 13E5 H2L2, 13E5 H4L2, 13E5 H4L4, 18H7 H1L1 and 18H7 H2L2 can specifically inhibit the proliferation of TF-1 cells induced by human IL-4 and human IL-13; antibodies 13E5 H1L1, 13E5 H4L4 and 18H7 H1L1 demonstrate comparable activities to reference antibody dupilumab.

### Example 10. 13E5H4L4 and 18H7H1L1 inhibiting up-regulation of CD23 expression in PBMCs

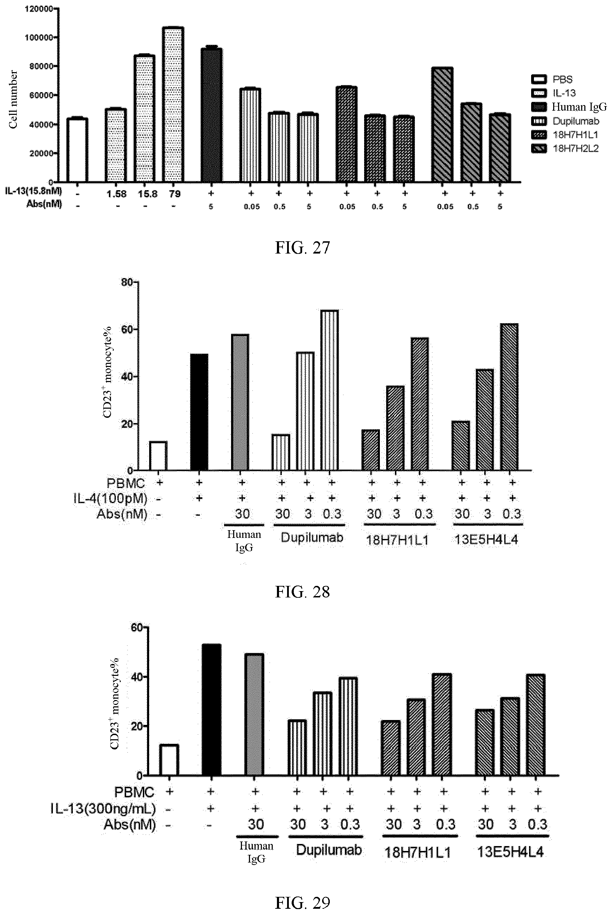

This example was intended to examine the bioactivity of 13E5 H4L4 and 18H7 H1L1 for neutralizing the CD23 expression up-regulation on human PBMC surface induced by human IL-4 and human IL-13 through flow cytometry. The procedures are as follows:
Normal human peripheral blood (with heparin) was separated by Ficoll density gradient centrifugation to give human fresh PBMCs. After 3 centrifugations and washes, the cells were counted and the density was adjusted to 2.5 × 10⁶ cells/mL. The cells were seeded into low attachment 96-well plates at 200 µL of PBMCs per well (i.e., 500,000 cells/well); 25 µL of the antibodies dupilumab, 13E5 H4L4 or 18H7 H1L1 (at final concentrations of 30, 3 and 0.3 nM, respectively) were added to each well, and blank reference and isotype reference human IgG (at a final concentration of 30 nM) were set. The cells were incubated at room temperature for 30 min. After 30 min, 25 µL of human IL-4 (final concentration of 100 pM) or 25 µL of human IL-13 (final concentration of 300 ng/mL) was added and the system was incubated for
2.5 days. After the 2.5 days, the PBMCs were separated and transferred into 1.5-mL EP tubes, added with 500 µL of 1% PBSA and centrifuged at 1000× g for 5 min. The supernatant was discarded, and the residues were added with 50 µL of CD23-PE antibody (50-fold diluted with 1% PBSA) and incubated on ice for 40 min. After the 40-min incubation, the system was added with 1 mL of 1% PBSA and centrifuged at 1000× g for 5 min before the supernatant was discarded. The cells were resuspended in 200 µL of 1% PBSA, and transferred into a flow cytometry tube for assay. The results are shown in FIGs. 28 and 29.

The results show that human IL-4 and human IL-13 can up-regulate the expression level of CD23 on the surface of human PBMCs, and 13E5 H4L4 and 18H7 H1L1 can specifically bind to human IL-4RA, thereby effectively blocking the up-regulation of the CD23 expression level by IL-4 and IL-13.

### Example 11. Effect of 13E5 H4L4 on B-hIL4Ra mouse model with HDM-induced skin inflammation

The procedures are as follows:
A B-hIL4Ra mouse (C57BL/6 background) skin inflammation model was established by inducing with HDM (human dust mite allergen, Greer Laborct, Lot No. 348717). The mice were divided into 6 groups with 7 mice in each, including isotype reference group (anti-HEL), dupilumab high-dose group
(80 mg/kg; commercially available dupilumab from Sanofi and Regeneron Pharmaceuticals) and low-dose group (20 mg/kg; commercially available as Dupilumab from Sanofi and Regeneron Pharmaceuticals), 13E5 H4L4 high-dose group (80 mg/kg) and low-dose group (20 mg/kg). The treatment was given by subcutaneous injection on D-1, D2, D5 and D8. In the normal group, the animal model was established by daily intradermal injection of normal saline. In the isotype reference group, the animal model was established with daily intradermal injection of HDM (37.5 µg × 2/25 µL/day) for 7 days and daily intradermal injection of HDM (25 µg × 2/25 µL/day) for 3 days. In the antibody treatment groups, the animal model was established with daily intradermal injection of HDM (37.5 µg × 2/25 µL/day) for 7 days and daily intradermal injection of HDM (25 µg × 2/25 µL/day) for 3 days.

Epidermal thickness of the mice was measured to investigate the effect of 13E5 H4L4 in inhibiting the epidermal thickness in B-hIL4Ra mouse skin inflammation model.

**Table 14. Dosage and regimen**

| **Group** | **n** | **Animal model** | **Treatment** |
|---|---|---|---|
| Normal group | 7 | Normal saline, 25 µL/day on D0-D9, intradermal injection, daily | Normal saline, subcutaneous injection, D-1, D2, D5 and D8 |
| hIgG4 (i.e., anti-HEL) | 7 | HDM, 37.5 µg × 2/25 µL/day, D0-D6; 25 µg × 2/25 µL/day, D7-D9, daily intradermal injection | Anti-HEL, 80 mg/kg, subcutaneous injection, D-1, D2, D5 and D8 |
| Dupilumab 80 mg/kg | 7 | | Dupilumab, 80 mg/kg, subcutaneous injection, D-1, D2, D5 and D8 |
| Dupilumab, 20 mg/kg | 7 | | Dupilumab, 80 mg/kg, subcutaneous injection, D-1, D2, D5 and D8 |
| 13E5 H4L4, 80 mg/kg | 7 | | 13E5 H4L4, 80 mg/kg, subcutaneous injection, D-1, D2, D5 and D8 |
| 13E5 H4L4, 20 mg/kg | 7 | | 13E5 H4L4, 20 mg/kg, subcutaneous injection, D-1, D2, D5 and D8 |

| | | | |
|---|---|---|---|
| Note: D0 is the first day of model establishment. The normal saline and HDM were administered at the upper right back and lower right back respectively. The results are shown in FIG. 30. As compared with the isotype reference antibody hIgG4, 13E5 H4L4 of different doses can effectively inhibit the thickening of epidermis, and the blood concentration of 13E5 H4L4 is higher than that of dupilumab at 80 mg/kg, which corresponds to the efficacy. | | | |

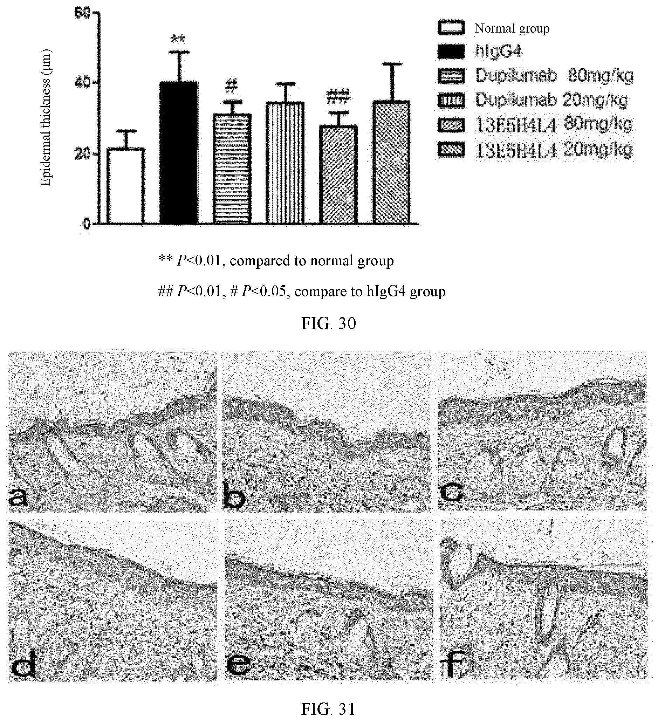

The pathological sections are shown in FIG. 31: in the B-hIL4Ra mouse skin inflammation model established by 37.5 µg + 25 µg HDM intradermal injection, the thickness of epidermis in the model group is significantly thickened as compared with that of the normal group, the thickening of epidermis in mice can be significantly inhibited by dupilumab 80 mg/kg and 13E5 H4L4 80 mg/kg subcutaneous injections with good dose-response relationship. 13E5 H4L4 80 mg/kg is slightly superior to dupilumab 80 mg/kg.

The preferred embodiments of the present invention have been described above in detail, but the present invention is not limited to the embodiments. Those skilled in the art can make various equivalent modifications or replacements without violating the spirit of the present invention. These equivalent modifications or replacements are included in the scope defined by the claims of the present application.

### SEQUENCE LISTING

13E5 heavy chain variable region 
GAGGTGCAGCTGCAGCAGAGCGGACCAGAGCTGGTGAAGCCTGGCGCCTCTGT
GAAGATCAGCTGTAAGACCTCCGGCTACACCTTCACAGAGTATACAATCCACTG
GGTGAAGCAGAACCACGGCAAGAGCCTGGAGTGGATCGGCGGCATCAATCCTA
ACAATGGCGGCACCGTGTACAACCAGAACTTCAAGGGCAAGGCCACCCTGACA
GTGGACAAGAGCTCCTCTACCGCCTATATGGAGCTGAGGTCTCTGACAAGCGAG
GACTCCGCCGTGTACTATTGCGCCAGAGTGCGGAGAGGCATGGATTACTGGGGC
CAGGGCACCTCCGTGACAGTGAGCTCC (SEQ ID NO: 1)
 
EVQLQQSGPELVKPGASVKISCKTSGYTFTEYTIHWVKQNHGKSLEWIGGINPNNG
GTVYNQNFKGKATLTVDKSSSTAYMELRSLTSEDSAVYYCARVRRGMDYWGQGT
SVTVSS (SEQ ID NO: 2)
13E5 light chain variable region 
GACATCGTGATGACCCAGTCCCACAAGTTTATGTCCACATCTGTGGGCGACAGG
GTGTCCATCACCTGTAAGGCCTCTCAGGATGTGACCACAGCCGTGGCCTGGTAC
CAGCAGAAGCCAGGCCAGTCTCCCAAGCTGCTGATCTATAGCGCCTCCTACCGG
TATACAGGCGTGCCCGACAGATTCACCGGCTCTGGCAGCGGCACAGATTTCACC
TTTACAATCAGCTCCGTGCAGGCAGAGGACCTGGCCGTGTACTATTGCCAGCAG
CACTACTCTGCCCCTTGGACCTTCGGCGGAGGAACAAACCTGGAGATCAAG
(SEQ ID NO: 3)
 
DIVMTQSHKFMSTSVGDRVSITCKASQDVTTAVAWYQQKPGQSPKLLIYSASYRYT
GVPDRFTGSGSGTDFTFTISSVQAEDLAVYYCQQHYSAPWTFGGGTNLEIK (SEQ ID
NO: 4)
18H7 heavy chain variable region 
GATGTGAAGCTGGTGGAGTCCGGCGGCGACCTGGTGAACCTGGGCGGAAGCCT
GAAGCTGAGCTGTGCCGCCAGCGGCTTCACATTTCCTCCAGCTACATGAGCTG
GGTGAGGCAGACACCCGAGAGGAGACTGGAGCTGGTGGCCGCCATCAACAGCA
ATGGCGGCAAGACATACTACCCTGATACCGTGAAGGGCAGATTCACCATCAGC
AGGGACAACGCCAAGAACACCCTGTACCTGCAGATGTCCGGCCTGAAGACAGA
GGACACAGCCCTGTACTACTGCACCAGGCAGAGGGGCAACTACGTGGGCGCCA
TGGACTACTGGGGCCAGGGCACAAGCGTGACCGTGAGCTCC (SEQ ID NO: 5)
 
DVKLVESGGDLVNLGGSLKLSCAASGFTFSSSYMSWVRQTPERRLELVAAINSNGG
KTYYPDTVKGRFTISRDNAKNTLYLQMSGLKTEDTALYYCTRQRGNYVGAMDYW
GQGTSVTVSS (SEQ ID NO: 6)
18H7 light chain variable region 
GACATCGTGATGACACAGTCCCACAAGTTTATGTCCACATCCGTGGGCGATAGG
GTGTCCATCACATGCAAGGCCAGCCAGGACGTGAGCACAGCCGTGGTGTGGTA
CCAGCAGAAGCCTGGCCAGAGCCCCACCCTGCTGATCTACTCCGCCAGCTACAG
GTACACAGGCGTGCCTGACAGATTCACAGGCTCCGGCAGCGGCACCGATTTCAC
CTTTACCATCTCCTCCGTGCAGGCCGAGGATCTGGCCGTGTACTACTGCCACCA
 
GTACTACGGCAGCCCCCCACATTCGGCGGCGGAACAAAGCTGGAGATCAAG
(SEQ ID NO: 7)
 
DIVMTQSHKFMSTSVGDRVSITCKASQDVSTAVVWYQQKPGQSPTLLIYSASYRYT
GVPDRFTGSGSGTDFTFTISSVQAEDLAVYYCHQYYGSPPTFGGGTKLEIK (SEQ ID
NO: 8)
**13E5 CDR**
   HCDR1: GYTFTEYT (SEQ ID NO: 9)
   HCDR2: INPNNGGT (SEQ ID NO: 10)
   HCDR3: ARVRRGMDY (SEQ ID NO: 11)
   LCDR1: QDVTTA (SEQ ID NO: 12)
   LCDR2: SAS (SEQ ID NO: 13)
   LCDR3: QQHYSAPWT (SEQ ID NO: 14)
**18H7 CDR**
   HCDR1: GFTFSSSY (SEQ ID NO: 15)
   HCDR2: INSNGGKT (SEQ ID NO: 16)
   HCDR3: TRQRGNYVGAMDY (SEQ ID NO: 17)
   LCDR1: QDVSTA (SEQ ID NO: 18)
   LCDR2: SAS (SEQ ID NO: 19)
   LCDR3: HQYYGSPPT (SEQ ID NO: 20)
**13E5 H1 heavy chain variable region** 
CAGGTGCAGCTGCAGCAGTCCGGAGCAGAGGTGGTGAAGCCAGGAGCCAGCGT
GAAGATCTCCTGTAAGACCTCTGGCTACACCTTCACAGAGTATACAATCCACTG
GGTGAAGCAGGCACACGGACAGAGCCTGGAGTGGATCGGCGGCATCAACCCTA
ACAATGGCGGCACCGTGTACAATCAGAAGTTTCAGGGCAAGGCCACCCTGACA
GTGGACAAGTCTACCAGCACAGCCTATATGGAGCTGAGGTCCCTGACCTCTGAG
GACACAGCCGTGTACTATTGCGCCCGGGTGCGGAGAGGCATGGATTACTGGGG
CCAGGGCACCTCCGTGACAGTGAGCTCC (SEQ ID NO: 21)
 
QVQLQQSGAEVVKPGASVKISCKTSGYTFTEYTIHWVKQAHGQSLEWIGGINPNNG
GTVYNQKFQGKATLTVDKSTSTAYMELRSLTSEDTAVYYCARVRRGMDYWGQGT
SVTVSS (SEQ ID NO: 22)
**13E5 L1 light chain variable region** 
GACATCCAGATGACCCAGTCTCCTAAGTCTCTGAGCACATCCGTGGGCGACCGG
GTGACCATCACATGTAGAGCCAGCCAGGATGTGACCACAGCAGTGGCATGGTA
CCAGCAGAAGCCTGGCAAGTCCCCTAAGCTGCTGATCTATTCTGCCAGCTACAG
GTATACCGGAGTGCCATCTCGGTTCTCCGGCTCTGGCAGCGGCACAGACTTCAC
CTTTACAATCAGCTCCGTGCAGCCAGAGGATCTGGCCACCTACTATTGCCAGCA
 
GCACTACAGCGCCCCATGGACCTTTGGCGGAGGAACAAACCTGGAGATCAAG
(SEQ ID NO: 23)
 
DIQMTQSPKSLSTSVGDRVTITCRASQDVTTAVAWYQQKPGKSPKLLIYSASYRYT
GVPSRFSGSGSGTDFTFTISSVQPEDLATYYCQQHYSAPWTFGGGTNLEIK (SEQ ID
NO: 24)
**13E5 H2 heavy chain variable region** 
CAGGTGCAGCTGGTGCAGTCCGGAGCAGAGGTGGTGAAGCCAGGAGCCAGCGT
GAAGGTGTCCTGTAAGACCTCTGGCTACACCTTCACAGAGTATACAATCCACTG
GGTGCGGCAGGCACCAGGACAGTCTCTGGAGTGGATCGGCGGCATCAACCCTA
ACAATGGCGGCACCAACTACAATCAGAAGTTTCAGGGCAAGGTGACCCTGACA
GTGGACAAGTCCACCTCTACAGCCTATATGGAGCTGAGCTCCCTGAGGTCTGAG
GACACAGCCGTGTACTATTGCGCCCGCGTGCGGAGAGGCATGGATTACTGGGGC
CAGGGCACCAGCGTGACAGTGTCTAGC (SEQ ID NO: 25)
 
QVQLVQSGAEVVKPGASVKVSCKTSGYTFTEYTIHWVRQAPGQSLEWIGGINPNNG
GTNYNQKFQGKVTLTVDKSTSTAYMELSSLRSEDTAVYYCARVRRGMDYWGQGT
SVTVSS (SEQ ID NO: 26)
**13E5 L2 light chain variable region** 
GACATCCAGATGACCCAGAGCCCTAGCTCCCTGAGCGCCTCCGTGGGCGACAGG
GTGACCATCACATGTAGAGCCTCCCAGGATGTGACCACAGCAGTGGCATGGTAC
CAGCAGAAGCCAGGCAAGGCCCCTAAGCTGCTGATCTACTCCGCCTCTAGCAGG
TATACCGGAGTGCCATCTCGGTTCTCTGGCAGCGGCTCCGGCACAGACTTTACC
CTGACAATCTCCTCTGTGCAGCCAGAGGATCTGGCCACATACTATTGCCAGCAG
CACTATTCTGCCCCCTGGACCTTTGGCGGCGGCACAAACCTGGAGATCAAG
(SEQ ID NO: 27)
 
DIQMTQSPSSLSASVGDRVTITCRASQDVTTAVAWYQQKPGKAPKLLIYSASSRYT
GVPSRFSGSGSGTDFTLTISSVQPEDLATYYCQQHYSAPWTFGGGTNLEIK (SEQ ID
NO: 28)
**13E5 H3 heavy chain variable region** 
CAGGTGCAGCTGGTGCAGTCCGGAGCAGAGGTGGTGAAGCCAGGAGCCAGCGT
GAAGGTGTCCTGTAAGGCCTCTGGCTACACCTTCACAGAGTATACCATCCACTG
GGTGCGGCAGGCACCAGGACAGGGACTGGAGTGGATCGGCGGCATCAACCCTA
ACAATGGCGGCACAAATTACGCCCAGAAGTTTCAGGGCAGGGTGACCATCACA
GTGGACAAGTCCACCTCTACAGCCTATATGGAGCTGAGCTCCCTGAGGTCTGAG
GACACCGCCGTGTACTATTGCGCCCGCGTGCGGAGAGGCATGGATTACTGGGGC
CAGGGCACCAGCGTGACAGTGTCTAGC (SEQ ID NO: 29)
 
QVQLVQSGAEVVKPGASVKVSCKASGYTFTEYTIHWVRQAPGQGLEWIGGINPNN
GGTNYAQKFQGRVTITVDKSTSTAYMELSSLRSEDTAVYYCARVRRGMDYWGQG
TSVTVSS (SEQ ID NO: 30)
**13E5 L3 light chain variable region** 
GACATCCAGATGACCCAGAGCCCTAGCTCCCTGAGCGCCTCCGTGGGCGACAGG
GTGACCATCACATGTAGAGCCTCCCAGGATGTGACCACAGCCCTGGCATGGTAC
CAGCAGAAGCCAGGCAAGGCCCCTAAGCTGCTGATCTACTCCGCCTCTAGCCTG
TATACCGGAGTGCCATCTCGGTTCTCTGGCAGCGGCTCCGGCACAGACTTTACC
CTGACAATCTCCTCTCTGCAGCCAGAGGATTTCGCCACATACTATTGCCAGCAG
CACTATTCTGCCCCCTGGACCTTTGGCGGCGGCACAAACCTGGAGATCAAG
(SEQ ID NO: 31)
 
DIQMTQSPSSLSASVGDRVTITCRASQDVTTALAWYQQKPGKAPKLLIYSASSLYTG
VPSRFSGSGSGTDFTLTISSLQPEDFATYYCQQHYSAPWTFGGGTNLEIK (SEQ ID
NO: 32)
**13E5 H4 heavy chain variable region** 
CAGGTGCAGCTGGTGCAGTCCGGAGCAGAGGTGGTGAAGCCAGGAGCCAGCGT
GAAGGTGTCCTGTAAGACCTCTGGCTACACCTTCACAGAGTATACAATCCACTG
GGTGCGGCAGGCACCAGGACAGTCTCTGGAGTGGATCGGCGGCATCAACCCTA
ACAATGGCGGCACCgtcTACAATCAGAAGTTTCAGGGCAAGGTGACCCTGACAGT
GGACAAGTCCACCTCTACAGCCTATATGGAGCTGAGCTCCCTGAGGTCTGAGGA
CACAGCCGTGTACTATTGCGCCCGCGTGCGGAGAGGCATGGATTACTGGGGCCA
GGGCACCAGCGTGACAGTGTCTAGC (SEQ ID NO: 33)
 
QVQLVQSGAEVVKPGASVKVSCKTSGYTFTEYTIHWVRQAPGQSLEWIGGINPNNG
GTVYNQKFQGKVTLTVDKSTSTAYMELSSLRSEDTAVYYCARVRRGMDYWGQGT
SVTVSS (SEQ ID NO: 34)
**13E5 L4 light chain variable region** 
GACATCCAGATGACCCAGAGCCCTAGCTCCCTGAGCGCCTCCGTGGGCGACAGG
GTGACCATCACATGTAGAGCCTCCCAGGATGTGACCACAGCAGTGGCATGGTAC
CAGCAGAAGCCAGGCAAGGCCCCTAAGCTGCTGATCTACTCCGCCTCTTAcAGG
TATACCGGAGTGCCATCTCGGTTCTCTGGCAGCGGCTCCGGCACAGACTTTACC
CTGACAATCTCCTCTGTGCAGCCAGAGGATCTGGCCACATACTATTGCCAGCAG
CACTATTCTGCCCCCTGGACCTTTGGCGGCGGCACAAACCTGGAGATCAAG
(SEQ ID NO: 35)
 
DIQMTQSPSSLSASVGDRVTITCRASQDVTTAVAWYQQKPGKAPKLLIYSASYRYT
GVPSRFSGSGSGTDFTLTISSVQPEDLATYYCQQHYSAPWTFGGGTNLEIK (SEQ ID
NO: 36)
**18H7 H1 heavy chain variable region** 
GACGTGCAGCTGGTGGAGAGCGGCGGCCTGGTGCAGCTGGGCGGCTCCCT
GAGGCTGTCTTGTGCAGCCAGCGGCTTCACCTTTAGCTCCTCTTACATGTCTTGG
GTGCGGCAGACACCCGAGAAGAGACTGGAGCTGGTGGCCGCCATCAACTCTAA
TGGCGGCAAGACCTACTATGCCGACAGCGTGAAGGGCAGGTTTACCATCTCCCG
CGATAACTCTAAGAATACACTGTATCTGCAGATGAGCTCCCTGAGAGCCGAGGA
CACCGCCGTGTACTATTGCACACGGCAGAGAGGCAACTACGTGGGCGCCATGG
ATTATTGGGGCCAGGGCACCAGCGTGACAGTGTCTAGC (SEQ ID NO: 37)
 
DVQLVESGGGLVQLGGSLRLSCAASGFTFSSSYMSWVRQTPEKRLELVAAINSNGG
KTYYADSVKGRFTISRDNSKNTLYLQMSSLRAEDTAVYYCTRQRGNYVGAMDYW
GQGTSVTVSS (SEQ ID NO: 38)
**18H7 L1 light chain variable region** 
GACATCCAGATGACCCAGTCTCCCAGCTCCCTGTCCACATCTGTGGGCGACCGG
GTGACCATCACCTGTAAGGCCTCTCAGGATGTGAGCACCGCCGTGGTGTGGTAC
CAGCAGAAGCCAGGCAAGAGCCCCAAGCTGCTGATCTATAGCGCCTCCTACCG
GTATACCGGCGTGCCTTCCAGATTCTCTGGCAGCGGCTCCGGCACAGACTTCAC
CTTTACAATCTCTAGCGTGCAGCCAGAGGATATCGCCACATACTATTGCCACCA
GTACTATGGCAGCCCTCCCACCTTCGGCGGCGGCACAAAGCTGGAGATCAAG
(SEQ ID NO: 39)
 
DIQMTQSPSSLSTSVGDRVTITCKASQDVSTAVVWYQQKPGKSPKLLIYSASYRYTG
VPSRFSGSGSGTDFTFTISSVQPEDIATYYCHQYYGSPPTFGGGTKLEIK (SEQ ID NO:
40)
**18H7 H2 heavy chain variable region** 
GAGGTGCAGCTGGTGGAGAGCGGCGGCCTGGTGCAGCCCGGCGGCTCCCT
GAGGCTGTCTTGTGCCGCCAGCGGCTTCACCTTTAGCTCCTCTTACATGTCTTGG
GTGCGGCAGGCACCTGGCAAGAGACTGGAGCTGGTGGCCGCCATCAACTCTAA
TGGCGGCAAGACATACTATGCCGACAGCGTGAAGGGCAGGTTTACCATCTCCCG
CGATAACTCTAAGAATACACTGTATCTGCAGATGAGCTCCCTGAGAGCCGAGGA
CACCGCCGTGTACTATTGCACACGGCAGAGAGGCAACTACGTGGGCGCCATGG
ATTATTGGGGCCAGGGCACCAGCGTGACAGTGTCTAGC (SEQ ID NO: 41)
 
EVQLVESGGGLVQPGGSLRLSCAASGFTFSSSYMSWVRQAPGKRLELVAAINSNGG
KTYYADSVKGRFTISRDNSKNTLYLQMSSLRAEDTAVYYCTRQRGNYVGAMDYW
GQGTSVTVSS (SEQ ID NO: 42)
**18H7 L2 light chain variable region** 
GACATCCAGATGACCCAGTCTCCAAGCTCCCTGTCCGCCTCTGTGGGCGACAGG
GTGACCATCACATGTCAGGCCTCTCAGGATGTGAGCACAGCCGTGGTGTGGTAC
CAGCAGAAGCCAGGCAAGAGCCCCAAGCTGCTGATCTATAGCGCCTCCTACCTG
TATACCGGCGTGCCTTCCAGATTCTCTGGCAGCGGCTCCGGCACAGACTTCACC
TTTACAATCTCTAGCCTGCAGCCAGAGGATATCGCCACCTACTATTGCCACCAG
TACTATGGCAGCCCTCCCACCTTCGGCGGCGGCACAAAGCTGGAGATCAAG
(SEQ ID NO: 43)
 
DIQMTQSPSSLSASVGDRVTITCQASQDVSTAVVWYQQKPGKSPKLLIYSASYLYTG
VPSRFSGSGSGTDFTFTISSLQPEDIATYYCHQYYGSPPTFGGGTKLEIK (SEQ ID NO:
44)
**18H7 H3 heavy chain variable region** 
GAGGTGCAGCTGGTGGAGAGCGGCGGCCTGGTGCAGCCCGGCGGCTCCCT
GCGGCTGTCTTGTGCCGCCAGCGGCTTCACCTTTAGCTCCTCTTACATGTCCTGG
GTGAGGCAGGCACCTGGCAAGGGACTGGAGTACGTGAGCGCCATCAACTCTAA
TGGCGGCAAGACATACTATGCCGACTCTGTGAAGGGCAGGTTTACCATCAGCCG
CGATAACTCCAAGAATACACTGTACCTGCAGATGAGCTCCCTGAGGGCAGAGG
 
ACACCGCCGTGTACTATTGCACACGGCAGAGAGGCAACTACGTGGGCGCCATG
GATTATTGGGGCCAGGGCACCTCCGTGACAGTGTCTAGC (SEQ ID NO: 45)
 
EVQLVESGGGLVQPGGSLRLSCAASGFTFSSSYMSWVRQAPGKGLEYVSAINSNGG
KTYYADSVKGRFTISRDNSKNTLYLQMSSLRAEDTAVYYCTRQRGNYVGAMDYW
GQGTSVTVSS (SEQ ID NO: 46)
**18H7 L3 light chain variable region** 
GACATCCAGATGACCCAGTCTCCAAGCTCCCTGTCCGCCTCTGTGGGCGACAGG
GTGACCATCACATGTCAGGCCTCTCAGGATGTGAGCACAGCCCTGGCCTGGTAC
CAGCAGAAGCCAGGCAAGGCCCCCAAGCTGCTGATCTATAGCGCCTCCAACCTG
GAGACCGGCGTGCCTAGCAGATTCTCTGGCAGCGGCTCCGGCACAGACTTCACC
TTTACAATCTCTAGCCTGCAGCCAGAGGATATCGCCACCTACTATTGCCACCAG
TACTATGGCAGCCCTCCCACCTTCGGCGGCGGCACAAAGCTGGAGATCAAG
(SEQ ID NO: 47)
 
DIQMTQSPSSLSASVGDRVTITCQASQDVSTALAWYQQKPGKAPKLLIYSASNLETG
VPSRFSGSGSGTDFTFTISSLQPEDIATYYCHQYYGSPPTFGGGTKLEIK (SEQ ID NO:
48)
**13E5 heavy chain framework region**
   FR-H1: EVQLQQSGPELVKPGASVKISCKTS (SEQ ID NO: 49)
   FR-H2: IHWVKQNHGKSLEWIGG (SEQ ID NO: 50)
   FR-H3: VYNQNFKGKATLTVDKSSSTAYMELRSLTSEDSAVYYC (SEQ ID NO: 51)
   FR-H4: WGQGTSVTVSS (SEQ ID NO: 52)
**13E5 light chain framework region**
   FR-L1: DIVMTQSHKFMSTSVGDRVSITCKAS (SEQ ID NO: 53)
   FR-L2: VAWYQQKPGQSPKLLIY (SEQ ID NO: 54)
   FR-L3: YRYTGVPDRFTGSGSGTDFTFTISSVQAEDLAVYYC (SEQ ID NO: 55)
   FR-L4: FGGGTNLEIK (SEQ ID NO: 56)
**18H7 heavy chain framework region**
   FR-H1: DVKLVESGGDLVNLGGSLKLSCAAS (SEQ ID NO: 57)
   FR-H2: MSWVRQTPERRLELVAA (SEQ ID NO: 58)
   FR-H3: YYPDTVKGRFTISRDNAKNTLYLQMSGLKTEDTALYYC (SEQ ID NO: 59)
   FR-H4: WGQGTSVTVSS (SEQ ID NO: 60)
**18H7 light chain framework region**
   FR-L1: DIVMTQSHKFMSTSVGDRVSITCKAS (SEQ ID NO: 61)
   FR-L2: VVWYQQKPGQSPTLLIY (SEQ ID NO:62)
   FR-L3: YRYTGVPDRFTGSGSGTDFTFTISSVQAEDLAVYYC (SEQ ID NO: 63)
   FR-L4: FGGGTKLEIK (SEQ ID NO: 64)
**13E5 H1 framework region**
   FR-H1: QVQLQQSGAEVVKPGASVKISCKTS (SEQ ID NO: 65)
   FR-H2: IHWVKQAHGQSLEWIGG (SEQ ID NO: 66)
   FR-H3: VYNQKFQGKATLTVDKSTSTAYMELRSLTSEDTAVYYC (SEQ ID NO: 67)
   FR-H4: WGQGTSVTVSS (SEQ ID NO: 68)
**13E5 L1 framework region**
   FR-L1: DIQMTQSPKSLSTSVGDRVTITCRAS (SEQ ID NO: 69)
   FR-L2: VAWYQQKPGKSPKLLIY (SEQ ID NO: 70)
   FR-L3: YRYTGVPSRFSGSGSGTDFTFTISSVQPEDLATYYC (SEQ ID NO: 71)
   FR-L4: FGGGTNLEIK (SEQ ID NO: 72)
**13E5 H2 framework region**
   FR-H1: QVQLVQSGAEVVKPGASVKVSCKTS (SEQ ID NO: 73)
   FR-H2: IHWVRQAPGQSLEWIGG (SEQ ID NO: 74)
   FR-H3: NYNQKFQGKVTLTVDKSTSTAYMELSSLRSEDTAVYYC (SEQ ID NO: 75)
   FR-H4: WGQGTSVTVSS (SEQ ID NO: 76)
**13E5 L2 framework region**
   FR-L1: DIQMTQSPSSLSASVGDRVTITCRAS (SEQ ID NO: 77)
   FR-L2: VAWYQQKPGKAPKLLIY (SEQ ID NO: 78)
   FR-L3: SRYTGVPSRFSGSGSGTDFTLTISSVQPEDLATYYC (SEQ ID NO: 79)
   FR-L4: FGGGTNLEIK (SEQ ID NO: 80)
**13E5 H3 framework region**
   FR-H1: QVQLVQSGAEVVKPGASVKVSCKAS (SEQ ID NO: 81)
   FR-H2: IHWVRQAPGQGLEWIGG (SEQ ID NO: 82)
   FR-H3: NYAQKFQGRVTITVDKSTSTAYMELSSLRSEDTAVYYC (SEQ ID NO: 83)
   FR-H4: WGQGTSVTVSS (SEQ ID NO: 84)
**13E5 L3 framework region**
   FR-L1: DIQMTQSPSSLSASVGDRVTITCRAS (SEQ ID NO: 85)
   FR-L2: LAWYQQKPGKAPKLLIY (SEQ ID NO: 86)
   FR-L3: SLYTGVPSRFSGSGSGTDFTLTISSLQPEDFATYYC (SEQ ID NO: 87)
   FR-L4: FGGGTNLEIK (SEQ ID NO: 88)
**13E5 H4 framework region**
   FR-H1: QVQLVQSGAEVVKPGASVKVSCKTS (SEQ ID NO: 89)
   FR-H2: IHWVRQAPGQSLEWIGG (SEQ ID NO: 90)
   FR-H3: VYNQKFQGKVTLTVDKSTSTAYMELSSLRSEDTAVYYC (SEQ ID NO: 91)
   FR-H4: WGQGTSVTVSS (SEQ ID NO: 92)
**13E5 L4 framework region**
   FR-L1: DIQMTQSPSSLSASVGDRVTITCRAS (SEQ ID NO: 93)
   FR-L2: VAWYQQKPGKAPKLLIY (SEQ ID NO: 94)
   FR-L3: YRYTGVPSRFSGSGSGTDFTLTISSVQPEDLATYYC (SEQ ID NO: 95)
   FR-L4: FGGGTNLEIK (SEQ ID NO: 96)
**18H7 H1 framework region**
   FR-H1: DVQLVESGGGLVQLGGSLRLSCAAS (SEQ ID NO: 97)
   FR-H2: MSWVRQTPEKRLELVAA (SEQ ID NO: 98)
   FR-H3: YYADSVKGRFTISRDNSKNTLYLQMSSLRAEDTAVYYC (SEQ ID NO: 99)
   FR-H4: WGQGTSVTVSS (SEQ ID NO: 100)
**18H7 L1 framework region**
   FR-L1: DIQMTQSPSSLSTSVGDRVTITCKAS (SEQ ID NO: 101)
   FR-L2: VVWYQQKPGKSPKLLIY (SEQ ID NO: 102)
   FR-L3: YRYTGVPSRFSGSGSGTDFTFTISSVQPEDIATYYC (SEQ ID NO: 103)
   FR-L4: FGGGTKLEIK (SEQ ID NO: 104)
**18H7 H2 framework region**
   FR-H1: EVQLVESGGGLVQPGGSLRLSCAAS (SEQ ID NO: 105)
   FR-H2: MSWVRQAPGKRLELVAA (SEQ ID NO: 106)
   FR-H3: YYADSVKGRFTISRDNSKNTLYLQMSSLRAEDTAVYYC (SEQ ID NO: 107)
   FR-H4: WGQGTSVTVSS (SEQ ID NO: 108)
**18H7 L2 framework region**
   FR-L1: DIQMTQSPSSLSASVGDRVTITCQAS (SEQ ID NO: 109)
   FR-L2: VVWYQQKPGKSPKLLIY (SEQ ID NO: 110)
   FR-L3: YLYTGVPSRFSGSGSGTDFTFTISSLQPEDIATYYC (SEQ ID NO: 111)
   FR-L4: FGGGTKLEIK (SEQ ID NO: 112)
**18H7 H3 framework region**
   FR-H1: EVQLVESGGGLVQPGGSLRLSCAAS (SEQ ID NO: 113)
   FR-H2: MSWVRQAPGKGLEYVSA (SEQ ID NO: 114)
   FR-H3: YYADSVKGRFTISRDNSKNTLYLQMSSLRAEDTAVYYC (SEQ ID NO: 115)
   FR-H4: WGQGTSVTVSS (SEQ ID NO: 116)
**18H7 L3 framework region**
   FR-L1: DIQMTQSPSSLSASVGDRVTITCQAS (SEQ ID NO: 117)
   FR-L2: LAWYQQKPGKAPKLLIY (SEQ ID NO: 118)
   FR-L3: NLETGVPSRFSGSGSGTDFTFTISSLQPEDIATYYC (SEQ ID NO: 119)
   FR-L4: FGGGTKLEIK (SEQ ID NO: 120)
Sequence of mFc tag: 
PRGPTIKPCPPCKCPAPNLLGGPSVFIFPPKIKDVLMISLSPIVTCVVVDVSEDDPDVQI
SWFVNNVEVHTAQTQTHREDYNSTLRVVSALPIQHQDWMSGKEFKCKVNNKDLP
APIERTISKPKGSVRAPQVYVLPPPEEEMTKKQVTLTCMVTDFMPEDIYVEWTNNG
KTELNYKNTEPVLDSDGSYFMYSKLRVEKKNWVERNSYSCSVVHEGLHNHHTTKS
FSRTPGK (SEQ ID NO: 121)
**hIgG heavy chain sequence** 
EVQLEQSGAELMKPGASVKISCKATGYTFTTYWIEWIKQRPGHSLEWIGEILPGSDS
TYYNEKVKGKVTFTADASSNTAYMQLSSLTSEDSAVYYCARGDGFYVYWGQGTT
LTVSSASTKGPSVFPLAPCSRSTSESTAALGCLVKDYFPEPVTVSWNSGALTSGVHT
FPAVLQSSGLYSLSSVVTVPSSSLGTKTYTCNVDHKPSNTKVDKRVESKYGPPCPPC
PAPEFLGGPSVFLFPPKPKDTLMISRTPEVTCVVVDVSQEDPEVQFNWYVDGVEVH
NAKTKPREEQFNSTYRVVSVLTVLHQDWLNGKEYKCKVSNKGLPSSIEKTISKAKG
QPREPQVYTLPPSQEEMTKNQVSLTCLVKGFYPSDIAVEWESNGQPENNYKTTPPV
LDSDGSFFLYSRLTVDKSRWQEGNVFSCSVMHEALHNHYTQKSLSLSLGK (SEQ ID
NO: 122)
**hIgG light chain sequence** 
DIELTQSPATLSVTPGDSVSLSCRASQSISNNLHWYQQKSHESPRLLIKYTSQSMSGIP
SRFSGSGSGTDFTLSINSVETEDFGVYFCQQSGSWPRTFGGGTKLDIKRTVAAPSVFI
FPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC (SEQ ID NO: 123)
VAB 16F3-1 VH-2 
EVQLVESGGGLVQPGRSLRLSCEASGFTFDDYAMHWVRQAPGKGLEWVSGLSRTS
VSIGYADSVKGRFTISRDNAKNSLYLEMNSLRPEDTALYYCAKWGTRGYFDYWGQ
GTLVTVSS (SEQ ID NO: 124)
**16F3-1 VL** 
DIQMTQSPSSVSASVGDRVTITCRASQDISIWLAWYQQSPGKAPKLLINVASRLQSG
VPSRFSGSGSGTDFTLTINSLQPEDFVTYYCQQANSFPITFGQGTRLATK (SEQ ID
NO: 125)
**20G 10H3 heavy chain variable region** 
CAGGTGACACTGAAGGAGTCCGGCCCAACCCTGGTGAAGCCCACCCAGACACT
GACCCTGACATGTACCTTCAGCGGCTTTTCTCTGAGCACATCCGGCATGGGAGT
 
GGGATGGATCAGACAGCCCCCTGGCAAGGCCCTGGAGTGGCTGGCCCTGATCTG
GTGGGCCGACGATAAGAGGTACTCTACCAGCCTGAAGTCCCGCCTGACAATCTC
CAAGGACACCTCTAAGAACCAGGTGGTGCTGACAATCACCAATGTGGACCCCGT
GGATACAGCCACCTACTATTGCGCCCGGATCACAAGAGGCAACTCTGCCATGGA
TTTTTGGGGCCAGGGCACATCTGTGACCGTGAGCTCC (SEQ ID NO: 126)
 
QVTLKESGPTLVKPTQTLTLTCTFSGFSLSTSGMGVGWIRQPPGKALEWLALIWWA
DDKRYSTSLKSRLTISKDTSKNQVVLTITNVDPVDTATYYCARITRGNSAMDFWGQ
GTSVTVSS (SEQ ID NO: 127)
**20G 10L3 light chain variable region** 
GACATCCAGATGACCCAGTCTCCTAGCTCCCTGTCCGCCTCTGTGGGCGATAGG
GTGACCATCACATGTAGAGCCTCTGAGAACGTGTACAGCTATCTGAATTGGTAC
CAGCAGAAGCCCGGCAAGGCCCCTAAGCTGCTGATCTATAACGCCAAGAGCCT
GCAGTCCGGAGTGCCAAGCCGGTTCAGCGGCTCCGGCTCTGGCACCGACTTTAC
CCTGACAATCTCTAGCCTGCAGCCAGAGGATTTCGCCACATACTATTGCCAGCA
CCACTACGGCATCCCCTGGACCTTCGGCGGCGGCACAAAGCTGGAGATCAAG
(SEQ ID NO:128)
 
DIQMTQSPSSLSASVGDRVTITCRASENVYSYLNWYQQKPGKAPKLLIYNAKSLQS
GVPSRFSGSGSGTDFTLTISSLQPEDFATYYCQHHYGIPWTFGGGTKLEIK (SEQ ID
NO: 129)
**20G10 CDR**
   HCDR1: GFSLSTSGMG (SEQ ID NO: 130)
   HCDR2: IWWADDK (SEQ ID NO: 131)
   HCDR3: ARITRGNSAMDF ( SEQ ID NO: 132)
   LCDR1: ENVYSY (SEQ ID NO: 133)
   LCDR2: NAK (SEQ ID NO: 134)
   LCDR3: QHHYGIPWT (SEQ ID NO: 135)
**20G 10H3 framework region**
   FR-H1: QVTLKESGPTLVKPTQTLTLTCTFS (SEQ ID NO: 136)
   FR-H2: VGWIRQPPGKALEWLAL (SEQ ID NO: 137)
   FR-H3: RYSTSLKSRLTISKDTSKNQVVLTITNVDPVDTATYYC (SEQ ID NO: 138)
   FR-H4: WGQGTSVTVSS (SEQ ID NO: 139)
**20G 10L3 framework region**
   FR-L1: DIQMTQSPSSLSASVGDRVTITCRAS (SEQ ID NO: 140)
   FR-L2: LNWYQQKPGKAPKLLIY (SEQ ID NO: 141)
   FR-L3: SLQSGVPSRFSGSGSGTDFTLTISSLQPEDFATYYC (SEQ ID NO: 142)
   FR-L4: FGGGTKLEIK (SEQ ID NO: 143)

## Claims

1. An antibody or an antigen-binding fragment thereof binding to IL4RA, preferably human IL4RA, wherein: (1) the antibody comprises:
an HCDR1, comprising or consisting of the sequence set forth in SEQ ID NO: 9, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
an HCDR2, comprising or consisting of the sequence set forth in SEQ ID NO: 10, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence, and
an HCDR3, comprising or consisting of the sequence set forth in SEQ ID NO: 11, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
and the antibody further comprises:
an LCDR1, comprising or consisting of the sequence set forth in SEQ ID NO: 12, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
an LCDR2, comprising or consisting of the sequence set forth in SEQ ID NO: 13, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence, and
an LCDR3, comprising or consisting of the sequence set forth in SEQ ID NO: 14, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence;
(2) the antibody comprises:
an HCDR1, comprising or consisting of the sequence set forth in SEQ ID NO: 15, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
an HCDR2, comprising or consisting of the sequence set forth in SEQ ID NO: 16, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence, and
an HCDR3, comprising or consisting of the sequence set forth in SEQ ID NO: 17, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
and the antibody further comprises:
an LCDR1, comprising or consisting of the sequence set forth in SEQ ID NO: 18, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
an LCDR2, comprising or consisting of the sequence set forth in SEQ ID NO: 19, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence, and
an LCDR3, comprising or consisting of the sequence set forth in SEQ ID NO: 20, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence;
or, (3) the antibody comprises:
an HCDR1, comprising or consisting of the sequence set forth in SEQ ID NO: 130, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
an HCDR2, comprising or consisting of the sequence set forth in SEQ ID NO: 131, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence, and
an HCDR3, comprising or consisting of the sequence set forth in SEQ ID NO: 132, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
and the antibody further comprises:
an LCDR1, comprising or consisting of the sequence set forth in SEQ ID NO: 133, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence,
an LCDR2, comprising or consisting of the sequence set forth in SEQ ID NO: 134, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence, and
an LCDR3, comprising or consisting of the sequence set forth in SEQ ID NO: 135, a sequence having at least 90%, preferably at least 91%, 92%, 93%, 94%, 95%, 96%, 97%, 98% or 99% sequence identity to the sequence, or an amino acid sequence having one or more (preferably 2 or 3) conservative amino acid mutations (preferably substitutions, insertions or deletions) compared to the sequence.

2. The antibody according to claim 1, comprising:
(1) (i) a heavy chain variable region comprising or consisting of:
the amino acid sequence set forth in SEQ ID NO: 2,
a sequence having at least 80%, 85% or 90%, preferably at least 91%, 92%, 93%, 94%, 95%, 96%, 97%, 98% or 99% sequence identity to the sequence set forth in SEQ ID NO: 2, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 2, and
(ii) a light chain variable region comprising or consisting of:
the amino acid sequence set forth in SEQ ID NO: 4,
a sequence having at least 80%, 85% or 90%, preferably at least 91%, 92%, 93%, 94%, 95%, 96%, 97%, 98% or 99% sequence identity to the sequence set forth in SEQ ID NO: 4, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 4;
(2) (i) a heavy chain variable region comprising or consisting of:
the amino acid sequence set forth in SEQ ID NO: 6,
a sequence having at least 80%, 85% or 90%, preferably at least 91%, 92%, 93%, 94%, 95%, 96%, 97%, 98% or 99% sequence identity to the sequence set forth in SEQ ID NO: 6, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 6, and
(ii) a light chain variable region comprising or consisting of:
the amino acid sequence set forth in SEQ ID NO: 8,
a sequence having at least 80%, 85% or 90%, preferably at least 91%, 92%, 93%, 94%, 95%, 96%, 97%, 98% or 99% sequence identity to the sequence set forth in SEQ ID NO: 8, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 8;
(3) (i) a heavy chain variable region comprising or consisting of:
the amino acid sequence set forth in SEQ ID NO: 22,
a sequence having at least 80%, 85% or 90%, preferably at least 91%, 92%, 93%, 94%, 95%, 96%, 97%, 98% or 99% sequence identity to the sequence set forth in SEQ ID NO: 22, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 22, and
(ii) a light chain variable region comprising or consisting of:
the amino acid sequence set forth in SEQ ID NO: 24,
a sequence having at least 80%, 85% or 90%, preferably at least 91%, 92%, 93%, 94%, 95%, 96%, 97%, 98% or 99% sequence identity to the sequence set forth in SEQ ID NO: 24, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 24;
(4) (i) a heavy chain variable region comprising or consisting of:
the amino acid sequence set forth in SEQ ID NO: 26,
a sequence having at least 80%, 85% or 90%, preferably at least 91%, 92%, 93%, 94%, 95%, 96%, 97%, 98% or 99% sequence identity to the sequence set forth in SEQ ID NO: 26, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 26, and
(ii) a light chain variable region comprising or consisting of:
the amino acid sequence set forth in SEQ ID NO: 28,
a sequence having at least 80%, 85% or 90%, preferably at least 91%, 92%, 93%, 94%, 95%, 96%, 97%, 98% or 99% sequence identity to the sequence set forth in SEQ ID NO: 28, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 28;
(5) (i) a heavy chain variable region comprising or consisting of:
the amino acid sequence set forth in SEQ ID NO: 30,
a sequence having at least 80%, 85% or 90%, preferably at least 91%, 92%, 93%, 94%, 95%, 96%, 97%, 98% or 99% sequence identity to the sequence set forth in SEQ ID NO: 30, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 30, and
(ii) a light chain variable region comprising or consisting of:
the amino acid sequence set forth in SEQ ID NO: 32,
a sequence having at least 80%, 85% or 90%, preferably at least 91%, 92%, 93%, 94%, 95%, 96%, 97%, 98% or 99% sequence identity to the sequence set forth in SEQ ID NO: 32, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 32;
(6) (i) a heavy chain variable region comprising or consisting of:
the amino acid sequence set forth in SEQ ID NO: 34,
a sequence having at least 80%, 85% or 90%, preferably at least 91%, 92%, 93%, 94%, 95%, 96%, 97%, 98% or 99% sequence identity to the sequence set forth in SEQ ID NO: 34, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 34, and
(ii) a light chain variable region comprising or consisting of:
the amino acid sequence set forth in SEQ ID NO: 36,
a sequence having at least 80%, 85% or 90%, preferably at least 91%, 92%, 93%, 94%, 95%, 96%, 97%, 98% or 99% sequence identity to the sequence set forth in SEQ ID NO: 36, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 36;
(7) (i) a heavy chain variable region comprising or consisting of:
the amino acid sequence set forth in SEQ ID NO: 38,
a sequence having at least 80%, 85% or 90%, preferably at least 91%, 92%, 93%, 94%, 95%, 96%, 97%, 98% or 99% sequence identity to the sequence set forth in SEQ ID NO: 38, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 38, and
(ii) a light chain variable region comprising or consisting of:
the amino acid sequence set forth in SEQ ID NO: 40,
a sequence having at least 80%, 85% or 90%, preferably at least 91%, 92%, 93%, 94%, 95%, 96%, 97%, 98% or 99% sequence identity to the sequence set forth in SEQ ID NO: 40, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 40;
(8) (i) a heavy chain variable region comprising or consisting of:
the amino acid sequence set forth in SEQ ID NO: 42,
a sequence having at least 80%, 85% or 90%, preferably at least 91%, 92%, 93%, 94%, 95%, 96%, 97%, 98% or 99% sequence identity to the sequence set forth in SEQ ID NO: 42, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 42, and
(ii) a light chain variable region comprising or consisting of:
the amino acid sequence set forth in SEQ ID NO: 44,
a sequence having at least 80%, 85% or 90%, preferably at least 91%, 92%, 93%, 94%, 95%, 96%, 97%, 98% or 99% sequence identity to the sequence set forth in SEQ ID NO: 44, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 44;
(9) (i) a heavy chain variable region comprising or consisting of:
the amino acid sequence set forth in SEQ ID NO: 46,
a sequence having at least 80%, 85% or 90%, preferably at least 91%, 92%, 93%, 94%, 95%, 96%, 97%, 98% or 99% sequence identity to the sequence set forth in SEQ ID NO: 46, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 46, and
(ii) a light chain variable region comprising or consisting of:
the amino acid sequence set forth in SEQ ID NO: 48,
a sequence having at least 80%, 85% or 90%, preferably at least 91%, 92%, 93%, 94%, 95%, 96%, 97%, 98% or 99% sequence identity to the sequence set forth in SEQ ID NO: 48, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 48;
(10) (i) a heavy chain variable region comprising or consisting of:
the amino acid sequence set forth in SEQ ID NO: 42,
a sequence having at least 80%, 85% or 90%, preferably at least 91%, 92%, 93%, 94%, 95%, 96%, 97%, 98% or 99% sequence identity to the sequence set forth in SEQ ID NO: 42, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 42, and
(ii) a light chain variable region comprising or consisting of:
the amino acid sequence set forth in SEQ ID NO: 48,
a sequence having at least 80%, 85% or 90%, preferably at least 91%, 92%, 93%, 94%, 95%, 96%, 97%, 98% or 99% sequence identity to the sequence set forth in SEQ ID NO: 48, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 48;
(11) (i) a heavy chain variable region comprising or consisting of:
the amino acid sequence set forth in SEQ ID NO: 46,
a sequence having at least 80%, 85% or 90%, preferably at least 91%, 92%, 93%, 94%, 95%, 96%, 97%, 98% or 99% sequence identity to the sequence set forth in SEQ ID NO: 46, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 46, and
(ii) a light chain variable region comprising or consisting of:
the amino acid sequence set forth in SEQ ID NO: 44,
a sequence having at least 80%, 85% or 90%, preferably at least 91%, 92%, 93%, 94%, 95%, 96%, 97%, 98% or 99% sequence identity to the sequence set forth in SEQ ID NO: 44, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 44;
(12) (i) a heavy chain variable region comprising or consisting of:
the amino acid sequence set forth in SEQ ID NO: 34,
a sequence having at least 80%, 85% or 90%, preferably at least 91%, 92%, 93%, 94%, 95%, 96%, 97%, 98% or 99% sequence identity to the sequence set forth in SEQ ID NO: 34,
or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 34, and
(ii) a light chain variable region comprising or consisting of:
the amino acid sequence set forth in SEQ ID NO: 28,
a sequence having at least 80%, 85% or 90%, preferably at least 91%, 92%, 93%, 94%, 95%, 96%, 97%, 98% or 99% sequence identity to the sequence set forth in SEQ ID NO: 28, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 28; or
(13) (i) a heavy chain variable region comprising or consisting of:
the amino acid sequence set forth in SEQ ID NO: 127,
a sequence having at least 80%, 85% or 90%, preferably at least 91%, 92%, 93%, 94%, 95%, 96%, 97%, 98% or 99% sequence identity to the sequence set forth in SEQ ID NO: 127, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 127, and
(ii) a light chain variable region comprising or consisting of:
the amino acid sequence set forth in SEQ ID NO: 129,
a sequence having at least 80%, 85% or 90%, preferably at least 91%, 92%, 93%, 94%, 95%, 96%, 97%, 98% or 99% sequence identity to the sequence set forth in SEQ ID NO: 129, or
an amino acid sequence having one or more (preferably 1, 2, 3, 4, 5, 6, 7, 8, 9 or 10) conservative amino acid mutations (preferably substitutions, insertions or deletions) compared to the sequence set forth in SEQ ID NO: 129.

3. The antibody or the antigen-binding fragment thereof according to any one of claims 1-2, wherein the antibody further comprises framework regions FR-H1, FR-H2, FR-H3 and FR-H4 in the heavy chain variable region, and the framework regions FR-L1, FR-L2, FR-L3 and FR-L4 in the light chain variable region, wherein
(1) the FR-H1 comprises or consists of the amino acid sequence set forth in SEQ ID NO: 49, a sequence having at least 80%, 85% or 90%, preferably at least 91%, 92%, 93%, 94%, 95%, 96%, 97%, 98% or 99% sequence identity to the sequence set forth in SEQ ID NO: 49, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 49,
the FR-H2 comprises or consists of the amino acid sequence set forth in SEQ ID NO: 50, a sequence having at least 80%, 85% or 90%, preferably at least 91%, 92%, 93%, 94%, 95%, 96%, 97%, 98% or 99% sequence identity to the sequence set forth in SEQ ID NO: 50, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 50,
the FR-H3 comprises or consists of the amino acid sequence set forth in SEQ ID NO: 51, a sequence having at least 80%, 85% or 90%, preferably at least 91%, 92%, 93%, 94%, 95%, 96%, 97%, 98% or 99% sequence identity to the sequence set forth in SEQ ID NO: 51, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 51,
the FR-H4 comprises or consists of the amino acid sequence set forth in SEQ ID NO: 52, a sequence having at least 80%, 85% or 90%, preferably at least 91%, 92%, 93%, 94%, 95%, 96%, 97%, 98% or 99% sequence identity to the sequence set forth in SEQ ID NO: 52, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 52,
the FR-L1 comprises or consists of the amino acid sequence set forth in SEQ ID NO: 53, a sequence having at least 80%, 85% or 90%, preferably at least 91%, 92%, 93%, 94%, 95%, 96%, 97%, 98% or 99% sequence identity to the sequence set forth in SEQ ID NO: 53, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 53, the FR-L2 comprises or consists of the amino acid sequence set forth in SEQ ID NO: 54, a sequence having at least 80%, 85% or 90%, preferably at least 91%, 92%, 93%, 94%, 95%, 96%, 97%, 98% or 99% sequence identity to the sequence set forth in SEQ ID NO: 54, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 54, the FR-L3 comprises or consists of the amino acid sequence set forth in SEQ ID NO: 55, a sequence having at least 80%, 85% or 90%, preferably at least 91%, 92%, 93%, 94%, 95%, 96%, 97%, 98% or 99% sequence identity to the sequence set forth in SEQ ID NO: 55, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 55, and the FR-L4 comprises or consists of the amino acid sequence set forth in SEQ ID NO: 56, a sequence having at least 80%, 85% or 90%, preferably at least 91%, 92%, 93%, 94%, 95%, 96%, 97%, 98% or 99% sequence identity to the sequence set forth in SEQ ID NO: 56, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 56;
(2) the FR-H1 comprises or consists of the amino acid sequence set forth in SEQ ID NO: 57, a sequence having at least 80%, 85% or 90%, preferably at least 91%, 92%, 93%, 94%, 95%, 96%, 97%, 98% or 99% sequence identity to the sequence set forth in SEQ ID NO: 57, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 57,
the FR-H2 comprises or consists of the amino acid sequence set forth in SEQ ID NO: 58, a sequence having at least 80%, 85% or 90%, preferably at least 91%, 92%, 93%, 94%, 95%, 96%, 97%, 98% or 99% sequence identity to the sequence set forth in SEQ ID NO: 58, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 58,
the FR-H3 comprises or consists of the amino acid sequence set forth in SEQ ID NO: 59, a sequence having at least 80%, 85% or 90%, preferably at least 91%, 92%, 93%, 94%, 95%, 96%, 97%, 98% or 99% sequence identity to the sequence set forth in SEQ ID NO: 59, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 59,
the FR-H4 comprises or consists of the amino acid sequence set forth in SEQ ID NO: 60, a sequence having at least 80%, 85% or 90%, preferably at least 91%, 92%, 93%, 94%, 95%, 96%, 97%, 98% or 99% sequence identity to the sequence set forth in SEQ ID NO: 60, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 60,
the FR-L1 comprises or consists of the amino acid sequence set forth in SEQ ID NO: 61, a sequence having at least 80%, 85% or 90%, preferably at least 91%, 92%, 93%, 94%, 95%, 96%, 97%, 98% or 99% sequence identity to the sequence set forth in SEQ ID NO: 61, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 61,
the FR-L2 comprises or consists of the amino acid sequence set forth in SEQ ID NO: 62, a sequence having at least 80%, 85% or 90%, preferably at least 91%, 92%, 93%, 94%, 95%, 96%, 97%, 98% or 99% sequence identity to the sequence set forth in SEQ ID NO: 62, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 62,
the FR-L3 comprises or consists of the amino acid sequence set forth in SEQ ID NO: 63, a sequence having at least 80%, 85% or 90%, preferably at least 91%, 92%, 93%, 94%, 95%, 96%, 97%, 98% or 99% sequence identity to the sequence set forth in SEQ ID NO: 63, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 63, and
the FR-L4 comprises or consists of the amino acid sequence set forth in SEQ ID NO: 64, a sequence having at least 80%, 85% or 90%, preferably at least 91%, 92%, 93%, 94%, 95%, 96%, 97%, 98% or 99% sequence identity to the sequence set forth in SEQ ID NO: 64, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 64;
(3) the FR-H1 comprises or consists of the amino acid sequence set forth in SEQ ID NO: 65, a sequence having at least 80%, 85% or 90%, preferably at least 91%, 92%, 93%, 94%, 95%, 96%, 97%, 98% or 99% sequence identity to the sequence set forth in SEQ ID NO: 65, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 65,
the FR-H2 comprises or consists of the amino acid sequence set forth in SEQ ID NO: 66, a sequence having at least 80%, 85% or 90%, preferably at least 91%, 92%, 93%, 94%, 95%, 96%, 97%, 98% or 99% sequence identity to the sequence set forth in SEQ ID NO: 66, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 66,
the FR-H3 comprises or consists of the amino acid sequence set forth in SEQ ID NO: 67, a sequence having at least 80%, 85% or 90%, preferably at least 91%, 92%, 93%, 94%, 95%, 96%, 97%, 98% or 99% sequence identity to the sequence set forth in SEQ ID NO: 67, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 67,
the FR-H4 comprises or consists of the amino acid sequence set forth in SEQ ID NO: 68, a sequence having at least 80%, 85% or 90%, preferably at least 91%, 92%, 93%, 94%, 95%, 96%, 97%, 98% or 99% sequence identity to the sequence set forth in SEQ ID NO: 68, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 68,
the FR-L1 comprises or consists of the amino acid sequence set forth in SEQ ID NO: 69, a sequence having at least 80%, 85% or 90%, preferably at least 91%, 92%, 93%, 94%, 95%, 96%, 97%, 98% or 99% sequence identity to the sequence set forth in SEQ ID NO: 69, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 69,
the FR-L2 comprises or consists of the amino acid sequence set forth in SEQ ID NO: 70, a sequence having at least 80%, 85% or 90%, preferably at least 91%, 92%, 93%, 94%, 95%, 96%, 97%, 98% or 99% sequence identity to the sequence set forth in SEQ ID NO: 70, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 70,
the FR-L3 comprises or consists of the amino acid sequence set forth in SEQ ID NO: 71, a sequence having at least 80%, 85% or 90%, preferably at least 91%, 92%, 93%, 94%, 95%, 96%, 97%, 98% or 99% sequence identity to the sequence set forth in SEQ ID NO: 71, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 71, and
the FR-L4 comprises or consists of the amino acid sequence set forth in SEQ ID NO: 72, a sequence having at least 80%, 85% or 90%, preferably at least 91%, 92%, 93%, 94%, 95%, 96%, 97%, 98% or 99% sequence identity to the sequence set forth in SEQ ID NO: 72, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 72;
(4) the FR-H1 comprises or consists of the amino acid sequence set forth in SEQ ID NO: 73, a sequence having at least 80%, 85% or 90%, preferably at least 91%, 92%, 93%, 94%, 95%, 96%, 97%, 98% or 99% sequence identity to the sequence set forth in SEQ ID NO: 73, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 73,
the FR-H2 comprises or consists of the amino acid sequence set forth in SEQ ID NO: 74, a sequence having at least 80%, 85% or 90%, preferably at least 91%, 92%, 93%, 94%, 95%, 96%, 97%, 98% or 99% sequence identity to the sequence set forth in SEQ ID NO: 74, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 74,
the FR-H3 comprises or consists of the amino acid sequence set forth in SEQ ID NO: 75, a sequence having at least 80%, 85% or 90%, preferably at least 91%, 92%, 93%, 94%, 95%, 96%, 97%, 98% or 99% sequence identity to the sequence set forth in SEQ ID NO: 75, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 75,
the FR-H4 comprises or consists of the amino acid sequence set forth in SEQ ID NO: 76, a sequence having at least 80%, 85% or 90%, preferably at least 91%, 92%, 93%, 94%, 95%, 96%, 97%, 98% or 99% sequence identity to the sequence set forth in SEQ ID NO: 76, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 76,
the FR-L1 comprises or consists of the amino acid sequence set forth in SEQ ID NO: 77, a sequence having at least 80%, 85% or 90%, preferably at least 91%, 92%, 93%, 94%, 95%, 96%, 97%, 98% or 99% sequence identity to the sequence set forth in SEQ ID NO: 77, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 77,
the FR-L2 comprises or consists of the amino acid sequence set forth in SEQ ID NO: 78, a sequence having at least 80%, 85% or 90%, preferably at least 91%, 92%, 93%, 94%, 95%, 96%, 97%, 98% or 99% sequence identity to the sequence set forth in SEQ ID NO: 78, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 78,
the FR-L3 comprises or consists of the amino acid sequence set forth in SEQ ID NO: 79, a sequence having at least 80%, 85% or 90%, preferably at least 91%, 92%, 93%, 94%, 95%, 96%, 97%, 98% or 99% sequence identity to the sequence set forth in SEQ ID NO: 79, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 79, and
the FR-L4 comprises or consists of the amino acid sequence set forth in SEQ ID NO: 80, a sequence having at least 80%, 85% or 90%, preferably at least 91%, 92%, 93%, 94%, 95%, 96%, 97%, 98% or 99% sequence identity to the sequence set forth in SEQ ID NO: 80, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 80;
(5) the FR-H1 comprises or consists of the amino acid sequence set forth in SEQ ID NO: 81, a sequence having at least 80%, 85% or 90%, preferably at least 91%, 92%, 93%, 94%, 95%, 96%, 97%, 98% or 99% sequence identity to the sequence set forth in SEQ ID NO: 81, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 81,
the FR-H2 comprises or consists of the amino acid sequence set forth in SEQ ID NO: 82, a sequence having at least 80%, 85% or 90%, preferably at least 91%, 92%, 93%, 94%, 95%, 96%, 97%, 98% or 99% sequence identity to the sequence set forth in SEQ ID NO: 82, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 82,
the FR-H3 comprises or consists of the amino acid sequence set forth in SEQ ID NO: 83, a sequence having at least 80%, 85% or 90%, preferably at least 91%, 92%, 93%, 94%, 95%, 96%, 97%, 98% or 99% sequence identity to the sequence set forth in SEQ ID NO: 83, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 83,
the FR-H4 comprises or consists of the amino acid sequence set forth in SEQ ID NO: 84, a sequence having at least 80%, 85% or 90%, preferably at least 91%, 92%, 93%, 94%, 95%, 96%, 97%, 98% or 99% sequence identity to the sequence set forth in SEQ ID NO: 84, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 84,
the FR-L1 comprises or consists of the amino acid sequence set forth in SEQ ID NO: 85, a sequence having at least 80%, 85% or 90%, preferably at least 91%, 92%, 93%, 94%, 95%, 96%, 97%, 98% or 99% sequence identity to the sequence set forth in SEQ ID NO: 85, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 85,
the FR-L2 comprises or consists of the amino acid sequence set forth in SEQ ID NO: 86, a sequence having at least 80%, 85% or 90%, preferably at least 91%, 92%, 93%, 94%, 95%, 96%, 97%, 98% or 99% sequence identity to the sequence set forth in SEQ ID NO: 86, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 86,
the FR-L3 comprises or consists of the amino acid sequence set forth in SEQ ID NO: 87, a sequence having at least 80%, 85% or 90%, preferably at least 91%, 92%, 93%, 94%, 95%, 96%, 97%, 98% or 99% sequence identity to the sequence set forth in SEQ ID NO: 87, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 87, and
the FR-L4 comprises or consists of the amino acid sequence set forth in SEQ ID NO: 88, a sequence having at least 80%, 85% or 90%, preferably at least 91%, 92%, 93%, 94%, 95%, 96%, 97%, 98% or 99% sequence identity to the sequence set forth in SEQ ID NO: 88, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 88;
(6) the FR-H1 comprises or consists of the amino acid sequence set forth in SEQ ID NO: 89, a sequence having at least 80%, 85% or 90%, preferably at least 91%, 92%, 93%, 94%, 95%, 96%, 97%, 98% or 99% sequence identity to the sequence set forth in SEQ ID NO: 89, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 89,
the FR-H2 comprises or consists of the amino acid sequence set forth in SEQ ID NO: 90, a sequence having at least 80%, 85% or 90%, preferably at least 91%, 92%, 93%, 94%, 95%, 96%, 97%, 98% or 99% sequence identity to the sequence set forth in SEQ ID NO: 90, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 90,
the FR-H3 comprises or consists of the amino acid sequence set forth in SEQ ID NO: 91, a sequence having at least 80%, 85% or 90%, preferably at least 91%, 92%, 93%, 94%, 95%, 96%, 97%, 98% or 99% sequence identity to the sequence set forth in SEQ ID NO: 91, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 91,
the FR-H4 comprises or consists of the amino acid sequence set forth in SEQ ID NO: 92, a sequence having at least 80%, 85% or 90%, preferably at least 91%, 92%, 93%, 94%, 95%, 96%, 97%, 98% or 99% sequence identity to the sequence set forth in SEQ ID NO: 92, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 92,
the FR-L1 comprises or consists of the amino acid sequence set forth in SEQ ID NO: 93, a sequence having at least 80%, 85% or 90%, preferably at least 91%, 92%, 93%, 94%, 95%, 96%, 97%, 98% or 99% sequence identity to the sequence set forth in SEQ ID NO: 93, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 93,
the FR-L2 comprises or consists of the amino acid sequence set forth in SEQ ID NO: 94, a sequence having at least 80%, 85% or 90%, preferably at least 91%, 92%, 93%, 94%, 95%, 96%, 97%, 98% or 99% sequence identity to the sequence set forth in SEQ ID NO: 94, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 94,
the FR-L3 comprises or consists of the amino acid sequence set forth in SEQ ID NO: 95, a sequence having at least 80%, 85% or 90%, preferably at least 91%, 92%, 93%, 94%, 95%, 96%, 97%, 98% or 99% sequence identity to the sequence set forth in SEQ ID NO: 95, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 95, and
the FR-L4 comprises or consists of the amino acid sequence set forth in SEQ ID NO: 96, a sequence having at least 80%, 85% or 90%, preferably at least 91%, 92%, 93%, 94%, 95%, 96%, 97%, 98% or 99% sequence identity to the sequence set forth in SEQ ID NO: 96, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 96;
(7) the FR-H1 comprises or consists of the amino acid sequence set forth in SEQ ID NO: 97, a sequence having at least 80%, 85% or 90%, preferably at least 91%, 92%, 93%, 94%, 95%, 96%, 97%, 98% or 99% sequence identity to the sequence set forth in SEQ ID NO: 97, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 97,
the FR-H2 comprises or consists of the amino acid sequence set forth in SEQ ID NO: 98, a sequence having at least 80%, 85% or 90%, preferably at least 91%, 92%, 93%, 94%, 95%, 96%, 97%, 98% or 99% sequence identity to the sequence set forth in SEQ ID NO: 98, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 98,
the FR-H3 comprises or consists of the amino acid sequence set forth in SEQ ID NO: 99, a sequence having at least 80%, 85% or 90%, preferably at least 91%, 92%, 93%, 94%, 95%, 96%, 97%, 98% or 99% sequence identity to the sequence set forth in SEQ ID NO: 99, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 99,
the FR-H4 comprises or consists of the amino acid sequence set forth in SEQ ID NO: 100, a sequence having at least 80%, 85% or 90%, preferably at least 91%, 92%, 93%, 94%, 95%, 96%, 97%, 98% or 99% sequence identity to the sequence set forth in SEQ ID NO: 100, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 100,
the FR-L1 comprises or consists of the amino acid sequence set forth in SEQ ID NO: 101, a sequence having at least 80%, 85% or 90%, preferably at least 91%, 92%, 93%, 94%, 95%, 96%, 97%, 98% or 99% sequence identity to the sequence set forth in SEQ ID NO: 101, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 101,
the FR-L2 comprises or consists of the amino acid sequence set forth in SEQ ID NO: 102, a sequence having at least 80%, 85% or 90%, preferably at least 91%, 92%, 93%, 94%, 95%, 96%, 97%, 98% or 99% sequence identity to the sequence set forth in SEQ ID NO: 102, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 102,
the FR-L3 comprises or consists of the amino acid sequence set forth in SEQ ID NO: 103, a sequence having at least 80%, 85% or 90%, preferably at least 91%, 92%, 93%, 94%, 95%, 96%, 97%, 98% or 99% sequence identity to the sequence set forth in SEQ ID NO: 103, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 103, and
the FR-L4 comprises or consists of the amino acid sequence set forth in SEQ ID NO: 104, a sequence having at least 80%, 85% or 90%, preferably at least 91%, 92%, 93%, 94%, 95%, 96%, 97%, 98% or 99% sequence identity to the sequence set forth in SEQ ID NO: 104, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 104;
(8) the FR-H1 comprises or consists of the amino acid sequence set forth in SEQ ID NO: 105, a sequence having at least 80%, 85% or 90%, preferably at least 91%, 92%, 93%, 94%, 95%, 96%, 97%, 98% or 99% sequence identity to the sequence set forth in SEQ ID NO: 105, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 105,
the FR-H2 comprises or consists of the amino acid sequence set forth in SEQ ID NO: 106, a sequence having at least 80%, 85% or 90%, preferably at least 91%, 92%, 93%, 94%, 95%, 96%, 97%, 98% or 99% sequence identity to the sequence set forth in SEQ ID NO: 106, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 106,
the FR-H3 comprises or consists of the amino acid sequence set forth in SEQ ID NO: 107, a sequence having at least 80%, 85% or 90%, preferably at least 91%, 92%, 93%, 94%, 95%, 96%, 97%, 98% or 99% sequence identity to the sequence set forth in SEQ ID NO: 107, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 107,
the FR-H4 comprises or consists of the amino acid sequence set forth in SEQ ID NO: 108, a sequence having at least 80%, 85% or 90%, preferably at least 91%, 92%, 93%, 94%, 95%, 96%, 97%, 98% or 99% sequence identity to the sequence set forth in SEQ ID NO: 108, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 108,
the FR-L1 comprises or consists of the amino acid sequence set forth in SEQ ID NO: 109, a sequence having at least 80%, 85% or 90%, preferably at least 91%, 92%, 93%, 94%, 95%, 96%, 97%, 98% or 99% sequence identity to the sequence set forth in SEQ ID NO: 109, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 109,
the FR-L2 comprises or consists of the amino acid sequence set forth in SEQ ID NO: 110, a sequence having at least 80%, 85% or 90%, preferably at least 91%, 92%, 93%, 94%, 95%, 96%, 97%, 98% or 99% sequence identity to the sequence set forth in SEQ ID NO: 110, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 110,
the FR-L3 comprises or consists of the amino acid sequence set forth in SEQ ID NO: 111, a sequence having at least 80%, 85% or 90%, preferably at least 91%, 92%, 93%, 94%, 95%, 96%, 97%, 98% or 99% sequence identity to the sequence set forth in SEQ ID NO: 111, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 111, and
the FR-L4 comprises or consists of the amino acid sequence set forth in SEQ ID NO: 112, a sequence having at least 80%, 85% or 90%, preferably at least 91%, 92%, 93%, 94%, 95%, 96%, 97%, 98% or 99% sequence identity to the sequence set forth in SEQ ID NO: 112, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 112;
(9) the FR-H1 comprises or consists of the amino acid sequence set forth in SEQ ID NO: 113, a sequence having at least 80%, 85% or 90%, preferably at least 91%, 92%, 93%, 94%, 95%, 96%, 97%, 98% or 99% sequence identity to the sequence set forth in SEQ ID NO: 113, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 113,
the FR-H2 comprises or consists of the amino acid sequence set forth in SEQ ID NO: 114, a sequence having at least 80%, 85% or 90%, preferably at least 91%, 92%, 93%, 94%, 95%, 96%, 97%, 98% or 99% sequence identity to the sequence set forth in SEQ ID NO: 114, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 114,
the FR-H3 comprises or consists of the amino acid sequence set forth in SEQ ID NO: 115, a sequence having at least 80%, 85% or 90%, preferably at least 91%, 92%, 93%, 94%, 95%, 96%, 97%, 98% or 99% sequence identity to the sequence set forth in SEQ ID NO: 115, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 115,
the FR-H4 comprises or consists of the amino acid sequence set forth in SEQ ID NO: 116, a sequence having at least 80%, 85% or 90%, preferably at least 91%, 92%, 93%, 94%, 95%, 96%, 97%, 98% or 99% sequence identity to the sequence set forth in SEQ ID NO: 116, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 116,
the FR-L1 comprises or consists of the amino acid sequence set forth in SEQ ID NO: 117, a sequence having at least 80%, 85% or 90%, preferably at least 91%, 92%, 93%, 94%, 95%, 96%, 97%, 98% or 99% sequence identity to the sequence set forth in SEQ ID NO: 117, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 117,
the FR-L2 comprises or consists of the amino acid sequence set forth in SEQ ID NO: 118, a sequence having at least 80%, 85% or 90%, preferably at least 91%, 92%, 93%, 94%, 95%, 96%, 97%, 98% or 99% sequence identity to the sequence set forth in SEQ ID NO: 118, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 118,
the FR-L3 comprises or consists of the amino acid sequence set forth in SEQ ID NO: 119, a sequence having at least 80%, 85% or 90%, preferably at least 91%, 92%, 93%, 94%, 95%, 96%, 97%, 98% or 99% sequence identity to the sequence set forth in SEQ ID NO: 119, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 119, and
the FR-L4 comprises or consists of the amino acid sequence set forth in SEQ ID NO: 120, a sequence having at least 80%, 85% or 90%, preferably at least 91%, 92%, 93%, 94%, 95%, 96%, 97%, 98% or 99% sequence identity to the sequence set forth in SEQ ID NO: 120, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 120; or
(10) the FR-H1 comprises or consists of the amino acid sequence set forth in SEQ ID NO: 136, a sequence having at least 80%, 85% or 90%, preferably at least 91%, 92%, 93%, 94%, 95%, 96%, 97%, 98% or 99% sequence identity to the sequence set forth in SEQ ID NO: 136, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 136,
the FR-H2 comprises or consists of the amino acid sequence set forth in SEQ ID NO: 137, a sequence having at least 80%, 85% or 90%, preferably at least 91%, 92%, 93%, 94%, 95%, 96%, 97%, 98% or 99% sequence identity to the sequence set forth in SEQ ID NO: 137, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 137,
the FR-H3 comprises or consists of the amino acid sequence set forth in SEQ ID NO: 138, a sequence having at least 80%, 85% or 90%, preferably at least 91%, 92%, 93%, 94%, 95%, 96%, 97%, 98% or 99% sequence identity to the sequence set forth in SEQ ID NO: 138, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 138,
the FR-H4 comprises or consists of the amino acid sequence set forth in SEQ ID NO: 139, a sequence having at least 80%, 85% or 90%, preferably at least 91%, 92%, 93%, 94%, 95%, 96%, 97%, 98% or 99% sequence identity to the sequence set forth in SEQ ID NO: 139, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 139,
the FR-L1 comprises or consists of the amino acid sequence set forth in SEQ ID NO: 140, a sequence having at least 80%, 85% or 90%, preferably at least 91%, 92%, 93%, 94%, 95%, 96%, 97%, 98% or 99% sequence identity to the sequence set forth in SEQ ID NO: 140, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 140,
the FR-L2 comprises or consists of the amino acid sequence set forth in SEQ ID NO: 141, a sequence having at least 80%, 85% or 90%, preferably at least 91%, 92%, 93%, 94%, 95%, 96%, 97%, 98% or 99% sequence identity to the sequence set forth in SEQ ID NO: 141, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 141,
the FR-L3 comprises or consists of the amino acid sequence set forth in SEQ ID NO: 142, a sequence having at least 80%, 85% or 90%, preferably at least 91%, 92%, 93%, 94%, 95%, 96%, 97%, 98% or 99% sequence identity to the sequence set forth in SEQ ID NO: 142, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 142, and
the FR-L4 comprises or consists of the amino acid sequence set forth in SEQ ID NO: 143, a sequence having at least 80%, 85% or 90%, preferably at least 91%, 92%, 93%, 94%, 95%, 96%, 97%, 98% or 99% sequence identity to the sequence set forth in SEQ ID NO: 143, or an amino acid sequence having one or more (preferably 1, 2, 3, 4, 5, 6, 7, 8, 9 or 10) conservative amino acid mutations (preferably substitutions, insertions or deletions) compared to the amino acid sequence set forth in SEQ ID NO: 143.

4. The antibody or the antigen-binding fragment thereof according to any one of claims 1-3, wherein the antibody binds to human IL-4RA protein with a K_{D} less than about 10⁻⁵ M, e.g., less than about 10⁻⁶ M, 10⁻⁷ M, 10⁻⁸ M, 10⁻⁹ M or 10⁻¹⁰ M or less.

5. The antibody or the antigen-binding fragment thereof according to any one of claims 1-4, wherein the antibody binds to human IL-4RA protein with an EC₅₀ less than about 100 nM, e.g., less than about 10 nM, 1 nM, 0.9 nM, 0.8 nM, 0.7 nM, 0.6 nM, 0.5 nM, 0.4 nM, 0.3 nM, 0.2 nM, 0.1 nM or less.

6. The antibody or the antigen-binding fragment thereof according to any one of claims 1-5, wherein the antibody is a humanized antibody, a chimeric antibody or a multispecific antibody (e.g., bispecific antibody).

7. The antibody or the antigen-binding fragment thereof according to claim 6, wherein the constant region of the antibody is humanized, preferably derived from human IgG, more preferably IgG4.

8. The antibody or the antigen-binding fragment thereof according to claim 7, wherein the heavy chain constant region of the antibody is an Ig gamma-4 chain C region, more preferably the Ig gamma-4 chain C region of GenBank ACCESSION No: P01861.1; the light chain constant region is an Ig kappa chain C region, more preferably the Ig kappa chain C region of GenBank ACCESSION No: P01834.

9. The antibody or the antigen-binding fragment thereof according to any one of claims 1-8, wherein the antigen-binding fragment is selected from Fab, Fab', F(ab')₂, Fd, Fv, dAb, Fab/c, complementarity determining region (CDR) fragment, single chain antibody (e.g., scFv), bivalent antibody and domain antibody.

10. An isolated polypeptide selected from the group consisting of:
(1) an isolated polypeptide comprising the sequences set forth in SEQ ID NOs: 9, 10 and 11, wherein the polypeptide specifically binds to human IL-4RA as part of an anti-human IL-4RA antibody, the antibody further comprising the sequences set forth in SEQ ID NOs: 12, 13 and 14;
(2) an isolated polypeptide comprising the sequences set forth in SEQ ID NOs: 15, 16 and 17, wherein the polypeptide specifically binds to human IL-4RA as part of an anti-human IL-4RA antibody, the antibody further comprising the sequences set forth in SEQ ID NOs: 18, 19 and 20;
(3) an isolated polypeptide comprising the sequences set forth in SEQ ID NOs: 12, 13 and 14, wherein the polypeptide specifically binds to human IL-4RA as part of an anti-human IL-4RA antibody, the antibody further comprising the sequences set forth in SEQ ID NOs: 9, 10 and 11;
(4) an isolated polypeptide comprising the sequences set forth in SEQ ID NOs: 18, 19 and 20, wherein the polypeptide specifically binds to human IL-4RA as part of an anti-human IL-4RA antibody, the antibody further comprising the sequences set forth in SEQ ID NOs: 15, 16 and 17;
(5) an isolated polypeptide comprising the sequences set forth in SEQ ID NOs: 130, 131 and 132, wherein the polypeptide specifically binds to human IL-4RA as part of an anti-human IL-4RA antibody, the antibody further comprising the sequences set forth in SEQ ID NOs: 133, 134 and 135;
(6) an isolated polypeptide comprising the sequences set forth in SEQ ID NOs: 133, 134 and 135, wherein the polypeptide specifically binds to human IL-4RA as part of an anti-human IL-4RA antibody, the antibody further comprising the sequences set forth in SEQ ID NOs: 130, 131 and 132;
(7) an isolated polypeptide comprising a sequence selected from the sequences set forth in SEQ ID NOs: 2, 6, 22, 26, 30, 34 and 38, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences, wherein the polypeptide specifically binds to human IL-4RA as part of an anti-human IL-4RA antibody, the antibody further comprising a sequence respectively selected from the sequences set forth in SEQ ID NOs: 4, 8, 24, 28, 32, 36 and 40, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences;
(8) an isolated polypeptide comprising a sequence selected from the sequence set forth in SEQ ID NO: 42, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 44, a sequence having at least 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
(9) an isolated polypeptide comprising a sequence selected from the sequence set forth in SEQ ID NO: 42, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 48, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
(10) an isolated polypeptide comprising a sequence selected from the sequence set forth in SEQ ID NO: 46, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 48, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
(11) an isolated polypeptide comprising a sequence selected from the sequence set forth in SEQ ID NO: 34, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 28, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
(12) an isolated polypeptide comprising a sequence selected from the sequences set forth in SEQ ID NOs: 4, 8, 24, 28, 32, 36 and 40, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences, wherein the polypeptide specifically binds to human IL-4RA as part of an anti-human IL-4RA antibody, the antibody further comprising a sequence respectively selected from the sequences set forth in SEQ ID NOs: 2, 6, 22, 26, 30, 34 and 38, a sequence having at least 80%, 85% or 90%, preferably at least 91%, 92%, 93%, 94%, 95%, 96%, 97%, 98% or 99% sequence identity to the sequences, or an amino acid sequence having one or more (preferably 1, 2, 3, 4, 5, 6, 7, 8, 9 or 10) conservative amino acid mutations (preferably substitutions, insertions or deletions) compared to the sequences;
(13) an isolated polypeptide comprising a sequence selected from the sequence set forth in SEQ ID NO: 44, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 42, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
(14) an isolated polypeptide comprising a sequence selected from the sequence set forth in SEQ ID NO: 48, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 42, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
(15) an isolated polypeptide comprising a sequence selected from the sequence set forth in SEQ ID NO: 48, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 46, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
(16) an isolated polypeptide comprising a sequence selected from the sequence set forth in SEQ ID NO: 28, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 34, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence;
(17) an isolated polypeptide comprising a sequence selected from the sequence set forth in SEQ ID NO: 127, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 129, a sequence having at least 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence; or
(18) an isolated polypeptide comprising a sequence selected from the sequence set forth in SEQ ID NO: 129, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence, wherein the polypeptide specifically binds to human IL-4RA as part of an anti-human IL-4RA antibody, the antibody further comprising a sequence selected from the sequence set forth in SEQ ID NO: 127, a sequence having at least 80%, 85% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared to the sequence.

11. An isolated polynucleotide encoding the isolated polypeptide according to claim 10.

12. A vector comprising the isolated polynucleotide according to claim 11.

13. A host cell comprising the isolated polynucleotide according to claim 11 or the vector according to claim 12.

14. A method for preparing the antibody or the antigen-binding fragment thereof according to any one of claims 1-9, comprising cultivating the host cell of claim 13.

15. An antibody conjugate comprising the antibody or the antigen-binding fragment thereof according to any one of claims 1-9 and a conjugated moiety coupled thereto, wherein preferably the conjugated moiety is selected from a purification tag (e.g., His-tag), a cytotoxic agent, a detectable label, a radioisotope, a luminescent substance, a colored substance, an enzyme or polyethylene glycol.

16. A multispecific antibody, preferably a bispecific antibody, comprising the antibody or the antigen-binding fragment thereof according to any one of claims 1-9, and an antibody or an antigen-binding fragment against another antigen and/or to another antigenic epitope.

17. A fusion protein comprising the antibody or the antigen-binding fragment thereof according to any one of claims 1-9.

18. A pharmaceutical composition comprising the antibody or antigen-binding fragment thereof according to any one of claims 1-9, the antibody conjugate according to claim 15, the multispecific antibody according to claim 16 or the fusion protein according to claim 17, and optionally, a pharmaceutically acceptable carrier and/or excipient.

19. The pharmaceutical composition according to claim 18, wherein the pharmaceutical composition is used alone or in combination with one or more medicaments.

20. The pharmaceutical composition according to claim 18, wherein the pharmaceutical composition is in a dosage form suitable for oral administration to the gastrointestinal (GI) tract, preferably the dosage form being selected from tablets, capsules, pills, powders, granules, emulsions, microemulsions, solutions, suspensions, syrups and elixirs; or the pharmaceutical composition is in a dosage form suitable for subcutaneous injection, intradermal injection, intravenous injection, intramuscular injection or intralesional injection.

21. A kit comprising the antibody or the antigen-binding fragment thereof according to any one of claims 1-9, the antibody conjugate according to claim 15, the multispecific antibody according to claim 16 or the fusion protein according to claim 17, wherein preferably the kit further comprises a second antibody that specifically recognizes the antibody or the antigen-binding fragment thereof according to any one of claims 1-9, the antibody conjugate according to claim 15, the multispecific antibody according to claim 16 or the fusion protein of claim 17; optionally, the second antibody further comprises a detectable label, such as a radioisotope, a luminescent substance, a colored substance, or an enzyme.

22. Use of the antibody or the antigen-binding fragment thereof according to any one of claims 1-9, the antibody conjugate according to claim 15, the multispecific antibody according to claim 16 or the fusion protein according to claim 17 in preparing a kit for detecting the presence or level of human IL-4RA in a sample or for the prevention and/or treatment and/or adjuvant treatment and/or diagnosis of an allergic disease, a tumor, an autoimmune disease, a skin infection, tissue fibrosis, rhinosinusitis, nasal polyps and chronic obstructive pulmonary disease, wherein preferably, the allergic disease is selected from atopic dermatitis, allergic rhinitis, asthma and allergy, and more preferably atopic dermatitis includes moderate and severe atopic dermatitis.

23. Use of the antibody or the antigen-binding fragment thereof according to any one of claims 1-9, the antibody conjugate according to claim 15, the multispecific antibody according to claim 16 or the fusion protein according to claim 17 in preparing a medicament selected from:
a medicament for blocking the binding of human IL-4RA to IL-4 or IL-13,
a medicament for blocking the activity of human IL-4RA or down-regulating the level of human IL-4RA, and
a medicament for blocking a cellular biological response mediated by the binding of human IL-4 or human IL-13 to IL-4RA;
wherein preferably, the ligand of human IL-4RA is human IL-4 or human IL-13, more preferably, human IL-4.

24. Use of the antibody or the antigen-binding fragment thereof according to any one of claims 1-9, the antibody conjugate according to claim 15, the multispecific antibody according to claim 16 or the fusion protein according to claim 17 in preparing a medicament for the prevention and/or treatment and/or adjuvant treatment and/or diagnosis of an allergic disease, a tumor, an autoimmune disease, a skin infection, tissue fibrosis, rhinosinusitis, nasal polyps, chronic obstructive pulmonary disease, wherein preferably, the allergic disease is selected from atopic dermatitis, allergic rhinitis, asthma and allergy, and more preferably atopic dermatitis includes moderate and severe atopic dermatitis.

25. An *in vivo* or *in vitro* method comprising administering a cell comprising the antibody or the antigen-binding fragment thereof according to any one of claims 1-9, the antibody conjugate according to claim 15, the multispecific antibody according to claim 16 or the fusion protein according to claim 17, or administering to a subject in need an effective amount of the antibody or the antigen-binding fragment thereof according to any one of claims 1-9, the antibody conjugate according to claim 15, the multispecific antibody according to claim 16 or the fusion protein according to claim 17, the method being selected from:
a method for blocking the binding of IL-4RA to IL-4 or IL-13,
a method for down-regulating IL-4RA activity or level, or
a method for blocking a cellular biological response mediated by the binding of human IL-4 or human IL-13 to IL-4R;
wherein preferably, the ligand of IL-4RA is IL-4 or IL-13, more preferably, IL-4;
more preferably, the *in vitro* method is non-therapeutic and/or non-diagnostic.

26. A method for the prevention and/or treatment and/or adjuvant treatment and/or diagnosis of an allergic disease, a tumor, an autoimmune disease, tissue fibrosis, rhinosinusitis, nasal polyps, chronic obstructive pulmonary disease, comprising administering the antibody or the antigen-binding fragment thereof according to any one of claims 1-9, the antibody conjugate according to claim 15, the multispecific antibody according to claim 16 or the fusion protein according to claim 17 to a subject in need, wherein preferably, the allergic disease is selected from atopic dermatitis, allergic rhinitis, asthma and allergy, and more preferably atopic dermatitis includes moderate and severe atopic dermatitis.

27. A hybridoma cell having the accession number of CCTCC NO: C202010, CCTCC NO: C2018131 or CCTCC NO: C2018132.

28. A monoclonal antibody secreted by the hybridoma cell according to claim 27.
